(19) Europäisches Patentamt
European Patent Office
Office européen des brevets

(11) EP 4 775 226 A1

(12) EUROPEAN PATENT APPLICATION
published in accordance with Art. 153(4) EPC

(43) Date of publication:
**15.07.2026 Bulletin 2026/29**

(21) Application number: **24862000.7**

(22) Date of filing: **04.09.2024**

(51) International Patent Classification (IPC):
***A61K 47/68*** (2017.01) ***A61K 47/54*** (2017.01)
***C07K 16/30*** (2006.01) ***A61P 35/00*** (2006.01)
***C12N 15/13*** (2006.01)

(52) Cooperative Patent Classification (CPC):
**A61K 47/54; A61K 47/68; A61P 35/00; C07K 16/00; C07K 16/30**

(86) International application number:
**PCT/CN2024/116899**

(87) International publication number:
**WO 2025/051159 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.09.2023 CN 202311141587**
**26.08.2024 CN 202411179009**

(71) Applicant: **Lepu Biopharma Co., Ltd.**
**Shanghai 201112 (CN)**

(72) Inventors:
- **LI, Hongfeng**
  **Shanghai 201112 (CN)**
- **WANG, Yanchun**
  **Shanghai 201112 (CN)**
- **LIU, Wenchao**
  **Shanghai 201112 (CN)**
- **LIU, Wei**
  **Shanghai 201112 (CN)**
- **MA, Na**
  **Shanghai 201112 (CN)**
- **HU, Chaohong**
  **Shanghai 201112 (CN)**
- **SHEN, Hao**
  **Shanghai 201112 (CN)**
- **CUI, Feifei**
  **Shanghai 201112 (CN)**
- **YANG, Liu**
  **Shanghai 201112 (CN)**
- **FANG, Lei**
  **Shanghai 201112 (CN)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) ANTIBODY DRUG CONJUGATE TARGETING GPC3 AND USE THEREOF

(57) The present invention relates to a GPC3 antibody drug conjugate and a use thereof, and specifically provides an antibody drug conjugate, or a pharmaceutically acceptable salt, solvate, or solvate of the salt thereof. The antibody drug conjugate has a structure as shown in formula I, Ab being an anti-GPC3 antibody. The antibody drug conjugate has good tumor cell growth inhibition activity *in vivo* and *in vitro,* and has good application prospects.

$$\text{Ab-(L-D)}_p \qquad (I)$$

EP 4 775 226 A1

## Description

### Cross-Reference to Related Applications

The present application is based on and claims the priorities to the application with the CN application number of 202311141587.3 and the filing date of September 5, 2023, as well as the application with the CN application number of 202411179009.3 and the filing date of August 26, 2024, the contents of both of which are hereby incorporated by reference into the present application in their entirety.

### Technical Field

The present invention relates to the field of pharmaceutical chemistry, and in particular to a GPC3 antibody drug conjugate and the use thereof.

### Background Art

Liver cancer is the fifth most prevalent cancer in the world and also the third most common cause of cancer-related death. Hepatocellular carcinoma (HCC) is the main form of liver cancer and accounts for 90% of all liver cancers. At present, a well-accepted therapeutic scheme is multidisciplinary comprehensive treatment with surgical resection as the core. Although there is an improved short-term curative effect, the long-term curative effect is still not ideal. Therefore, exploring a more effective new therapeutic strategy for treating liver cancer is currently a research hotspot at home and abroad, and it is still urgent to develop a new drug for treating liver cancer.

GPC3, with its full name of Glypican-3, is a member of the heparan sulfate proteoglycan family, and is anchored to the cell surface via glycosyl phosphatidylinositol on the cell membrane. The protein core of GPC3 consists of two subunits, wherein the N-terminal subunit is about 40 kDa in size, and the C-terminal subunit is about 30 kDa in size. Six Glypicans (GPC1-6) have been identified in mammals. GPC3 plays an important role in regulating cell proliferation, differentiation, adhesion, and migration. It is believed in some studies that GPC3 interacts with Wnt protein and Frizzled receptor to form a complex and trigger downstream signaling. The core protein of GPC3 can act as a Wnt co-receptor or receiver.

In the human body, the expression of GPC3 protein expressed from the GPC3 gene varies significantly in different development stages and different tissues. For example, the content of the protein in normal adult tissues is very low, and the protein is under-expressed or not expressed in cancers such as gastric cancer, breast cancer, and ovarian cancer but often over-expressed (with a positive rate of 70% or more) in hepatocellular carcinoma (HCC). Studies have shown that GPC3 is a liver cancer-specific associated antigen, which can help to confirm the nature of lesions in the early stage of HCC and can significantly improve the accuracy of diagnosis. Therefore, GPC3 is considered to have great potential in targeted therapy for liver cancer.

In recent years, GPC3 targeted therapy has attracted increasing attention. At present, the therapeutic strategies based on GPC3 target mainly focus on antibody drugs, cell therapies, vaccines, etc. Therapeutic anti-GPC3 antibodies have been developed, including antibodies such as GC33 (with the phase 2 clinical trial completed), YP7 and HN3 and bispecific antibodies (e.g., ERY974 and CM350 under clinical trials). Some of these antibodies can function by inhibiting Wnt signaling in liver cancer cells. In addition, chimeric antigen receptor (CAR) T cell immunotherapies have been developed against GPC3 in different studies for treating liver cancer. In studies using models of mice with xenograft or orthotopic liver cancer, CAR-T cells can eliminate GPC3-positive cancer cells by inducing cell death mediated by perforin and granzyme and reducing WNT signaling in tumor cells.

### Summary of the Invention

Through a lot of experiments and creative effort, the inventors of the present application prepared an anti-GPC3 antibody drug conjugate (ADC) and confirmed that it has good biological activity, thus completing the present invention.

To this end, in a first aspect of the present invention, the present invention provides an antibody drug conjugate, or a pharmaceutically acceptable salt or solvate thereof, or a solvate of the salt, wherein the antibody drug conjugate has a structure represented by formula I,

$$Ab\text{-}(L\text{-}D)_p \qquad \text{formula I}$$

in which:

Ab is an anti-GPC3 antibody or an antigen binding fragment thereof, and the anti-GPC3 antibody comprises a heavy chain variable region and a light chain variable region, wherein CDR1, CDR2, and CDR3 in the heavy chain variable

region comprise the sequences as set forth in SEQ ID NO: 1, SEQ ID NO: 2, and SEQ ID NO: 3, or mutants thereof, respectively, and CDR1, CDR2, and CDR3 in the light chain variable region comprise the sequences as set forth in SEQ ID NO: 9, SEQ ID NO: 10, and SEQ ID NO: 11, or mutants thereof, respectively;
L is a linker selected from mc-AAN, mc-AAQ, mcc-AAN, mcc-AAQ, mcc-Ala-Ala-(3-cyano-alanine), mcc-Ala-Ala-(2-amino-4-cyanobutanoic acid), and mcc-Ala-Ala-Ala-Ala-Gln;
D is a cytotoxin, which is Exatecan (abbreviated as Exa); and
p is any numerical value between 1 and 10 (e.g., 1, 1.5, 2, 2.5, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10, or e.g., 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, or 9.5-10).

In formula I, the structural formulas of mc and mcc are as shown below:

| | |
|---|---|
| mcc | 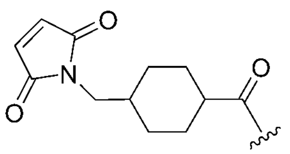 |
| mc | 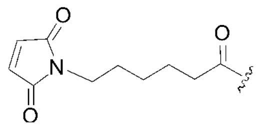 |

In formula I, L-D means that the linker is linked to the cytotoxin via a covalent bond to form an L-D molecule; and Ab-(L-D)$_p$ means that p L-D molecules are conjugated to Ab via covalent bonds.
Those skilled in the art can understand that mcc and Ab, upon connection via a covalent bond, form the following structural fragment:

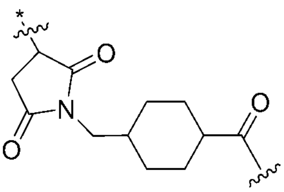
,

and mc and Ab, upon connection via a covalent bond, form the following structural fragment:

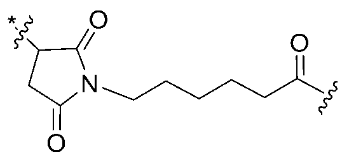
,

wherein the * end is connected to Ab.
In some embodiments, L is selected from mc-AAN, mc-AAQ, mcc-AAN, mcc-AAQ, mcc-Ala-Ala-(3-cyano-L-alanine), mcc-Ala-Ala-((*S*)-2-amino-4-cyanobutanoic acid), and mcc-Ala-Ala-Ala-Ala-Gln.
In some embodiments, in formula I, the structural formulas of L-Ds before conjugation to Ab are as shown below:

| | |
|---|---|
| 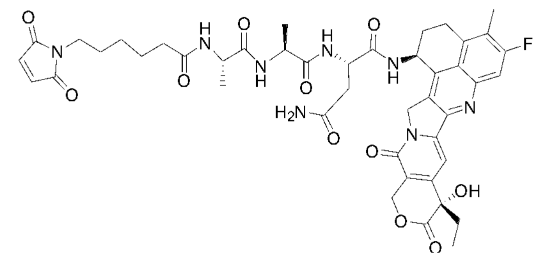 | 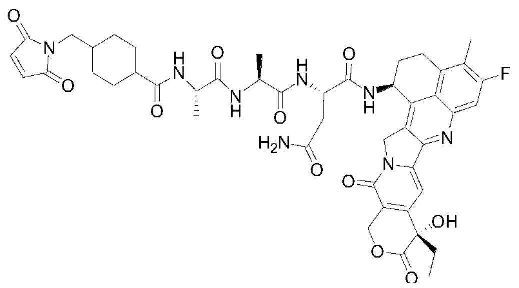 |
| 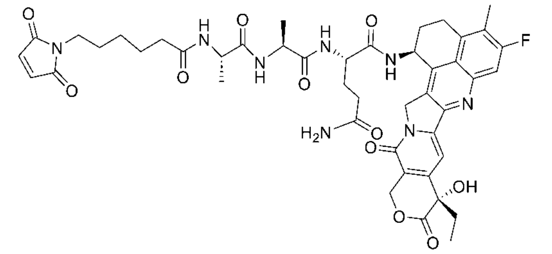 | 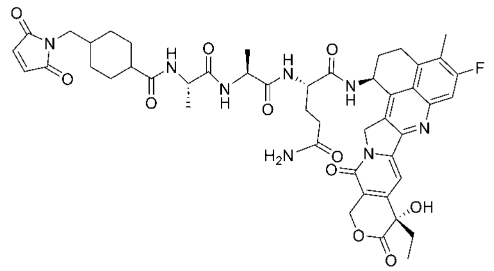 |
| 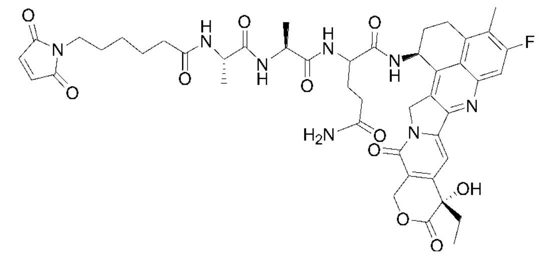 | 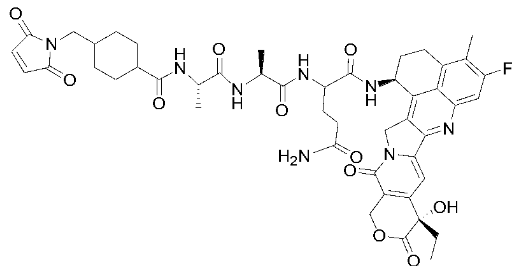 |
| 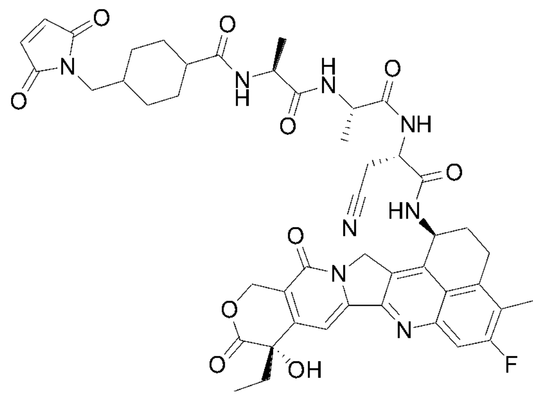<br>mcc-Ala-Ala-(3-cyano-L-alanine)-Exatecan | 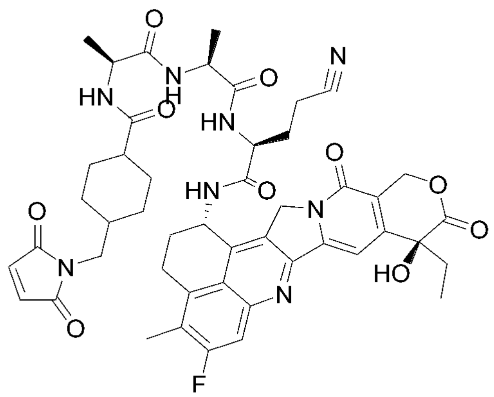<br>mcc-Ala-Ala-((*S*)-2-amino-4-cyanobutanoic acid)-Exatecan |
| 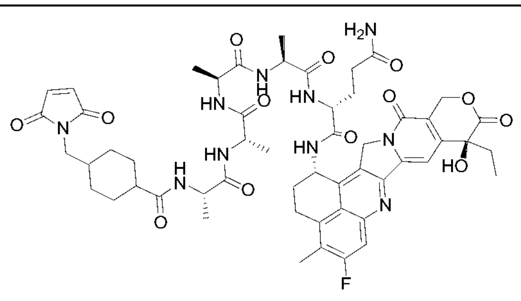<br>mcc-Ala-Ala-Ala-Ala-Gln-Exatecan | |

When the above nine L-Ds are conjugated to Ab via covalent bonds, they are all formed by conjugating succinimide at one end of L-D to mercapto in the antibody. For example, when mc-AAN-Exatecan is conjugated to Ab via a covalent bond, the structural formula of the formed ADC is as shown below:

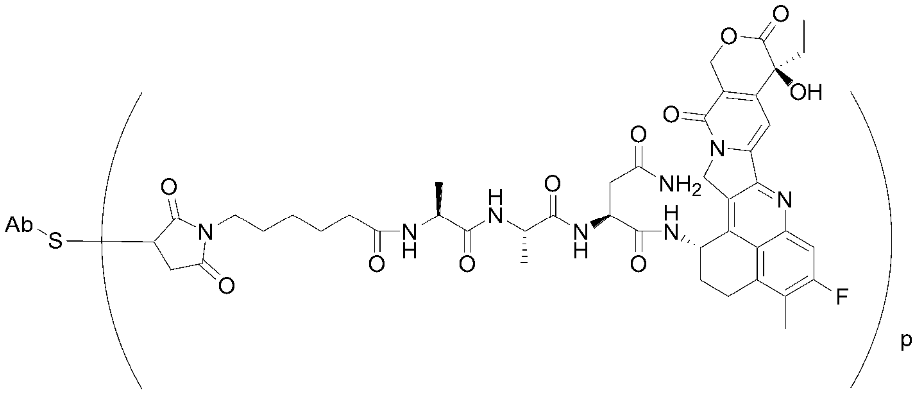

(IIa).

Similarly, those skilled in the art can understand that when mcc-AAN-Exatecan is conjugated to Ab via a covalent bond, the structural formula of the formed ADC is as shown below:

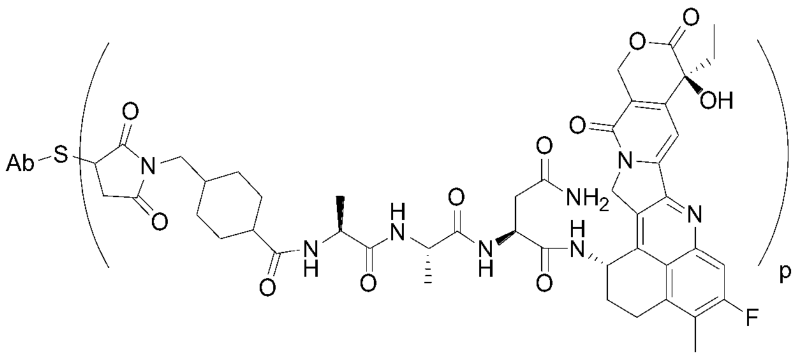

(IIb).

Also similarly, those skilled in the art can understand that when mc-AAQ-Exatecan is conjugated to Ab via a covalent bond, the structural formula of the formed ADC is as shown below:

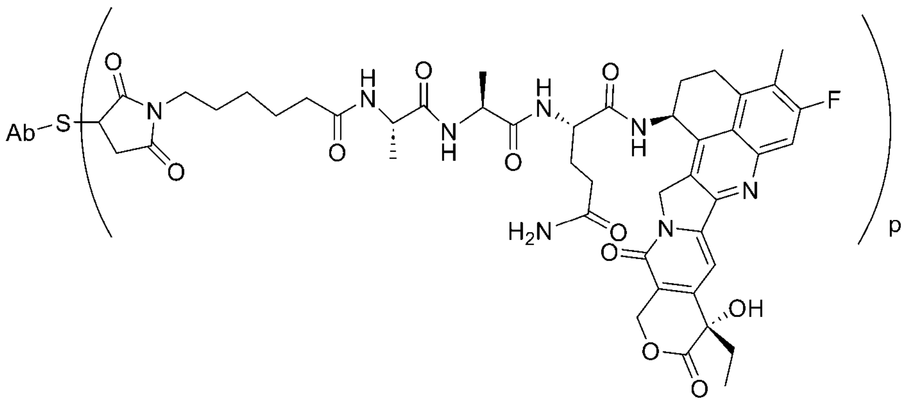

(IIc).

Also similarly, those skilled in the art can understand that when mcc-AAQ-Exatecan is conjugated to Ab via a covalent bond, the structural formula of the formed ADC is as shown below:

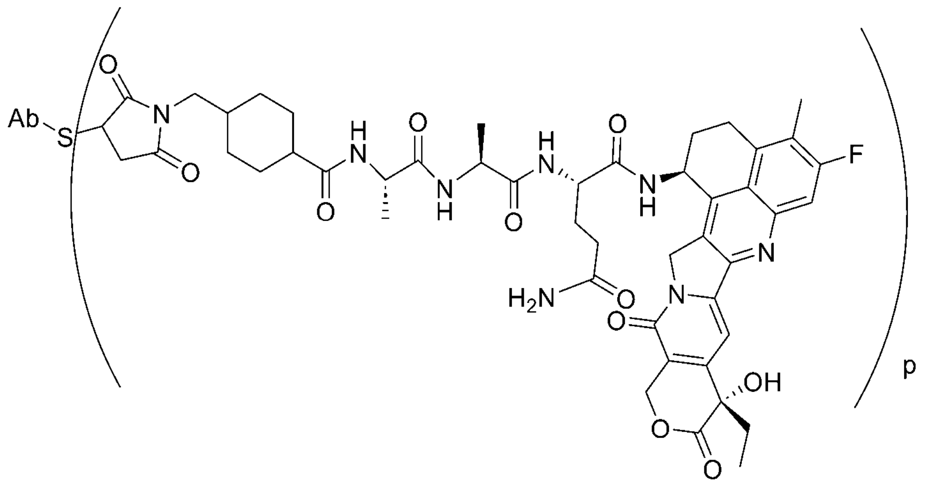

(IId).

In addition, also similarly, those skilled in the art can understand that when

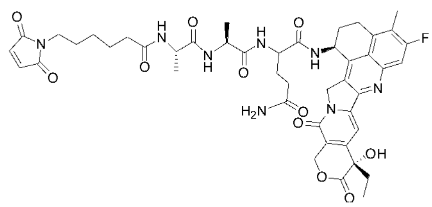

is conjugated to Ab via a covalent bond, the structural formula of the formed ADC is as shown below:

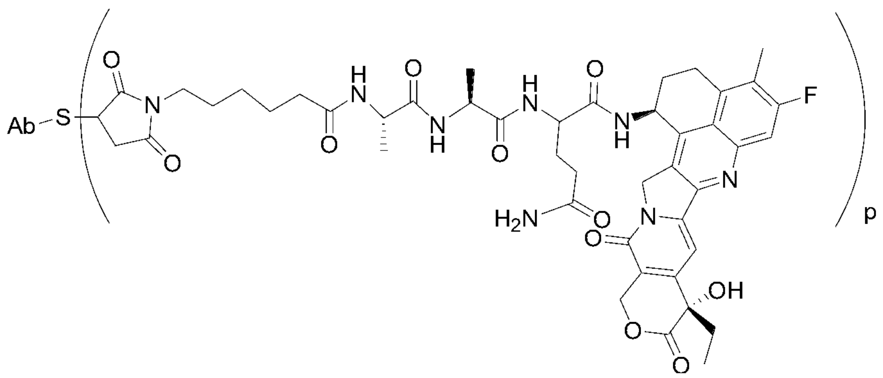

(IIe).

Also similarly, those skilled in the art can understand that when

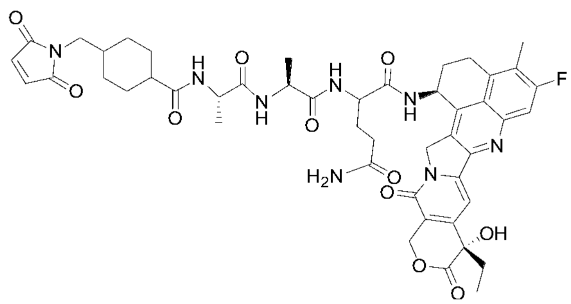

is conjugated to Ab via a covalent bond, the structural formula of the formed ADC is as shown below:

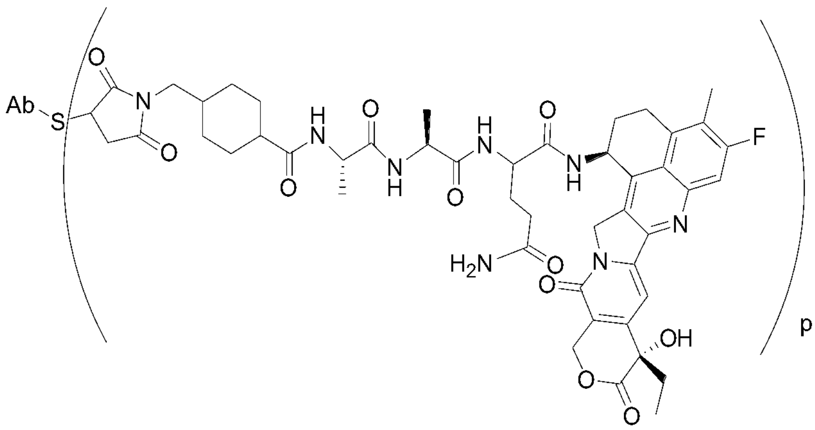

(IIf).

Also similarly, those skilled in the art can understand that when mcc-Ala-Ala-(3-cyano-L-alanine)-Exatecan is conjugated to Ab via a covalent bond, the structural formula of the formed ADC is as shown below:

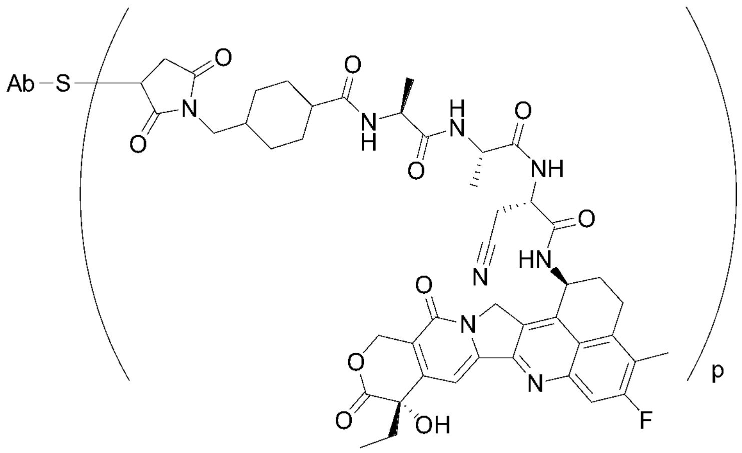

(IIg).

Also similarly, those skilled in the art can understand that when mcc-Ala-Ala-((*S*)-2-amino-4-cyanobutanoic acid)-Exatecan is conjugated to Ab via a covalent bond, the structural formula of the formed ADC is as shown below:

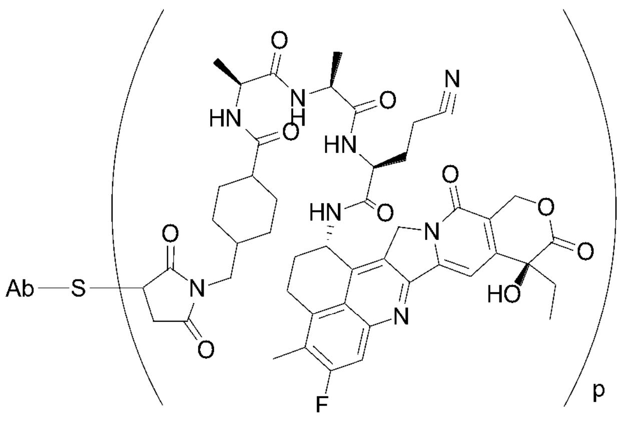

(IIh).

Also similarly, those skilled in the art can understand that when mcc-Ala-Ala-Ala-Ala-Gln-Exatecan is conjugated to Ab via a covalent bond, the structural formula of the formed ADC is as shown below:

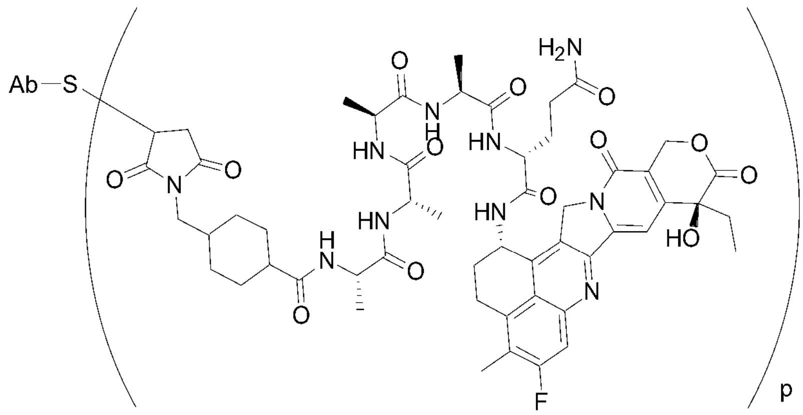

(IIi).

In the formed ADC above, the antibody Ab is linked to a carbon atom of the succinimide at the end of L-D via -S-, and the -S- is not a mercapto group additionally introduced into Ab, but a mercapto group contained in the antibody itself after the antibody Ab is reduced to break a disulfide bond.

In the present invention, the drug antibody ratio (DAR) refers to the number of drug molecules conjugated to the antibody (e.g., p in formula I). The number of drug molecules contained in the antibody drug conjugate described herein can be either an integer or a decimal. The number, whether an integer or a decimal, refers to the average number of drug molecules conjugated to each antibody. "p is any numerical value between 1 and 10" means that p may be either any integer between 1 and 10 (including endpoints 1 and 10) or any decimal between 1 and 10. Moreover, those skilled in the art can understand that even if the same preparation method is used, the DAR values of antibody drug conjugates prepared in different batches are not necessarily the same, for example, they can fluctuate within a range of no more than 0.5 up and down. For example, when p is 7.5, it means that the DAR value can fluctuate between 7.0 to 8.0.

The drug antibody ratio (DAR) can be determined by conventional means, such as mass spectrometry, ELISA assay, hydrophobic interaction chromatography (HIC), and HPLC. ADCs may also be measured for quantitative distribution in terms of p. In some cases, the separation of homogeneous ADCs with p as a certain numerical value from ADCs with other drug loads, followed by purification and verification can be achieved by means such as HIC, reversed-phase HPLC or electrophoresis.

In some embodiments, in the anti-GPC3 antibody, the sequences of CDR1, CDR2, and CDR3 in the heavy chain variable region are as set forth in SEQ ID NO: 1, SEQ ID NO: 2, and SEQ ID NO: 3, respectively, and the sequences of CDR1, CDR2, and CDR3 in the light chain variable region are as set forth in SEQ ID NO: 9, SEQ ID NO: 10, and SEQ ID NO: 11, respectively.

In some embodiments, FR1, FR2, FR3, and FR4 regions in the heavy chain variable region of the anti-GPC3 antibody comprise the sequences as set forth in SEQ ID NO: 4, SEQ ID NO: 5, SEQ ID NO: 6, and SEQ ID NO: 7, or mutants thereof, respectively.

In some embodiments, the sequences of FR1, FR2, FR3, and FR4 regions in the heavy chain variable region of the anti-GPC3 antibody are as set forth in SEQ ID NO: 4, SEQ ID NO: 5, SEQ ID NO: 6, and SEQ ID NO: 7, respectively.

In some embodiments, FR1, FR2, FR3, and FR4 regions in the light chain variable region of the anti-GPC3 antibody comprise the sequences as set forth in SEQ ID NO: 12, SEQ ID NO: 13, SEQ ID NO: 14, and SEQ ID NO: 15, or mutants thereof, respectively, or FR1, FR2, FR3, and FR4 regions in the light chain variable region of the anti-GPC3 antibody comprise the sequences as set forth in SEQ ID NO: 12, SEQ ID NO: 13, SEQ ID NO: 17, and SEQ ID NO: 15, or mutants thereof, respectively.

In some embodiments, the sequences of FR1, FR2, FR3, and FR4 regions in the light chain variable region of the anti-GPC3 antibody are as set forth in SEQ ID NO: 12, SEQ ID NO: 13, SEQ ID NO: 14, and SEQ ID NO: 15, respectively, or the sequences of FR1, FR2, FR3, and FR4 regions in the light chain variable region of the anti-GPC3 antibody are as set forth in SEQ ID NO: 12, SEQ ID NO: 13, SEQ ID NO: 17, and SEQ ID NO: 15, respectively.

In some embodiments, the heavy chain variable region of the anti-GPC3 antibody comprises the sequence as set forth in SEQ ID NO: 8 or a mutant thereof.

In some embodiments, the sequence of the heavy chain variable region of the anti-GPC3 antibody is as set forth in SEQ ID NO: 8.

In some embodiments, the light chain variable region of the anti-GPC3 antibody comprises the sequence as set forth in SEQ ID NO: 16 or SEQ ID NO: 18, or a mutant thereof.

In some embodiments, the sequence of the light chain variable region of the anti-GPC3 antibody is as set forth in SEQ ID NO: 16 or SEQ ID NO: 18.

In some embodiments, a heavy chain constant region of the anti-GPC3 antibody is selected from human IgG,

IgM, IgA, IgD, and IgE constant regions, or mutants of the constant regions. In some embodiments, the IgG is selected from IgG1, IgG2, IgG3, and IgG4.

In some embodiments, a light chain constant region of the anti-GPC3 antibody is selected from human lambda constant region or kappa constant region, or a mutant of the constant region.

In some embodiments, the heavy chain constant region of the anti-GPC3 antibody comprises the sequence as set forth in SEQ ID NO: 19, or a sequence with more than 70%, preferably more than 75%, 80%, 85%, 90%, 95%, or 99% homology with the sequence as set forth in SEQ ID NO: 19.

In some embodiments, the sequence of the heavy chain constant region of the anti-GPC3 antibody is as set forth in SEQ ID NO: 19.

In some embodiments, the light chain constant region of the anti-GPC3 antibody comprises the sequence as set forth in SEQ ID NO: 20, or a sequence with more than 70%, preferably more than 75%, 80%, 85%, 90%, 95%, or 99% homology with the sequence as set forth in SEQ ID NO: 20.

In some embodiments, the sequence of the light chain constant region of the anti-GPC3 antibody is as set forth in SEQ ID NO: 20.

In some embodiments, p is any numerical value between 2 and 8.

In some embodiments, p is any numerical value between 4 and 8 (e.g., 4.0, 7.9, or 8.0).

In a second aspect of the present invention, the present invention provides a pharmaceutical composition comprising the aforementioned antibody drug conjugate, or the pharmaceutically acceptable salt or solvate thereof, or the solvate of the salt.

In some embodiments, the pharmaceutical composition further comprises at least one pharmaceutical auxiliary material.

In some embodiments, the pharmaceutical composition further comprises at least one of a chemotherapeutic drug, an immunotherapeutic drug, and an immunosuppressant for treating a tumor.

In some embodiments, the chemotherapeutic drug is, for example, doxorubicin (Adriamycin), cyclophosphamide, a taxane (e.g., paclitaxel (Taxol) or docetaxel (Taxotere)), capecitabine (Xeloda), gemcitabine (Gemzar), vinorelbine (Navelbine), tamoxifen, an aromatase inhibitor (Arimidex, Femara, or Aromasin), 5-FU + folinic acid, irinotecan (Camptosar), oxaliplatin, cisplatin, carboplatin, estramustine, mitoxantrone (Novantrone), prednisone, vincristine (Oncovin), adriamycin, prednisone, etc., or a combination thereof.

In some embodiments, the immunotherapeutic drug is, for example, an anti-PD-1 monoclonal antibody (e.g., Pembrolizumab or Nivolumab), an anti-PD-L1 monoclonal antibody (e.g., Atezolizumab), an anti-TIGIT monoclonal antibody, an anti-4-1BB monoclonal antibody, an anti-VEGFR2 monoclonal antibody (e.g., Ramucirumab or apatinib), an anti-HER2 monoclonal antibody (e.g., Trastuzumab, Trastuzumab biosimilar, or Trastuzumab-dkst), etc., or a combination thereof.

In some embodiments, the immunosuppressant is selected from: (1) glucocorticoids, such as cortisone and prednisone; (2) microbial metabolites, such as cyclosporine and Fujimycin; (3) antimetabolites, such as azathioprine and 6-mercaptopurine; (4) polyclonal and monoclonal anti-lymphocyte antibodies, such as anti-lymphocyte globulin and OKT3; and (5) alkylating agents, such as cyclophosphamide. In some specific embodiments, the immunosuppressant is, for example, methylprednisolone, prednisone, azathioprine, Prograf, Zenapax, Simulect, cyclosporine, tacrolimus, rapamycin, mycophenolate mofetil, mizoribine, cyclophosphamide, or fingolimod.

In a third aspect of the present invention, the present invention provides the use of the aforementioned antibody drug conjugate, or the pharmaceutically acceptable salt or solvate thereof, or the solvate of the salt or the aforementioned pharmaceutical composition in the preparation of a drug for preventing and/or treating a GPC3 positivity-associated disease.

In a fourth aspect of the present invention, the present invention provides the aforementioned antibody drug conjugate, or the pharmaceutically acceptable salt or solvate thereof, or the solvate of the salt or the aforementioned pharmaceutical composition, for use in preventing and/or treating a GPC3 positivity-associated disease.

In a fifth aspect of the present invention, the present invention provides a method for treating and/or preventing a GPC3 positivity-associated disease, comprising: administering to a subject in need thereof a therapeutically and/or prophylactically effective amount of the aforementioned antibody drug conjugate, or the pharmaceutically acceptable salt or solvate thereof, or the solvate of the salt or the aforementioned pharmaceutical composition.

In some embodiments, the above GPC3 positivity-associated disease is selected from liver cancer, lung cancer, gastric cancer, head and neck cancer, esophageal cancer, Merkel cell cancer, liposarcoma, breast cancer, ovarian cancer, melanoma, squamous cell carcinoma, renal cell carcinoma, pancreatic cancer, prostate cancer, colorectal cancer, glioma, kidney cancer, bladder cancer, cervical cancer, gallbladder carcinoma, and glioblastoma.

In some embodiments, the above GPC3 positivity-associated disease is liver cancer.

In a sixth aspect of the present invention, the present invention provides a method for non-therapeutically inhibiting tumor angiogenesis, delaying tumor progression, inhibiting tumor growth or inhibiting tumor cell proliferation *in vitro*, comprising: contacting a tumor cell with the aforementioned antibody drug conjugate, or the pharmaceutically

acceptable salt or solvate thereof, or the solvate of the salt or the aforementioned pharmaceutical composition, wherein the tumor is a GPC3-expressing tumor.

In some embodiments, the GPC3-expressing tumor is selected from liver cancer, lung cancer, gastric cancer, head and neck cancer, esophageal cancer, Merkel cell cancer, liposarcoma, breast cancer, ovarian cancer, melanoma, squamous cell carcinoma, renal cell carcinoma, pancreatic cancer, prostate cancer, colorectal cancer, glioma, kidney cancer, bladder cancer, cervical cancer, gallbladder carcinoma, and glioblastoma.

In some embodiments, the GPC3-expressing tumor is liver cancer.

Beneficial Effects

1. The newly developed GPC3-ADCs of the present invention exhibit a strong cell killing effect in various tumor (e.g., liver cancer) cells.
2. The newly developed GPC3-ADCs of the present invention exhibit a significant pharmacodynamic effect on inhibiting tumor cell growth in various tumor models (e.g., a PDX tumor model of human liver cancer).

## Brief Description of the Drawings

Figure 1 shows the affinity of each GPC3 antibody for GPC3-CHO-K1 cells;
Figure 2 shows the affinity of each GPC3 antibody for HepG2 cells;
Figure 3 shows the affinity of each GPC3 antibody for Huh-7 cells;
Figure 4 shows the internalization of each GPC3 antibody on HepG2 cells;
Figure 5 shows a hydrophobic interaction chromatogram of Hu 52H5D3B8-8-mc-AAN-Exatecan (ADC-1);
Figure 6 shows a hydrophobic interaction chromatogram of Hu 52H5D3B8-8-mcc-AAN-Exatecan (ADC-2);
Figure 7 shows a hydrophobic interaction chromatogram of Hu 52H5D3B8-8-mc-AAQ-Exatecan (ADC-3);
Figure 8 shows a hydrophobic interaction chromatogram of Hu 52H5D3B8-8-mcc-AAQ-Exatecan (ADC-4);
Figure 9 shows a hydrophobic interaction chromatogram of Hu 52H5D3B8-7-mcc-AAQ-Exatecan (ADC-5);
Figure 10 shows a hydrophobic interaction chromatogram of Hu 52H5D3B8-8-mcc-AAQ-Exatecan (ADC-6);
Figure 11 shows a hydrophobic interaction chromatogram of Hu 52H5D3B8-7-mcc-AAQ-Exatecan (ADC-7);
Figure 12 shows a hydrophobic interaction chromatogram of Hu 52H5D3B8-8-mcc-AA-(3-cyano-L-alanine)-Exatecan (ADC-8);
Figure 13 shows a hydrophobic interaction chromatogram of Hu 52H5D3B8-8-mcc-AAAAQ-Exatecan (ADC-9);
Figure 14 shows the affinity of each GPC3 antibody and ADC for the Huh7 cells;
Figure 15 shows the result of endocytosis of the GPC3 antibody and each ADC on the Huh7 cells;
Figures 16-18 are representative diagrams of the killing effects of various GPC3-ADCs on the Huh7 cells;
Figure 19 shows the inhibitory activity of each GPC3-ADC on tumor growth in PDX model LIV#219 of nude mice with human liver cancer;
Figure 20 shows the effect of each GPC3-ADC on the animal body weight of PDX model LIV#219 of nude mice with human liver cancer;
Figure 21 shows the inhibitory activity of each GPC3-ADC on tumor growth in PDX model LI6653 of nude mice with human liver cancer; and
Figure 22 shows the effect of each GPC3-ADC on the animal body weight of PDX model LI6653 of nude mice with human liver cancer.

## Detailed Description of Embodiments

Embodiments of the present invention will be described in detail below with reference to examples; however, those skilled in the art will understand that the following examples are only used to illustrate the present invention and should not be regarded as limiting the scope of the present invention. For examples in which specific conditions are not specified, they are carried out according to the conventional conditions or the conditions recommended by the manufacturer. The reagents or instruments used without indicating the manufacturer are all commercially available conventional products.

In the present invention, the scientific and technical terms used herein have the meanings that are commonly understood by those skilled in the art unless otherwise specified. In addition, the terms and laboratory operation steps related to the protein and nucleic acid chemistry, molecular biology, cell and tissue culture, microbiology and immunology

used herein are all terms and conventional steps that are widely used in the corresponding art. Moreover, for better understanding of the present invention, definitions and explanations of related terms are provided below.

In the present invention, any numerical range should be interpreted as including any value within the range or any subrange unless otherwise specified.

In the present invention, the term "antibody" refers to an immunoglobulin molecule that generally consists of two pairs of identical polypeptide chains (each pair has one "light" (L) chain and one "heavy" (H) chain). Antibody light chains can be divided into two categories, i.e., κ and λ. Heavy chains can be divided into five types, i.e., μ, δ, γ, α, or ε. According to different heavy chains, antibodies can be divided into five categories, i.e., IgM, IgD, IgG, IgA, and IgE. Within light and heavy chains, the variable and constant regions are joined by a "J" region of about 12 or more amino acids, with the heavy chain further comprising a "D" region of about 3 or more amino acids. Each heavy chain consists of a heavy chain variable region ($V_H$) and a heavy chain constant region ($C_H$). The heavy chain constant region consists of three domains ($C_H1$, $C_H2$, and $C_H3$). Each light chain consists of a light chain variable region ($V_L$) and a light chain constant region ($C_L$). The light chain constant region consists of one domain, i.e., $C_L$. The constant regions of the antibodies can mediate the binding of the immunoglobulin to host tissues or factors, including various cells of the immune system (e.g., effector cells) and component C1q of the complement system. The $V_H$ and $V_L$ regions can be further subdivided into regions of hypervariability, termed complementarity determining regions (CDRs), interspersed with regions that are more conserved, termed framework regions (FRs). Each $V_H$ and $V_L$ consists of three CDRs and four FRs, arranged from amino-terminus to carboxy-terminus in the following order: FR1, CDR1, FR2, CDR2, FR3, CDR3, FR4. The variable regions ($V_H$ and $V_L$) of each heavy/light chain pair form antibody binding sites, respectively. Distribution of amino acids in various regions or domains follows the definitions in: Kabat Sequences of Proteins of Immunological Interest (National Institutes of Health, Bethesda, Md. (1987 and 1991)), or Chothia & Lesk (1987) J. Mol. Biol. 196:901-917; and Chothia et al. (1989) Nature 342:878-883.

In the present invention, a "humanized" antibody refers to a non-human (e.g., mouse) antibody format, which is a chimeric immunoglobulin, immunoglobulin chain, or a fragment thereof (e.g., Fv, Fab, Fab', F(ab')2, or other antigen binding subsequences of the antibody) and contains minimal sequence derived from non-human immunoglobulin. Preferably, the humanized antibody is a human immunoglobulin (recipient antibody) in which a residue in the complementarity determining region (CDR) of the recipient antibody is replaced by a CDR residue from a non-human species (donor antibody) such as mouse, rat, or rabbit having the desired specificity, affinity, and capacity.

In addition, during humanization, it is also possible to mutate amino acid residues in CDR1, CDR2 and/or CDR3 regions of VH and/or VL, thereby improving one or more binding properties (e.g., affinity) of the antibody. Mutations can be introduced, for example, by PCR-mediated mutations, the effect of which on antibody binding or other functional properties can be evaluated using *in vitro* or *in vivo* assays as described herein. Generally, conservative mutations are introduced. Such mutations may be amino acid substitutions, additions or deletions. In addition, there are generally no more than one or two mutations within a CDR.

In the present invention, the term "antigen binding fragment" of an antibody refers to a polypeptide comprising a fragment of a full-length antibody that retains the ability to specifically bind to the same antigen to which the full-length antibody binds and/or competes with the full-length antibody for specific binding to the antigen, which is also referred to as "antigen binding moiety". See generally, Fundamental Immunology, Ch. 7, Paul, W., ed., 2nd ed., Raven Press, N.Y. (1989), which is incorporated herein by reference in its entirety for all purposes. An antigen binding fragment of an antibody can be generated by recombinant DNA techniques or by enzymatic or chemical cleavage of an intact antibody. Non-limiting examples of the antigen binding fragment include Fab, Fab', $F(ab')_2$, Fd, Fv, a complementarity determining region (CDR) fragment, scFv, a diabody, a single domain antibody, a chimeric antibody, a linear antibody, a nanobody (with technology from Domantis), a probody, and such a polypeptide that comprises at least a portion of an antibody which is sufficient to confer the polypeptide with specific antigen binding capability. Engineered antibody variants are reviewed in Holliger et al., 2005; Nat Biotechnol, 23: 1126-1136.

In the present invention, the term "Fd" means an antibody fragment consisting of VH and CH1 domains; the term "Fab fragment" means an antibody fragment consisting of VL, VH, CL, and CH1 domains; the term "$F(ab')_2$ fragment" means an antibody fragment comprising two Fab fragments linked via a disulfide bridge on the hinge region; and the term "Fab' fragment" means a fragment obtained by reducing the disulfide bond linking two heavy chain fragments in the $F(ab')_2$ fragment, and which consists of an entire light chain and an Fd fragment of a heavy chain (consisting of VH and CH1 domains).

In the present invention, the term "Fv" means an antibody fragment consisting of VL and VH domains of a single arm of an antibody. The Fv fragment is generally considered as the minimum antibody fragment that can form a complete antigen binding site. Generally, it is believed that the six CDRs confer an antibody with antigen binding specificity. However, even a variable region (e.g., an Fd fragment, which contains only three CDRs specific for an antigen) is capable of recognizing and binding to an antigen, although its affinity may be lower than that of a complete binding site.

In the present invention, the term "scFv" refers to a single polypeptide chain comprising VL and VH domains, wherein the VL and VH are linked via a linker (see, e.g., Bird et al., Science 242:423-426 (1988); Huston et al., Proc. Natl.

Acad. Sci. USA 85:5879-5883 (1988); and Pluckthun, The Pharmacology of Monoclonal Antibodies, vol. 113, Roseburg and Moore eds., Springer-Verlag, New York, pp. 269-315 (1994)). Such scFv molecules may have a general structure of $NH_2$-VL-linker-VH-COOH or $NH_2$-VH-linker-VL-COOH. Suitable linkers in the prior art consist of a repeated GGGGS amino acid sequence or a variant thereof. For example, a linker having an amino acid sequence $(GGGGS)_4$ can be used, and a variant thereof can also be used (Holliger et al. (1993), Proc. Natl. Acad. Sci. USA 90: 6444-6448). Other linkers which can be used in the present invention are described in Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between the VH and VL of the scFv. In certain embodiments of the present invention, the scFv may form a di-scFv, which refers to an antibody formed by two or more single scFvs in tandem. In certain embodiments of the present invention, the scFv may form an $(scFv)_2$, which refers to an antibody formed by two or more single scFvs in parallel.

In the present invention, the term "diabody" means an antibody in which VH and VL domains are expressed on a single polypeptide chain; however, the linker used is too short to allow pairing between the two domains of the same chain, thereby forcing the domains to pair with the complementary domains of another chain and producing two antigen binding sites (see, e.g., Holliger P. et al., Proc. Natl. Acad. Sci. USA 90:6444-6448 (1993); and Poljak R. J. et al., Structure 2:1121-1123 (1994)).

In the present invention, the term "single-domain antibody (sdAb)" has the meaning commonly understood by those skilled in the art, and refers to an antibody fragment consisting of a single monomeric variable antibody domain (e.g., a single heavy chain variable region), and retaining the ability to specifically bind to the same antigen to which the full-length antibody binds. The single-domain antibody is also referred to as nanobody.

In the present invention, the term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

Each of the above antibody fragments retains the ability to specifically bind to the same antigen to which the full-length antibody binds and/or competes with the full-length antibody for specific binding to the antigen.

An antigen binding fragment of an antibody (e.g., the above antibody fragment) can be obtained from a given antibody (e.g., the antibody provided by the present invention) by using conventional techniques known to those skilled in the art (e.g., recombinant DNA techniques or enzymatic or chemical cleavage), and the antigen binding fragment of the antibody can be screened for specificity in the same manner as for an intact antibody.

The antigen binding fragments of the present invention can be obtained by hydrolysis of intact antibody molecules (see Morimoto et al., J. Biochem. Biophys. Methods 24:107-117 (1992); and Brennan et al., Science 229:81 (1985)). In addition, these antigen binding fragments can also be produced directly from recombinant host cells (see Hudson, Curr. Opin. Immunol. 11: 548-557 (1999); and Little et al., Immunol. Today, 21: 364-370 (2000)). For example, Fab' fragments can be obtained directly from the host cells; and $F(ab')_2$ fragments can be formed by chemical coupling of Fab' fragments (Carter et al., Bio/Technology, 10: 163-167 (1992)). Moreover, Fv, Fab, or $F(ab')_2$ fragments can also be obtained directly by direct isolation from recombinant host cell cultures. Other techniques for preparing these antigen binding fragments are fully known to those of ordinary skill in the art.

In the present invention, algorithms for determining the percentage of sequence homology and sequence similarity are, for example, BLAST and BLAST 2.0 algorithms, which are described in Altschul et al. (1977) Nucl. Acid. Res. 25:3389-3402 and Altschul et al. (1990) J. Mol. Biol. 215:403-410, respectively. BLAST and BLAST 2.0 can be used to determine the percentage of amino acid sequence homology of the present invention, using parameters such as those described in the literatures or default parameters. Software for performing BLAST analyses is publicly available through the National Center for Biotechnology Information (NCBI).

In the present invention, the amino acid sequence having at least 70% sequence homology with an amino acid sequence includes a polypeptide sequence essentially identical to the amino acid sequence, e.g., those sequences comprising at least 70% sequence homology, preferably at least 75%, 80%, 85%, 86%, 87%, 88%, 89%, 90%, 91%, 92%, 93%, 94%, 95%, 96%, 97%, 98%, 99%, or more sequence homology with the polypeptide sequence of the present invention when using the method described herein (e.g., BLAST analyses using standard parameters).

In the present invention, a mutant of the amino acid sequence refers to a sequence having more than 70%, for example, more than 75%, 80%, 85%, 90%, 91%, 92%, 93%, 94%, 95%, 96%, 97%, 98%, or 99% homology with the amino acid sequence, e.g., a sequence with three, two, or one substituted, deleted or added amino acids. Preferably, no more than three amino acids are substituted, added or deleted. More preferably, no more than two amino acids are substituted, added or deleted. Most preferably, no more than one amino acid is substituted, added or deleted.

A "substitution" variant is a variant in which at least one amino acid residue in the natural sequence is removed and a different amino acid is inserted in the same position therein. The substitution may be single, wherein only one amino acid in the molecule is substituted; or may be multiple, wherein two or more amino acids in the same molecule are substituted. The multiple substitutions may be at consecutive sites. Similarly, an amino acid can be substituted by multiple

residues, wherein such variants comprise both substitutions and insertions. An "insertion" (or "addition") variant is a variant in which one or more amino acids are inserted immediately adjacent to amino acids at a specific position in a natural sequence. Immediately adjacent to an amino acid means linked to either the $\alpha$-carboxyl or $\alpha$-amino functional group of the amino acid. A "deletion" variant is a variant in which one or more amino acids in the natural amino acid sequence are removed. Generally, a deletion variant has one or two amino acids deleted in a specific region of its molecule.

In certain embodiments, fewer than the theoretical maximum number of drug modules are conjugated to the antibody in the conjugation reaction. Generally, the antibody does not contain many free and reactive cysteine thiol groups, which can link drug modules; and in fact, most cysteine thiol groups in the antibody exist as disulfide bridges. In certain embodiments, the antibody can be reduced with a reducing agent such as dithiothreitol (DTT) or tris(2-carboxyethyl) phosphine (TCEP) under partial or complete reducing conditions to produce reactive cysteine thiol groups.

In the present invention, the term "pharmaceutically acceptable salt" refers to (i) salts formed from acidic functional groups present in the conjugate provided by the present invention and appropriate inorganic or organic cations (bases), including but not limited to alkali metal salts, such as sodium salts, potassium salts, and lithium salts; alkaline earth metal salts, such as calcium salts and magnesium salts; other metal salts, such as aluminum salts, iron salts, zinc salts, copper salts, nickel salts, and cobalt salts; inorganic base salts, such as ammonium salts; and organic base salts, such as tert-octylamine salts, dibenzylamine salts, morpholine salts, glucosamine salts, alkyl phenylglycinate salts, ethylenediamine salts, N-methylglucosamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenylethylamine salts, piperazine salts, tetramethylamine salts, and tris(hydroxymethyl)aminomethane salts; and (ii) salts formed from basic functional groups present in the conjugate provided by the present invention and appropriate inorganic or organic anions (acids), including but not limited to hydrohalides, such as hydrofluorides, hydrochlorides, hydrobromides, and hydroiodides; inorganic acid salts, such as nitrates, perchlorates, sulfates, and phosphates; lower alkane sulfonates, such as methanesulfonates, trifluoromethanesulfonates, and ethanesulfonates; arylsulfonates, such as benzenesulfonates and p-benzenesulfonates; organic acid salts, such as acetates, malates, fumarates, succinates, citrates, tartrates, oxalates, and maleates; and amino acid salts, such as glycine salts, trimethylglycine salts, arginine salts, ornithine salts, glutamic acid salts, and aspartic acid salts.

Pharmaceutically acceptable salts can be obtained using standard procedures well known in the art, for example, by reacting a sufficient amount of a basic substance with a suitable acid providing a pharmaceutically acceptable anion, or by reacting a sufficient amount of an acidic substance with a suitable base providing a pharmaceutically acceptable cation.

In the present invention, a solvate refers to the following form of the antibody drug conjugate of the present invention: a solid or liquid complex formed by coordination of the antibody drug conjugate with solvent molecules. A hydrate is a specific form of solvate, which has coordinated water molecules. In the present invention, the hydrate is a preferred solvate.

Methods for preparing various pharmaceutical compositions containing certain amounts of active ingredients are known or apparent to those skilled in the art according to the invention of the present invention. As described in REMINGTON'S PHARMACEUTICAL SCIENCES, Martin, E.W., ed., Mack Publishing Company, 19th ed. (1995), a method for preparing the pharmaceutical composition comprises incorporating appropriate pharmaceutical excipients, carriers, diluents, etc., which are non-toxic to cells or mammals exposed thereto at the used dosage and concentration.

In the present invention, pharmaceutical auxiliary materials refer to excipients and additives used during drug production and prescription deployment, and refer to substances that have been reasonably evaluated in terms of safety except active ingredients and are included in pharmaceutical preparation. Pharmaceutical auxiliary materials not only form dosage forms, act as carriers, and enhance stability, but also have important functions such as solubilization, dissolution enhancement, and sustained and controlled release. They are important ingredients that may affect the quality, safety, and efficacy of drug products. According to the sources thereof, they can be divided into natural, semi-synthetic, and fully synthetic. According to the functions and uses thereof, they can be divided into: solvents, propellants, solubilizers, cosolvents, emulsifiers, colorants, binders, disintegrants, fillers, lubricants, wetting agents, osmotic pressure regulators, stabilizers, glidants, correctants, preservatives, suspending agents, coating materials, fragrances, anti-adhesives, antioxidants, chelating agents, penetration enhancers, pH regulators, buffers, plasticizers, surfactants, foaming agents, defoamers, thickeners, inclusion agents, humectants, absorbents, diluents, flocculants and deflocculants, filter aids, release retardants, etc. According to the routes of administration thereof, they can be divided into pharmaceutical auxiliary materials given via oral administration, injection, mucosal administration, transdermal or local administration, nasal or oral inhalation administration, ocular administration, etc. The same pharmaceutical auxiliary material can be used in pharmaceutical preparations for different routes of administration and have different functions and uses.

In the present invention, the pharmaceutical composition can be prepared into various suitable dosage forms according to the route of administration. Examples are tablets, capsules, granules, oral solutions, oral suspensions, oral emulsions, powders, tinctures, syrups, injections, suppositories, ointments, creams, pastes, ophthalmic preparations, pills, implants, aerosols, inhalation powders, sprays, etc. The pharmaceutical composition or suitable dosage form can contain 0.01 mg to 1000 mg of the antibody drug conjugate, or the pharmaceutically acceptable salt or solvate thereof, or

the solvate of the salt of the present invention.

As used herein, the term "treatment" generally refers to obtaining a desired pharmacological and/or physiological effect. The effect may be prophylactic on the basis of the complete or partial prevention of a disease or symptoms thereof; and/or the effect may be therapeutic on the basis of the partial or complete stabilization or cure of the disease and/or side effects due to the disease. As used herein, "treatment" covers any treatment of a disease in a patient, including: (a) prevention of a disease or symptom in a patient who is susceptible to the disease or symptom but has not been diagnosed with the disease; (b) suppressing a symptom of a disease, i.e., preventing the development thereof; or (c) relieving a symptom of a disease, i.e., causing the disease or symptom to resolve.

In the present invention, a "subject" refers to a vertebrate. In certain embodiments, a vertebrate refers to a mammal. Mammals include, but are not limited to, livestock (such as cattle), pets (such as cats, dogs, and horses), primates, mice, and rats. In certain embodiments, a mammal refers to a human.

In the present invention, an "effective amount" refers to an amount effective, at dosages and for periods of time necessary, to achieve a desired therapeutic or prophylactic effect. A "therapeutically effective amount" of the substance/molecule of the present invention may vary depending on factors such as the disease state, age, sex and body weight of the individual and the ability of the substance/molecule to elicit a desired response in the individual. The therapeutically effective amount further covers an amount in which the therapeutically beneficial effect of the substance/molecule outweighs any toxic or harmful consequences. A "prophylactically effective amount" refers to an amount effective, at dosages and for periods of time necessary, to achieve a desired prophylactic effect. Usually but not necessarily, since the prophylactic dose is given to the subject before the onset of the disease or at the early stage of the disease, the prophylactically effective amount will be lower than the therapeutically effective amount. In the case of cancer, the therapeutically effective amount of a drug can reduce the number of cancer cells; reduce the tumor volume; inhibit (i.e., slow down to some extent, preferably stop) the infiltration of cancer cells into surrounding organs; inhibit (i.e., slow down to some extent, preferably stop) tumor metastasis; inhibit tumor growth to some extent; and/or alleviate one or more symptoms related to cancer to some extent.

In the present invention, 20 conventional amino acids and abbreviations thereof follow conventional usages. See Immunology-A Synthesis (2nd ed., E.S. Golub and D.R. Gren, Eds., Sinauer Associates, Sunderland, Mass. (1991)), which is incorporated herein by reference.

The present invention will be further explained with reference to specific examples below, and these examples do not limit the scope of the present invention.

Example 1. Preparation, sequence, and characterization of humanized GPC3 antibody

I. Preparation of humanized GPC3 antibody

In order to obtain mouse monoclonal antibodies targeting human GPC3, BALB/c mice and C57BL/6 mice were immunized with human GPC3 protein, and the antibody titer in serum was detected by using ELISA method (human GPC3 protein) and FACS method (CHO-K1 cells overexpressing human GPC3). After several rounds of immunization, mice with a higher titer were selected to establish an anti-human GPC3 hybridoma cell line, and positive clones were identified by means of ELISA method and FACS method. Subsequently, subclones were screened and identified, and monoclonal 52H5D3B8 was obtained. By means of an antibody humanization design, novel humanized antibodies Hu 52H5D3B8-7 and Hu 52H5D3B8-8 were finally obtained.

II. Sequence and characterization of humanized GPC3 antibody

1. Antibody sequence

Hu 52H5D3B8-7:

VH:

EVQLVQSGAEVKKPGASVKVSCKASGYTFTDYEMHWVRQAPGQGL
EWMGAIHPGSGGTAYNQKFKGRVTMTADKSISTAYMELSRLRSDDTAVY
YCTRFYSYAYWGQGTLVTVSS (SEQ ID NO: 8)

wherein the underlined parts are CDR1 (SEQ ID NO: 1), CDR2 (SEQ ID NO: 2), and CDR3 (SEQ ID NO: 3) in

order from left to right; and
the non-underlined parts are FR1 (SEQ ID NO: 4), FR2 (SEQ ID NO: 5), FR3 (SEQ ID NO: 6), and FR4 (SEQ ID NO: 7) in order from left to right.

VL:

DVVMTQTPLSLSVTPGQPASISCRSSQSLVHSNGNTYLQWYLQKPGQSPQLLIYKVSNRFSGVPDRFSGSGSGTDFTLKISRVEAEDVGVYYCSQSIHVPYTFGGGTKVEIK (SEQ ID NO: 16)

wherein the underlined parts are CDR1 (SEQ ID NO: 9), CDR2 (SEQ ID NO: 10), and CDR3 (SEQ ID NO: 11) in order from left to right; and
the non-underlined parts are FR1 (SEQ ID NO: 12), FR2 (SEQ ID NO: 13), FR3 (SEQ ID NO: 14), and FR4 (SEQ ID NO: 15) in order from left to right.

Hu 52H5D3B8-8:

VH:

EVQLVQSGAEVKKPGASVKVSCKASGYTFTDYEMHWVRQAPGQGLEWMGAIHPGSGGTAYNQKFKGRVTMTADKSISTAYMELSRLRSDDTAVYYCTRFYSYAYWGQGTLVTVSS (SEQ ID NO: 8)

wherein the underlined parts are CDR1 (SEQ ID NO: 1), CDR2 (SEQ ID NO: 2), and CDR3 (SEQ ID NO: 3) in order from left to right; and
the non-underlined parts are FR1 (SEQ ID NO: 4), FR2 (SEQ ID NO: 5), FR3 (SEQ ID NO: 6), and FR4 (SEQ ID NO: 7) in order from left to right.

VL:

DVVMTQTPLSLSVTPGQPASISCRSSQSLVHSNGNTYLQWYLQKPGQSPQLLIYKVSNRFSGVPDRFSGSGSGTDFTLKISRVEAEDVGVYFCSQSIHVPYTFGGGTKVEIK (SEQ ID NO: 18)

wherein the underlined parts are CDR1 (SEQ ID NO: 9), CDR2 (SEQ ID NO: 10), and CDR3 (SEQ ID NO: 11) in order from left to right; and
the non-underlined parts are FR1 (SEQ ID NO: 12), FR2 (SEQ ID NO: 13), FR3 (SEQ ID NO: 17), and FR4 (SEQ ID NO: 15) in order from left to right.

The heavy chain constant regions of Hu 52H5D3B8-7 and Hu 52H5D3B8-8 are both:

ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK (SEQ ID NO: 19)

The light chain constant regions of Hu 52H5D3B8-7 and Hu 52H5D3B8-8 are both:

RTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPV TKSFNRGEC (SEQ ID NO: 20)

Control antibody:

(1) GC33 antibody (Chugai Pharmaceutical)

The preparation of the GC33 antibody is described in US 7919086 B2.

The CDR information of the heavy chain and light chain of the GC33 antibody is as follows:

| Antibody | CDR | Amino acid sequence | SEQ ID NO |
|---|---|---|---|
| GC33 (H) | CDR1 | DYEMH | 21 |
| | CDR2 | ALDPKTGDTAYSQKFKG | 22 |
| | CDR3 | FYSYTY | 23 |
| GC33 (L) | CDR1 | RSSQSLVHSNGNTYLH | 24 |
| | CDR2 | KVSNRFS | 25 |
| | CDR3 | SQNTHVPPT | 26 |

The FR information of the heavy chain and light chain of the GC33 antibody is as follows:

| Antibody | FR | Amino acid sequence | SEQ ID NO |
|---|---|---|---|
| GC33 (H) | FR1 | QVQLVQSGAEVKKPGASVKVSCKASGYTFT | 27 |
| | FR2 | WVRQAPGQGLEWMG | 28 |
| | FR3 | RVTLTADKSTSTAYMELSSLTSEDTAVYYCTR | 29 |
| | FR4 | WGQGTLVTVSS | 30 |
| GC33 (L) | FR1 | DVVMTQSPLSLPVTPGEPASISC | 31 |
| | FR2 | WYLQKPGQSPQLLIY | 32 |
| | FR3 | GVPDRFSGSGSGTDFTLKISRVEAEDVGVYYC | 33 |
| | FR4 | FGQGTKLEIK | 34 |

(2) Y035 antibody (CARSGEN Therapeutics)

The preparation of the Y035 antibody is described in CN 106397593 B. The information about the amino acid sequence of the variable regions of the heavy chain and light chain of the humanized Y035 antibody is as follows:

Humanized Y035 heavy chain variable region (SEQ ID NO: 42):

EVQLVQSGAEVKKPGASVKVSCKASGYTHSDYEMHWVRQAPGQGL
EWMGAIHPGSGDTAYNQRFKGRVTITADKSTSTAYMELSSLRSEDTAVYY
CARFYSYAYWGQGTLVTVSA

wherein the underlined parts are CDR1 (SEQ ID NO: 35), CDR2 (SEQ ID NO: 36), and CDR3 (SEQ ID NO: 37) in order from left to right; and
the non-underlined parts are FR1 (SEQ ID NO: 38), FR2 (SEQ ID NO: 39), FR3 (SEQ ID NO: 40), and FR4 (SEQ ID NO: 41) in order from left to right.

Humanized Y035 light chain variable region (SEQ ID NO: 50):

DIVMTQTPLSLPVTPGEPASISCRSSQSLVHSNGNTYLQWYLQWYLQK
PGQSPQLLIYKVSNRFSGVPDRFSGSGSGTDFTLKISRVEAEDVGVYYCSQS
IYVPYTFGQGTKLEIKR

wherein the underlined parts are CDR1 (SEQ ID NO: 43), CDR2 (SEQ ID NO: 44), and CDR3 (SEQ ID NO: 45) in order from left to right; and
the non-underlined parts are FR1 (SEQ ID NO: 46), FR2 (SEQ ID NO: 47), FR3 (SEQ ID NO: 48), and FR4 (SEQ ID NO: 49) in order from left to right.

The heavy chain constant regions of the GC33 antibody and the Y035 antibody are both:

ASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNG
KEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRW
QQGNVFSCSVMHEALHNHYTQKSLSLSPGK (SEQ ID NO: 19)

The light chain constant regions of the GC33 antibody and the Y035 antibody are both:

RTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPV
TKSFNRGEC (SEQ ID NO: 20)

2. Binding of humanized GPC3 antibody to cells

The inventors evaluated the binding activities of Hu 52H5D3B8-7 and Hu 52H5D3B8-8 to CHO-K1 cells, HepG2

cells, and HuH-7 cells overexpressing human GPC3 by FACS, and made a comparison with the known anti-GPC3 antibodies GC33 and Y035. In order to evaluate the CDR regions of the two antibodies Hu 52H5D3B8-7 and Hu 52H5D3B8-8 and the FR regions of the antibody Hu 52H5D3B8-7, the inventors prepared two recombinant antibodies. "Hu 52H5D3B8-7/8 CDRs with Y035 FRs" means that the antibody is formed by recombining the CDR regions of the antibody Hu 52H5D3B8-7 or Hu 52H5D3B8-8 with the FR regions of the Y035 antibody. The antibody "Y035 CDRs with Hu 52H5D3B8-7 FRs" means that the antibody is formed by recombining the CDR regions of the Y035 antibody with the FR regions of the antibody Hu 52H5D3B8-7.

Firstly, the GPC3-CHO-K1 cells, HepG2 cells, and Huh-7 cells were incubated with an Fc blocker at 4°C for 15 minutes. The cells were washed with FACS buffer (PBS + 2% FBS), and 5-fold serially diluted solutions of Hu 52H5D3B8-7, Hu 52H5D3B8-8, Hu 52H5D3B8-7/8 CDRs with Y035 FRs, Y035 CDRs with Hu 52H5D3B8-7 FRs, or Y035 mAb (starting concentration 100 nM) were added to each well and incubated at 4°C for 60 minutes. The cells were washed with FACS buffer and fixed with 2% PFA for 15 minutes at room temperature. After washing with FACS buffer, Alexa Fluor® 647 AffiniPure goat anti-human IgG was added to each well and incubated at room temperature for 30 minutes. The sample was washed twice with FACS buffer. The cell sample was analyzed for fluorescence signal by flow cytometer MACSQuant Analyzer 16 and expressed by MFI (GeoMean fluorescence intensity). The experimental results are as shown in Figures 1-3, in which Hu 52H5D3B8-7 and Hu 52H5D3B8-8 had comparable binding affinities for the GPC3-CHO-K1 cells, HepG2 cells, and Huh-7 cells, which were higher than the other antibodies. In addition, both the antibody "Hu 52H5D3B8-7/8 CDRs with Y035 FRs" and the antibody "Y035 CDRs with Hu 52H5D3B8-7 FR" exhibited higher cell affinities than Y035, which means that the CDR regions of the antibodies Hu 52H5D3B8-7 and Hu 52H5D3B8-8 or the FR regions of the antibody Hu 52H5D3B8-7 are both superior to those of the Y035 antibody. The $EC_{50}$ values of each test antibody on the GPC3-CHO-K1 cells, HepG2 cells, and Huh-7 cells are as shown in Table 1 below.

Table 1: Affinities of each GPC3 antibody for GPC3-CHO-K1 cells, HepG2 cells, and Huh-7 cells ($EC_{50}$)

| Antibody name | $EC_{50}$ (nM) | | |
|---|---|---|---|
| | GPC3-CHO-K1 | HepG2 | Huh-7 |
| Hu 52H5D3B8-7 | 0.4459 | 0.4051 | 0.4637 |
| Hu 52H5D3B8-8 | 0.4106 | 0.3512 | 0.4177 |
| GC33 | 0.9556 | 0.7449 | 0.9528 |
| Y035 | 1.460 | 1.250 | 1.178 |
| Hu 52H5D3B8-7/8 CDRs with Y035 FRs | 0.7627 | 0.7687 | 0.7824 |
| Y035 CDRs with Hu 52H5D3B8-7 FRs | 0.9072 | 0.6107 | 0.6674 |

3. Cellular internalization of humanized GPC3 antibody

The internalization of Hu 52H5D3B8-7 and Hu 52H5D3B8-8 on the HepG2 cells was evaluated and compared with the known GPC3 antibody Y035 (CARSGEN Therapeutics) for internalization. In addition, in order to evaluate the CDRs and FRs of the antibodies Hu 52H5D3B8-7 and Hu 52H5D3B8-8, the inventors also evaluated the antibody "Hu 52H5D3B8-7/8 CDRs with Y035 FRs" and the antibody "Y035 CDRs with Hu 52H5D3B8-7 FRs" in an *in vitro* internalization experiment.

pHAb thiol dye is a pH sensor dye, which has very low fluorescence at pH > 7 and significantly increased fluorescence when it is internalized into endosomes or lysosomes (at pH 6.3 or 4.7, respectively). In brief, α-hIgG secondary antibody labeled with the pHAb thiol dye (10 μg/mL) was incubated with the antibody (20 μg/mL) for 30 minutes. After incubation, the mixed antibody was 2-fold serially diluted, and the diluted mixture was added to each well in a 96-well assay plate pre-seeded with $2 \times 10^4$ HepG2 cells. After 48 hours of culture, the fluorescence signal was captured on a multimode reader (Envision® 2105). The experimental results are as shown in Figure 4, in which both Hu 52H5D3B8-7 and Hu 52H5D3B8-8 showed higher internalization effects than the other antibodies. In addition, the antibody "Hu 52H5D3B8-7/8 CDRs with Y035 FRs" showed a higher internalization effect (a higher Top value and comparable $EC_{50}$) than Y035, which means that the CDRs of Hu 52H5D3B8-7 and Hu 52H5D3B8-8 are superior to those of Y035. The $EC_{50}$ and Top values of each test antibody are as shown in Table 2 below.

Table 2: Endocytosis ($EC_{50}$, Top) of each GPC3 antibody on HepG2 cells

| Antibody name | $EC_{50}$ (μg/mL) | Top |
|---|---|---|
| Hu 52H5D3B8-7 | 0.7195 | 58555 |

(continued)

| Antibody name | $EC_{50}$ (μg/mL) | Top |
|---|---|---|
| Hu 52H5D3B8-8 | 0.7805 | 62378 |
| Y035 | 1.078 | 51973 |
| Hu 52H5D3B8-7/8 CDRs with Y035 FRs | 1.142 | 59223 |
| Y035 CDRs with Hu 52H5D3B8-7 FRs | 1.107 | 52157 |

Example 2. Preparation of linker-payload

I. Preparation of mc-AAN-Exatecan

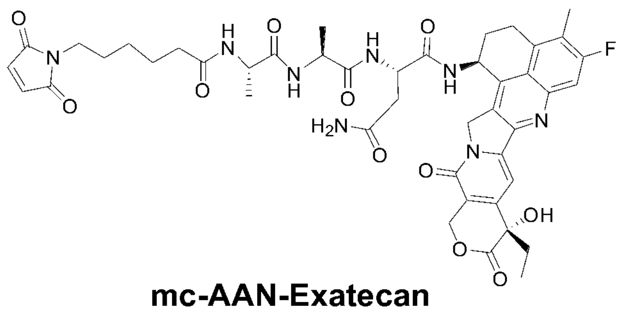

**mc-AAN-Exatecan**

1. Synthesis of Intermediate 1

After Fmoc-Ala-OH (N-fluorenylmethyloxycarbonyl-L-alanine, CAS No.: 35661-39-3) was activated with HOSu (N-hydroxysuccinimide, CAS No.: 6066-82-6), it was reacted with L-Ala (L-alanine, CAS No.: 56-41-7) to obtain Intermediate 1. The specific steps were as follows. Fmoc-Ala-OH (3 g, 1.0 eq.) and HOSu (1.45 g, 1.3 eq.) were added into a reaction flask, followed by 21 mL of THF. DCC (2.59 g, 1.3 eq.) was slowly added under stirring while controlling the temperature at room temperature. Then, the reaction was carried out at room temperature. The reaction was monitored by HPLC. After the reaction was completed, the reaction mixture was filtered, and the filter cake was rinsed with THF (6 mL). Purified water (15 mL) was added into the filtrate, followed by L-Ala (1.12 g, 1.3 eq.) and solid sodium bicarbonate (0.81 g, 1.0 eq.), and the mixture was stirred and reacted at room temperature. The reaction was monitored by HPLC. After the reaction was completed, citric acid (2.02 g, 1.0 eq.) was added, and the mixture was stirred. Then, the reaction mixture was extracted with ethyl acetate. The organic phase was concentrated, and DMF (12 mL) was added to dissolve the product. After filtering, prep-HPLC (preparative high performance liquid chromatography) was carried out. After the preparative solution was concentrated until no obvious droplets flowed out, it was extracted with ethyl acetate. The ethyl acetate phase was concentrated to dryness to obtain Intermediate 1. The reaction formula was as shown below:

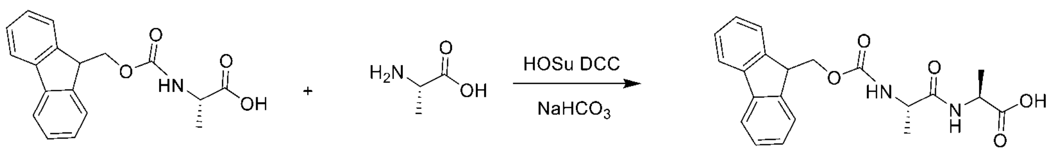


Fmoc-Ala-OH L-Ala Fmoc-Ala--Ala-OH

2. Synthesis of Intermediate 2

After Intermediate 1 (N-[fluorenylmethoxycarbonyl]-L-alanyl-L-alanine, CAS No.: 87512-31-0) was activated with HOSu (N-hydroxysuccinimide, CAS No.: 6066-82-6), it was reacted with L-Asn (L-asparagine, CAS No.: 70-47-3) to obtain Intermediate 2. The specifical steps were as follows. Intermediate 1 (0.87 g, 1.0 eq.), HOSu (0.34 g, 1.3 eq.), and THF (9 mL) were added into a reaction flask. DCC (0.61 g, 1.3 eq.) was slowly added under stirring while controlling the temperature at room temperature. The reaction was carried out at room temperature. The reaction was monitored by

HPLC. After the reaction was completed, the reaction mixture was filtered, and the filter cake was rinsed with THF (2 mL). Purified water (10 mL) was added into the filtrate, followed by L-Asn (0.34 g, 1.1 eq.) and solid sodium bicarbonate (0.19 g, 1.0 eq.), and the mixture was stirred and reacted at room temperature. The reaction was monitored by HPLC. After the reaction was completed, citric acid monohydrate (0.48 g, 1.0 eq.) was added, and the mixture was stirred. The reaction mixture was concentrated to remove most of the solvent, and the residue was subjected to preparation and purification. After the preparative solution was concentrated until no obvious droplets flowed out, it was extracted with ethyl acetate. The organic phase was concentrated to dryness to obtain Intermediate 2. The reaction formula was as shown below:

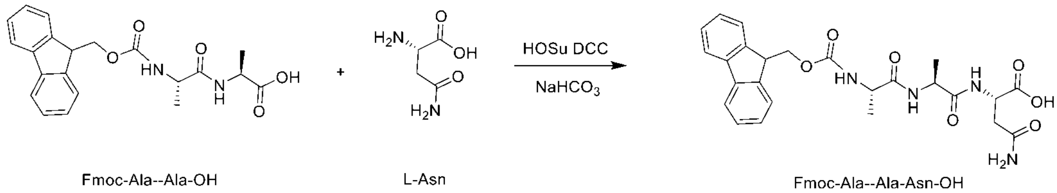


Fmoc-Ala--Ala-OH L-Asn Fmoc-Ala--Ala-Asn-OH

3. Synthesis of Intermediate 3

After the Fmoc group was removed from Intermediate 2 by adding DEA (diethylamine, CAS No.: 109-89-7), Intermediate 3 was obtained. The specific steps were as follows. Intermediate 2 (100 mg) and DMF (1.5 mL) were added into a reaction flask. DEA (300 μL) was added dropwise while controlling the temperature at room temperature. The reaction was carried out at room temperature. The reaction was monitored by HPLC until no Intermediate 2 remained. DMF was removed by concentration. DCM (4 mL) and purified water (4 mL) were added. After stirring, liquid separation was carried out. The aqueous phase was concentrated to dryness to obtain Intermediate 3. The reaction formula was as shown below:

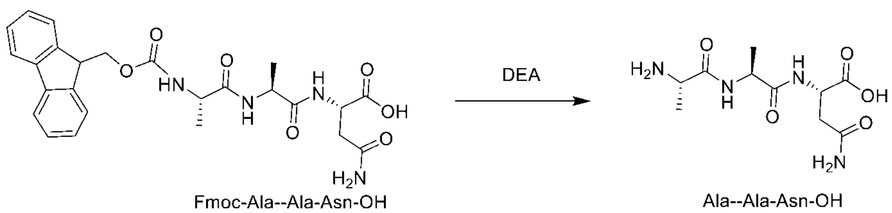


Fmoc-Ala--Ala-Asn-OH Ala--Ala-Asn-OH

4. Synthesis of Intermediate 4

Intermediate 3 was reacted with N-succinimidyl 6-maleimidohexanoate (CAS No.: 55750-63-5) to obtain Intermediate 4. The specific steps were as follows. Intermediate 3 (92 mg, 1.0 eq.), N-succinimidyl 6-maleimidohexanoate (135 mg, 1.3 eq.), DMF (1.5 mL), and DIPEA (0.059 mL, 1.0 eq.) were added into a reaction flask. The reaction was monitored by HPLC. After the reaction was completed, preparation and purification were carried out. The preparative solution was concentrated to obtain Intermediate 4. The reaction formula was as shown below:

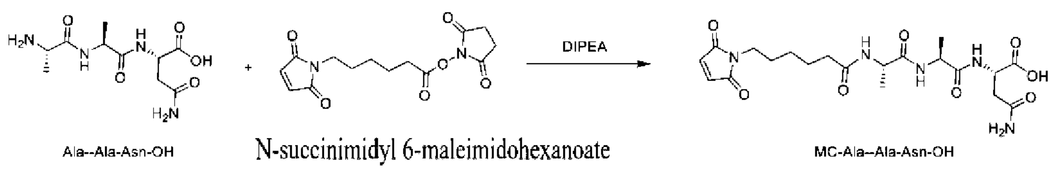


Ala--Ala-Asn-OH N-succinimidyl 6-maleimidohexanoate MC-Ala--Ala-Asn-OH

5. Synthesis of the product

Intermediate 4 was subjected to a condensation reaction with exatecan mesylate (CAS No.: 169869-90-3) to obtain the product. The specific steps were as follows. Intermediate 4 (26 mg, 1.0 eq.) was added into a reaction flask at room temperature, followed by DMF (1.5 mL). Exatecan mesylate (29.6 mg, 1.0 eq.), EEDQ (20.7 mg, 1.5 eq.), HATU (31.8 mg, 1.5 eq.), DMAP (0.7 mg, 0.1 eq.), and DIPEA (29.2 μL, 3.0 eq.) were added successively, and the reaction was carried out at room temperature. The reaction was monitored by HPLC. After the reaction was completed, preparation and purification were carried out. The preparative solution was concentrated to obtain the product. The reaction formula was as shown below:

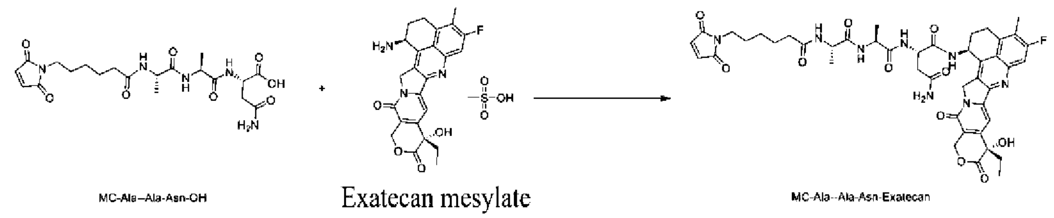

MC-Ala--Ala-Asn-OH Exatecan mesylate MC-Ala--Ala-Asn-Exatecan

$^1$H NMR (400 MHz, DMSO-$d_6$) $\delta$ 8.22-8.29 (d, $J$ =2.0 Hz, 1H), 7.94-8.05 (d, $J$ =2.0Hz, 3H), 7.83-7.88(d, $J$ =2.0 Hz,1H), 7.75-7.82(d, $J$ =2.0 Hz, 1H), 7.34-7.40 (m,1H), 7.28-7.33 (m,1H), 6.96-7.02(d, $J$ =8.0 Hz,2H), 6.87-6.94(m,1H), 5.42-5.55(m,1H), 5.41-5.46(m,2H), 5.21-5.26(m,1H), 4.41-4.49(m,1H), 4.00-4.12(m,2H), 3.32-3.40(m,2H), 3.12-3.17(m,2H), 2.86-2.96(m,2H), 2.71-2.76(m,2H), 2.36-2.43(m,2H), 2.14-2.24(m,1H), 1.97-2.07(m,3H), 1.81-1.93(m,2H), 1.36-1.52(m,4H), 1.05-1.25(m,8H), 0.83-0.93(m,3H).

II. Preparation of mc-AAQ-Exatecan

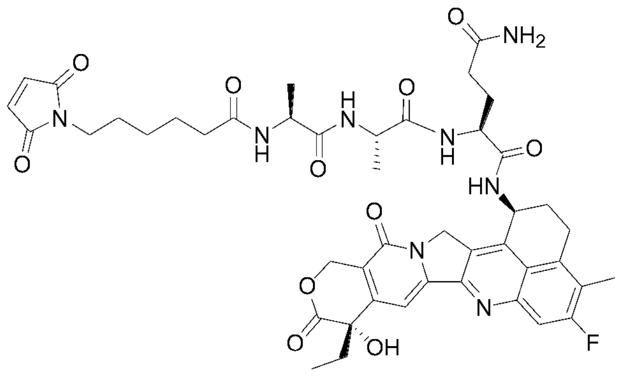

**mc-AAQ-Exatecan**

1. Synthesis of Intermediate 1, Fmoc-Gln-Exatecan

Fmoc-Gln-OH (N-fluorenylmethyloxycarbonyl-L-glutamine) (76.3 mg, 1.1 eq.) and Exatecan (100 mg, 1.0 eq.) were added into a reaction flask, followed by DMF (1 ml), DIEA (29 mg, 1.5 eq.), and TBTU (72.5 mg, 1.1 eq.), and the reaction was carried out at room temperature. The reaction was monitored by HPLC until no starting materials remained. After the reaction was completed, silica gel was added and thoroughly mixed with the sample. The mixture was concentrated, followed by purification through column chromatography. The product was collected and concentrated to dryness to obtain Intermediate 1.

The reaction formula was as shown below:

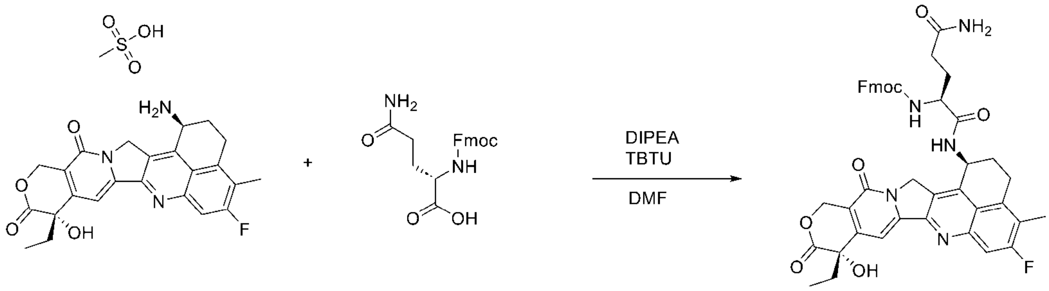


2. Synthesis of Intermediate 2, Gln-Exatecan

Intermediate 1, Fmoc-Gln-Exatecan (130 mg, 1.0 eq.) was added into a reaction flask, followed by DCM (2 ml). The mixture was stirred at room temperature, and failed to be dissolved and clarified. DEA (0.5 ml) was added, and the

reaction was carried out at room temperature. The reaction was monitored by HPLC until no starting materials remained. The reaction was stopped. MTBE (10 ml) was slowly added, so that a large amount of solid was precipitated out, and the mixture was stirred for 30 min. The solid was filtered to obtain an off-white solid, which was dried to obtain Intermediate 2.

The reaction formula was as shown below:

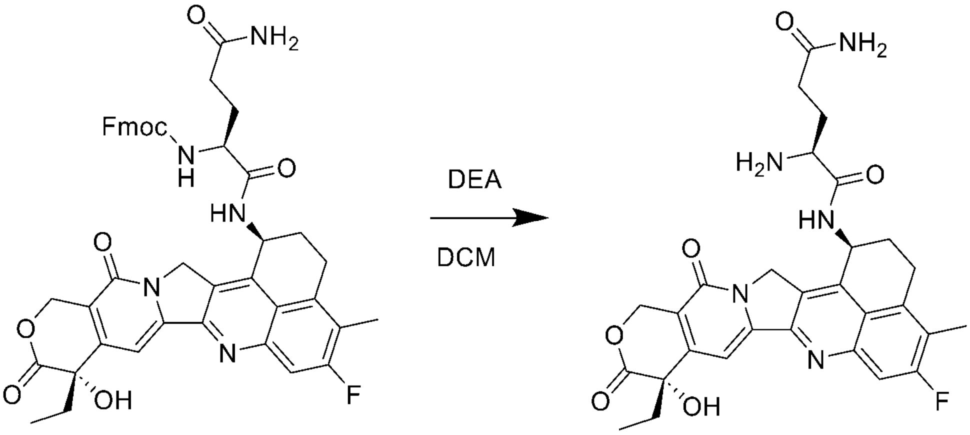


3. Synthesis of Intermediate 3, Fmoc-Ala-Ala-Gln-Exatecan

Intermediate 2, Gln-Exatecan (100 mg, 1.0 eq.) was added into a reaction flask, followed by DMF (1 mL), Fmoc-Ala-Ala-OH (67.8 mg, 1.0 eq.), TBTU (68.4 mg, 1.2 eq.), and DIEA (34.4 mg, 1.5 eq.), and the reaction was carried out at room temperature. The reaction was monitored by HPLC until no Intermediate 2 remained, and the reaction was stopped. Silica gel was added and thoroughly mixed with the sample. Purification was carried out by column chromatography. The product was collected to obtain Intermediate 3.

The reaction formula was as shown below:

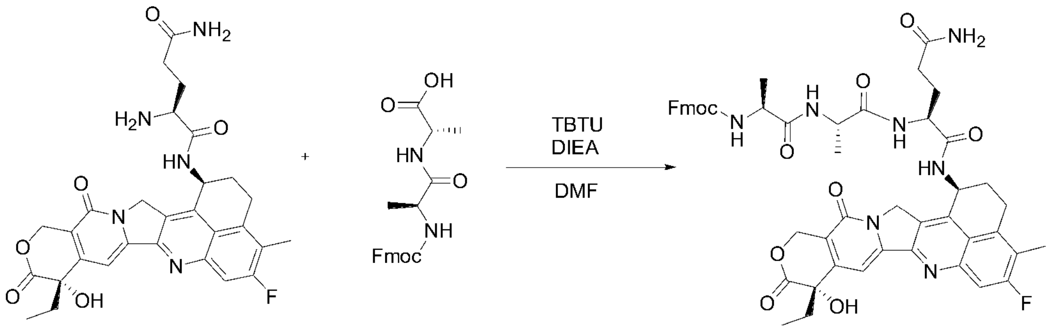


4. Synthesis of Intermediate 4, Ala-Ala-Gln-Exatecan

Intermediate 3, Fmoc-Ala-Ala-Gln-Exatecan (140 mg, 1.0 eq.) was added into a reaction flask, followed by DCM (2 ml) and DEA (0.5 ml), and the reaction was carried out at room temperature. The reaction was monitored by HPLC until no starting materials remained. The reaction was stopped. MTBE (10 ml) was slowly added, so that a large amount of solid was precipitated out, and the mixture was stirred for 30 min. The solid was filtered to obtain an off-white solid, which was dried to obtain Intermediate 4.

The reaction formula was as shown below:

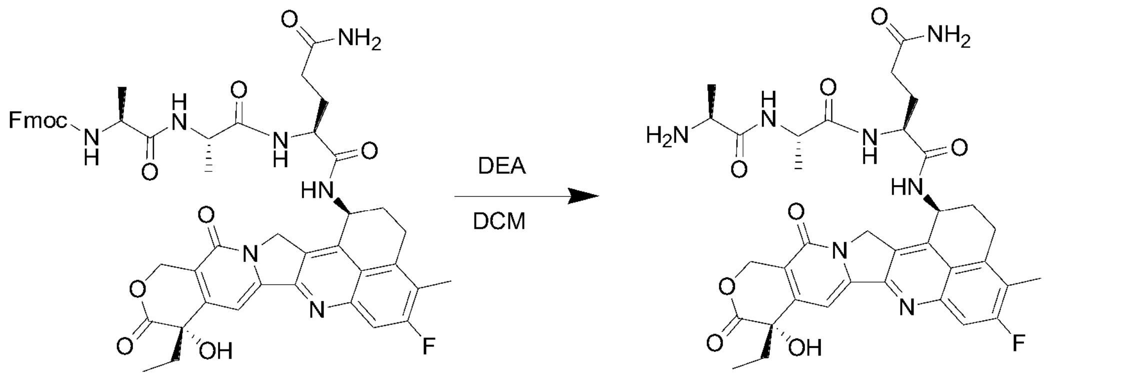


5. Synthesis of the product, mc-Ala-Ala-Gln-Exatecan

Intermediate 4, Ala-Ala-Gln-Exatecan (50 mg, 1.0 eq.) was added into a reaction flask, followed by DMF (1 mL), N-succinimidyl 6-maleimidohexanoate (21.8 mg, 1.1 eq.) and DIEA (12.1 mg, 1.5 eq.), and the reaction was carried out at room temperature. The reaction was monitored by HPLC until no Intermediate 4 remained, and the reaction was stopped. Preparation and purification were carried out. The product was collected and concentrated to dryness to obtain the product.

The reaction formula was as shown below:

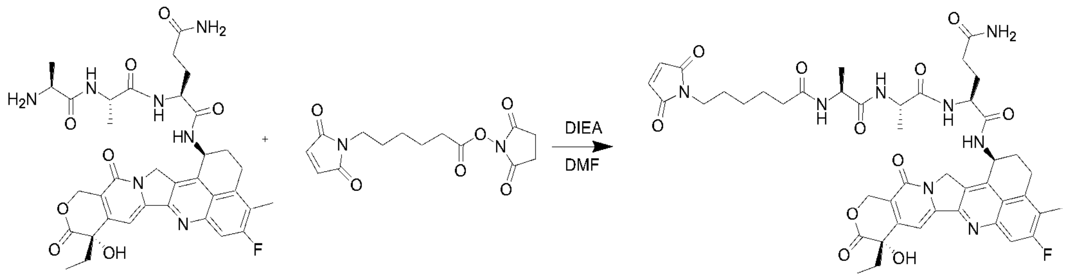


$^1$H NMR (400 MHz, DMSO-$d_6$) δ 8.28-8.36 (d, *J* =2.0 Hz, 1H), 7.90-7.96(d, *J* =2.0Hz, 2H), 7.75-7.87(d, *J* =2.0 Hz,2H), 7.27-7.33(d, *J* =2.0 Hz, 1H), 7.20-7.25 (m,1H), 6.97-7.03(d, *J* =8.0 Hz,2H), 6.69-6.75(m,1H), 6.49-6.53(m,1H), 5.46-5.55(m,1H), 5.40-5.45(m,2H), 5.24-5.34(m,1H), 5.04-5.14(m,1H), 4.31-4.38(m,1H), 4.02-4.23(m,2H), 3.86-3.96(m,1H), 3.40-3.49(m,1H), 3.33-3.40(m,2H), 3.12-3.20(m,1H), 2.36-2.42(m,2H), 2.15-2.25(m,1H), 2.00-2.12(m,4H), 1.71-1.93(m,3H), 1.36-1.52(m,4H), 1.01-1.20(m,10H), 0.83-0.92(m,3H).

III. Preparation of mcc-AAN-Exatecan

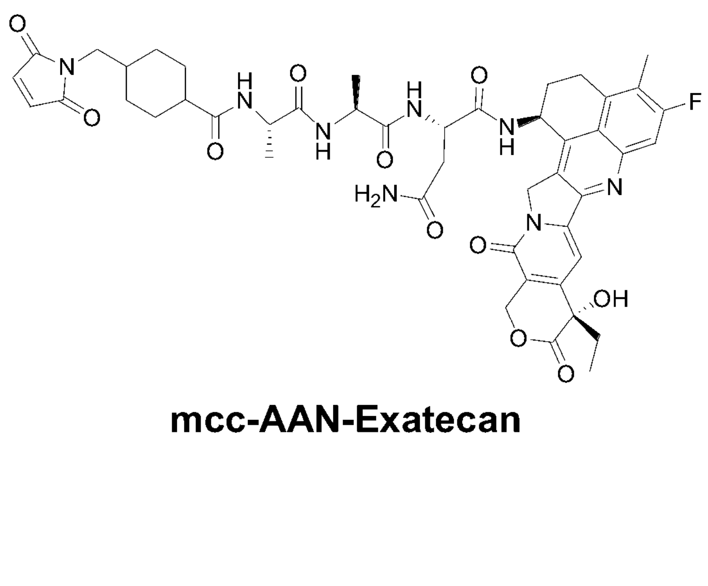

**mcc-AAN-Exatecan**

1. Synthesis of Intermediate 1

After Fmoc-Ala-Ala-OH (N-[fluorenylmethyloxycarbonyl]-L-alanyl-L-alanine) was activated with HOSu (N-hydroxysuccinimide), it was reacted with L-Asn (L-asparagine) to obtain Intermediate 1.

The specific steps were as follows. Fmoc-Ala-Ala-OH (1.0 eq.), HOSu (1.3 eq.), and THF (10 v/w) were added into a reaction flask at room temperature. DCC (1.3 eq.) was slowly added under stirring while controlling the temperature at room temperature. The reaction was carried out at room temperature. The reaction was monitored by HPLC until the residual amount of Fmoc-Ala-Ala-OH was low (using the area normalization method). The reaction mixture was filtered, and the filter cake was rinsed with THF (2.5 v/w). Purified water (11.5 v/w) was added into the filtrate, followed by L-Asn (1.1 eq.) and solid sodium bicarbonate (1.0 eq.), and the mixture was stirred and reacted at room temperature. The reaction was monitored by HPLC until the residual amount of Fmoc-Ala-Ala-OSu was low. Then, citric acid monohydrate (1.0 eq.) was added, and the mixture was stirred. The reaction mixture was concentrated to remove most of the solvent. DMF (4 v/w) was added to dissolve the product. After filtering, reverse-phase preparation was carried out by preparative liquid chromatography. After the preparative solution was concentrated until no obvious droplets flowed out, it was extracted with ethyl acetate. The ethyl acetate phase was concentrated to dryness to obtain Intermediate 1. The reaction formula was as shown below:

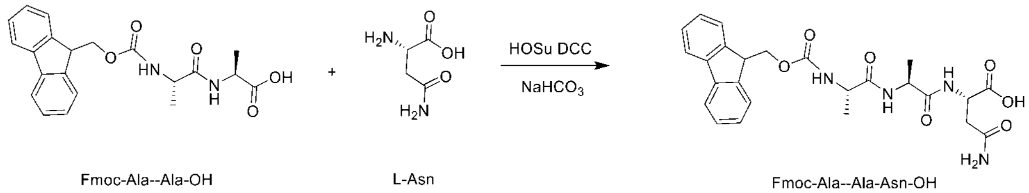


Fmoc-Ala--Ala-OH L-Asn Fmoc-Ala--Ala-Asn-OH

2. Synthesis of Intermediate 2

After the Fmoc group was removed from Intermediate 1 by adding DEA (diethylamine), Intermediate 2 was obtained.

The specific steps were as follows. Intermediate 1 (1.0 eq.) was added into a reaction flask at room temperature, followed by DMF (15 v/w). DEA (3 v/w) was added dropwise while controlling the temperature at room temperature. The reaction was carried out at room temperature. The reaction was monitored by HPLC until no Intermediate 1 remained. DMF was removed by concentration. DCM (40 v/w) and purified water (40 v/w) were added. After stirring, liquid separation was carried out. The aqueous phase was concentrated to dryness to obtain Intermediate 2. The reaction formula was as shown below:

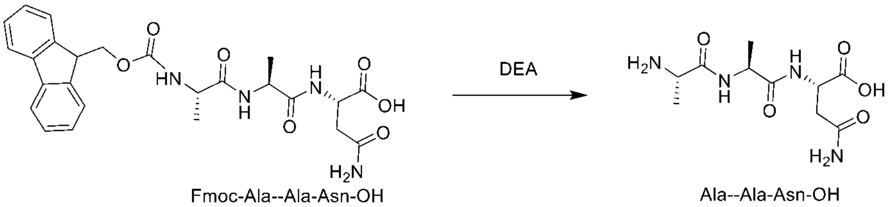


Fmoc-Ala--Ala-Asn-OH Ala--Ala-Asn-OH

3. Synthesis of Intermediate 3

Intermediate 2 was reacted with N-succinimidyl 4-(N-maleimidomethyl)cyclohexanecarboxylate to obtain Intermediate 3.

The specific steps were as follows. Intermediate 2 (1.0 eq.) and N-succinimidyl 4-(N-maleimidomethyl)cyclohexanecarboxylate (1.1 eq.) were added into a reaction flask at room temperature, followed by DMF (22 v/w), water (9.5 v/w), and DIPEA (1.0 eq.). Then, the reaction was carried out at room temperature until the residual amount of Intermediate 2 was low (detected by QDA). After the reaction was completed, most of the solvent was removed by concentration. Water (50 v/w) and EA (50 v/w) were added, followed by 1.0 eq. of DIEA. After the mixture was stirred until dissolved and clarified, it was left to stand for liquid separation. The aqueous phase was extracted with EA (50 v/w * 2), and the aqueous phase was concentrated to dryness to obtain Intermediate 3. The reaction formula was as shown below:

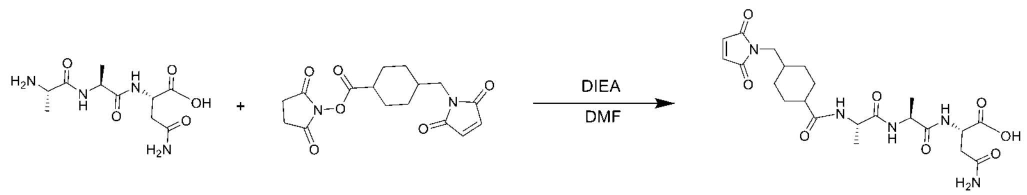


Ala-Ala-Asn-OH MCC-OSu MCC-Ala-Ala-Asn-OH

4. Synthesis of the product

Intermediate 3 was subjected to a condensation reaction with exatecan mesylate (CAS No.: 169869-90-3) using HATU to obtain the product. The specific steps were as follows. Intermediate 3 (1.2 eq.) was added into a reaction flask at room temperature, followed by DMF (15 v/w). Exatecan mesylate (1.0 eq.), TBTU (1.4 eq.), and DIPEA (4.5 eq.) were added successively. Then, the reaction was carried out at room temperature, and in-process monitoring was carried out by HPLC. After the reaction was completed, purification was carried out by prep-HPLC (preparative high performance liquid chromatography). The preparative solution was concentrated, and extracted with DCM to obtain the product. The reaction formula was as shown below:

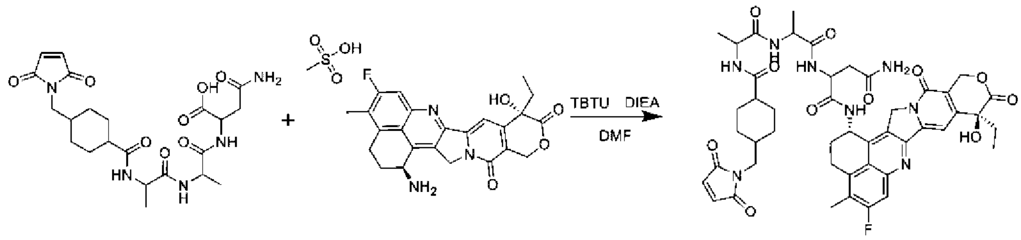


MCC-Ala-Ala-Asn-OH Exatecan mesylate MCC-Ala-Ala-Asn-Exatecan

$^1$H NMR (400 MHz, DMSO-$d_6$) δ 8.15-8.25 (d, *J* =2.0 Hz, 1H), 7.85-8.0 (d, *J* =7.5Hz, 2H), 7.7-7.85(d, *J* =2.0 Hz, 1H), 7.33-7.4 (d, *J* =8.0 Hz,1H), 7.28-7.32 (m,1H), 6.98-7.05(d, *J* =8.0 Hz,2H), 6.85-6.95(m,1H), 6.45-6.57(m,1H), 5.45-5.55(m,1H), 5.40-5.45(m,1H), 5.20-5.27(m,1H), 4.40-4.50(m,1H), 3.95-4.05(m, 1H), 3.20-3.27(m,2H), 3.10-3.18(m,2H), 2.86-2.97(m,2H), 2.36-2.43(m,3H), 2.15-2.25(m,1H), 1.95-2.10(m,3H), 1.80-1.93(m,3H), 1.73-1.78(m,1H), 1.55-1.70(m,3H), 1.45-1.55(m,3H), 1.0-1.3(m,10H), 0.80-0.95(m,5H).

IV. Preparation of mcc-AAQ-Exatecan

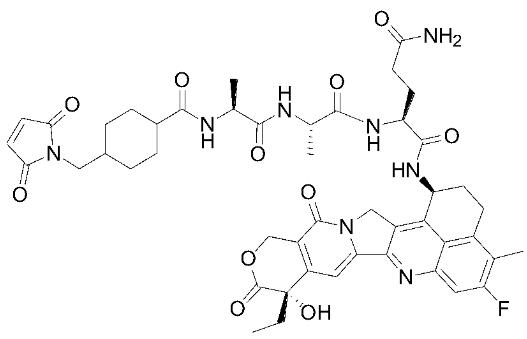

**mcc-AAQ-Exatecan**

1. Synthesis of Intermediate 1, Fmoc-Gln-Exatecan

Fmoc-Gln-OH (N-fluorenylmethyloxycarbonyl-L-glutamine) (76.3 mg, 1.1 eq.) and Exatecan (100 mg, 1.0 eq.) were added into a reaction flask, followed by DMF (1 ml), DIEA (29 mg, 1.5 eq.), and TBTU (72.5 mg, 1.1 eq.), and the reaction was carried out at room temperature. The reaction was monitored by HPLC until no starting materials remained. After the reaction was completed, purification was carried out by medium-pressure column chromatography

(DCM/MeOH). The product was collected and concentrated to dryness to obtain Intermediate 1.

The reaction formula was as shown below:

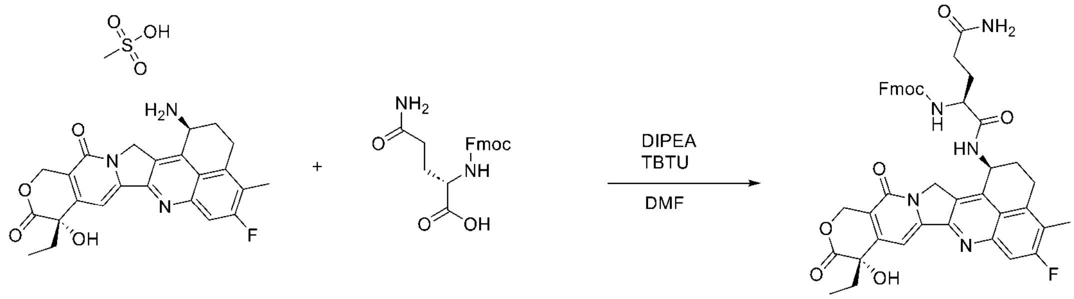


2. Synthesis of Intermediate 2, Gln-Exatecan

Intermediate 1, Fmoc-Gln-Exatecan (130 mg, 1.0 eq.) was added into a reaction flask, followed by DCM (2 ml). The mixture was stirred at room temperature. DEA (0.5 ml) was added, and the reaction was carried out at room temperature. The reaction was monitored by HPLC until no starting materials remained. The reaction was stopped. MTBE (10 ml) was slowly added, so that a large amount of solid was precipitated out, and the mixture was stirred for 30 min. The solid was filtered and washed with MTBE to obtain an off-white solid, which was dried to obtain Intermediate 2.

The reaction formula was as shown below:

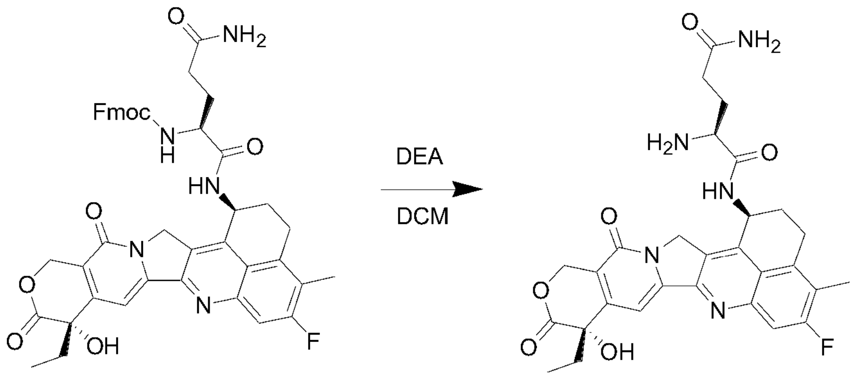


3. Synthesis of Intermediate 3, Fmoc-Ala-Ala-Gln-Exatecan

Intermediate 2, Gln-Exatecan (100 mg, 1.0 eq.) was added into a reaction flask, followed by DMF (1 mL), Fmoc-Ala-Ala-OH (67.8 mg, 1.0 eq.), TBTU (68.4 mg, 1.2 eq.), and DIEA (34.4 mg, 1.5 eq.), and the reaction was carried out at room temperature. The reaction was monitored by HPLC until it was completed. Purification was carried out by medium-pressure column chromatography (DCM/MeOH). The product was collected to obtain Intermediate 3.

The reaction formula was as shown below:

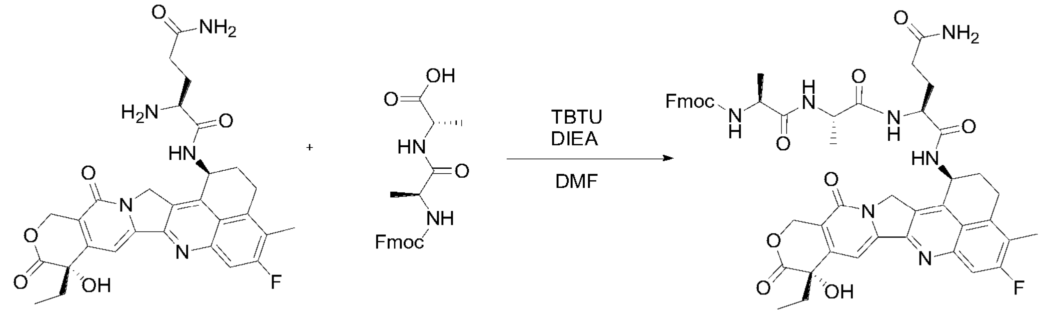

4. Synthesis of Intermediate 4, Ala-Ala-Gln-Exatecan

Intermediate 3, Fmoc-Ala-Ala-Gln-Exatecan (140 mg, 1.0 eq.) was added into a reaction flask, followed by DCM (2 ml). The mixture was stirred at room temperature. DEA (0.5 ml) was added, and the reaction was carried out at room temperature. The reaction was monitored by HPLC until it was completed. MTBE (10 ml) was slowly added, so that a large amount of solid was precipitated out, and the mixture was stirred for 30 min. The solid was filtered and washed with MTBE to obtain an off-white solid, which was dried to obtain Intermediate 4.

The reaction formula was as shown below:

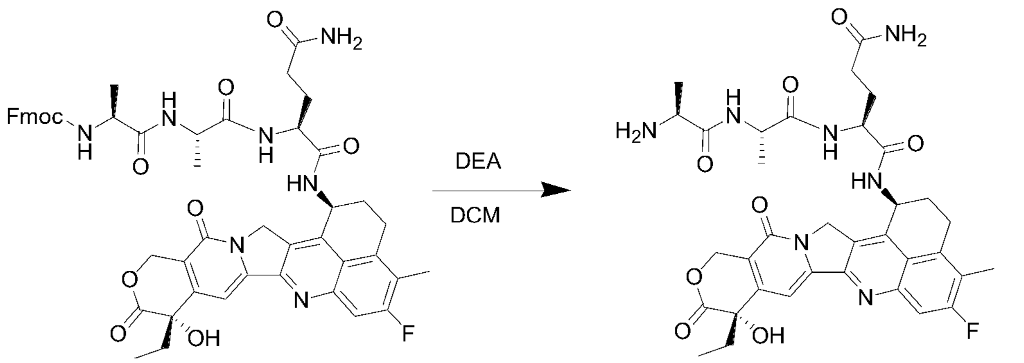


5. Synthesis of the product, MCC-Ala-Ala-Gln-Exatecan

Intermediate 4, Ala-Ala-Gln-Exatecan (50 mg, 1.0 eq.) was added into a reaction flask, followed by DMF (1 mL), MCC (18 mg, 1.1 eq.), and DIEA (12.1 mg, 1.5 eq.), and the reaction was carried out at room temperature. The reaction was monitored by HPLC until no Intermediate 4 remained, and the reaction was stopped. Purification was carried out by prep-HPLC. The product was collected and concentrated to dryness to obtain the product.

The reaction formula was as shown below:

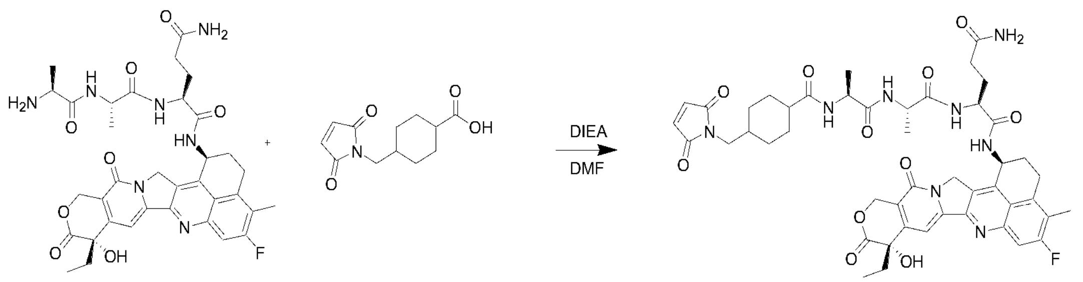


$^1$H NMR (400 MHz, DMSO-$d_6$) δ 8.17-8.26 (d, $J$=2.0 Hz, 1H), 7.83-7.91 (d, $J$=2.0 Hz, 2H), 7.65-7.74(d, $J$=2.0 Hz, 2H), 7.21-7.28(d, $J$ =8.0 Hz,2H), 6.98-7.04(m,2H), 5.43-5.50(m,1H), 5.36-5.43(m,2H), 5.15-5.24(m,1H), 5.01-5.11(m,1H), 4.14-4.24(m,1H), 3.97-4.08(m,1H), 3.47-3.57(m,1H), 3.20-3.27(m,1H), 3.05-3.16(m,2H), 2.30-2.36(m,2H), 2.00-2.15(m,4H), 1.77-1.92(m,3H), 1.65-2.00(m,5H), 1.45-1.64(m,4H), 1.09-1.24(m,5H), 0.95-1.07(m,4H), 0.79-0.94(m,5H).

V. Preparation of mcc-Ala-Ala-(3-cyano-L-alanine)-Exatecan

1. Synthesis of Intermediate 1, (3-cyano-L-alanine)-Exatecan

Fmoc-Asn-OH (40 mg, 1.2 eq.) was added into a reaction flask, followed by DMF (1.5 ml), TBTU (45 mg, 1.5 eq.), and DIEA (60 mg, 2.5 eq.), and the mixture was stirred until it was completely dissolved. After stirring for 30 min, exatecan mesylate (50 mg, 1.0 eq.) was added, and the mixture was stirred for 4 hrs. The reaction was monitored by HPLC until it was stopped. Additional TBTU (45 mg, 1.5 eq.) and DIEA (60 mg, 2.5 eq.) were added, and the reaction was continued at room temperature. The reaction was monitored by HPLC until no starting material Exatecan remained, and Fmoc-(3-cyano-L-alanine)-Exatecan accounted for approximately 70%. The reaction was stopped, and preparation and purification were carried out by medium-pressure chromatography. The product was collected.

The above product was added into a reaction flask, followed by DCM (4 ml), and the mixture was stirred. DEA (1

ml) was added, and the mixture was stirred at room temperature until it was completely dissolved. The reaction was monitored by HPLC until no starting materials remained. 10 ml of MTBE was added, and the mixture was stirred at room temperature. A large amount of solid was precipitated out, and the solid was filtered to obtain an off-white solid, namely Intermediate 1.

The reaction formula was as shown below:

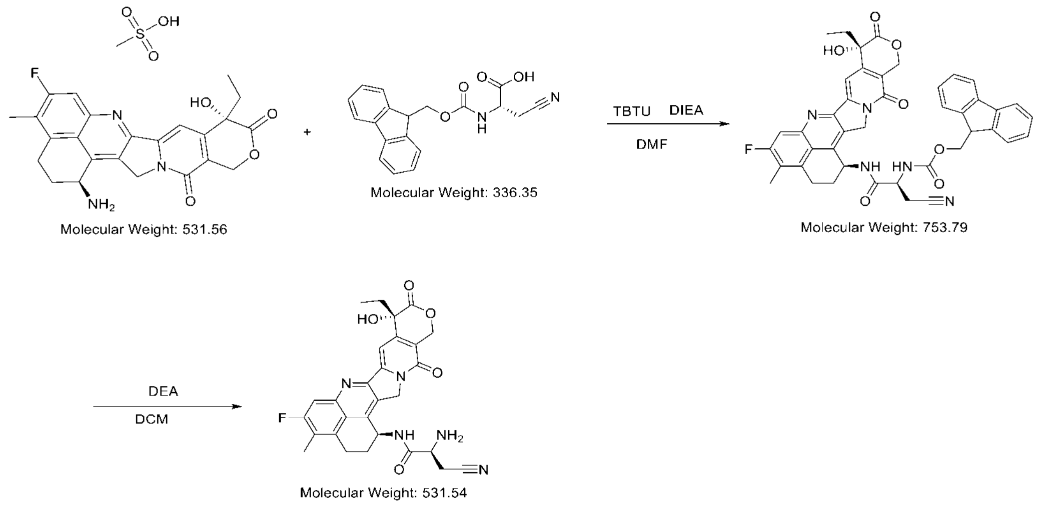


2. Synthesis of Intermediate 2, mcc-Ala-Ala-OH

SMCC (460 mg, 1.1 eq.) was added into a reaction flask, followed by $NH_2$-Ala-Ala-OH (200 mg, 1.0 eq.) and DMF (4 ml, 20 V), and the mixture was stirred. Then, DIPEA (161.4 mg, 1.0 eq.) was added. The mixture was heated to 60°C for reaction. As the reaction proceeded, the reaction mixture was gradually dissolved and clarified. The reaction was carried out for at least 8 hours. A sample was taken for LC-MS detection. After the reaction was completed, the reaction mixture was cooled down to room temperature. DMF was removed by concentration under reduced pressure at 35°C. Then, purified water (4 ml, 20 V) and DIPEA (161.4 mg, 1.0 eq.) were added. EA (4 ml, 20 V) was added, and the mixture was stirred and washed. The organic phase was discarded. Then, EA (2 ml, 10 V) was added for extraction twice. The organic phase was discarded. The aqueous phase was retained, and the acidity was adjusted to pH 1-2 with 2 M hydrochloric acid. A large amount of white solid was precipitated out. The mixture was stirred for one hour, and filtered to obtain a white solid, namely Intermediate 2.

The reaction formula was as shown below:

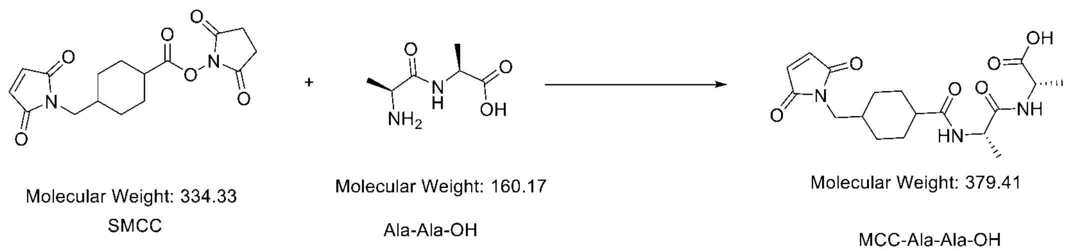


3. Synthesis of the product, mcc-Ala-Ala-(3-cyano-L-alanine)-Exatecan

Intermediate 1, (3-cyano-L-alanine)-Exatecan (35 mg, 1.0 eq.) was added into a reaction flask, followed by DMF (1 mL), Intermediate 2, mcc-Ala-Ala-OH (28 mg, 1.1 eq.), TBTU (25.3 mg, 1.2 eq.), and DIPEA (12.8 mg, 1.5 eq.). After the reaction was carried out at room temperature for 2 hours, it was monitored by HPLC until no Intermediate 1 remained, and the reaction was stopped. Purification was carried out by prep-HPLC. The product was collected.

The reaction formula was as shown below:

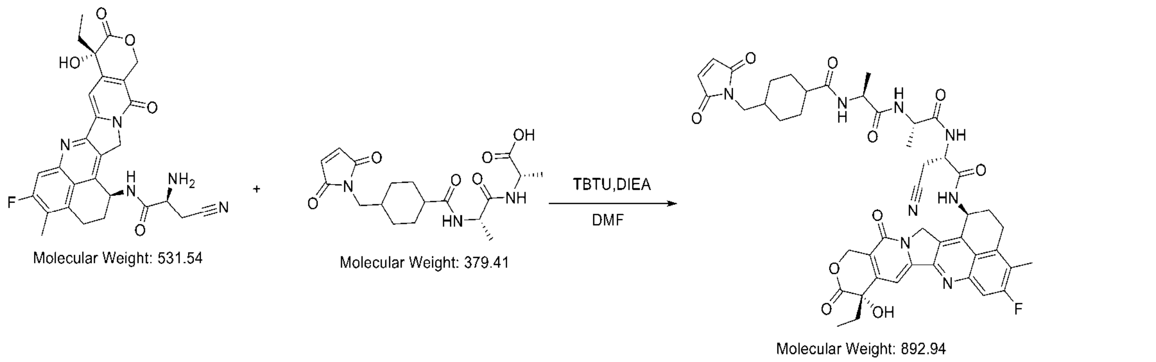


$^1H$ NMR (400 MHz, DMSO-$d_6$) δ 8.45-8.55 (d, *J* =2.0 Hz, 1H), 8.23-8.29 (d, *J* =2Hz, 2H), 7.95-8.03(d, *J* =2.0 Hz, 1H), 7.84-7.92 (d, *J* =8.0 Hz,1H), 7.76-7.84 (m,1H), 7.27-7.33(d, *J* =8.0 Hz,1H), 6.98-7.02(m,1H), 5.47-5.56(m,1H), 5.40-5.45(m,1H), 5.26-5.36(m,1H), 5.1-5.2(m,1H), 4.44-4.55(m,1H), 4.07-4.19(m,1H), 3.88-4.0(m,2H), 3.19-3.26(m,2H), 3.13-3.18(m,1H), 2.90-3.0(m,2H), 2.76-2.88(m,2H), 2.36-2.43(m,3H), 2.00-2.12(m,2H), 1.80-1.93(m,3H), 1.80-1.92(m,2H), 1.55-1.70(m,3H), 1.13-1.26(m,5H), 1.05-1.12(m,3H), 0.80-0.95(m,5H).

VI. Synthesis route of mcc-Ala-Ala-((*S*)-2-amino-4-cyanobutanoic acid)-Exatecan

1. Synthesis of Intermediate 1, ((*S*)-2-amino-4-cyanobutanoic acid)-Exatecan

(S)-2-((((9H-fluoren-9-yl)methoxy)carbonyl)amino)-4-cyanobutyric acid (79.8 mg, 1.1 eq.) and exatecan mesylate (110 mg, 1.0 eq.) were added into a reaction flask, followed by DMF (1.5 ml), TBTU (79.73 mg, 1.5 eq.), and DIEA (80 mg, 2.5 eq.), and the reaction was carried out at room temperature for 3 hours. The reaction was monitored by HPLC. After the reaction was completed, 9 ml of ACN was added, and the mixture was stirred at room temperature. Then, 1.0 ml of DEA was added, and the reaction was continued at room temperature for 1 hour. A sample was taken for monitoring until no starting materials remained. 10 ml of MTBE was added, and the mixture was stirred until no obvious solid was precipitated out. After stirring for 1 hour, there was no obvious change.

The solvent was removed by concentration under reduced pressure at 30°C. During the concentration process, a large amount of solid was precipitated out. At this time, only a small amount of the solvent, approximately 3 ml, was removed. The concentration was stopped, and the mixture was continuously stirred at room temperature for 1 hour. After being filtered, the solid was washed with MTBE, and dried to obtain an off-white solid, namely Intermediate 1.

The reaction formula was as shown below:

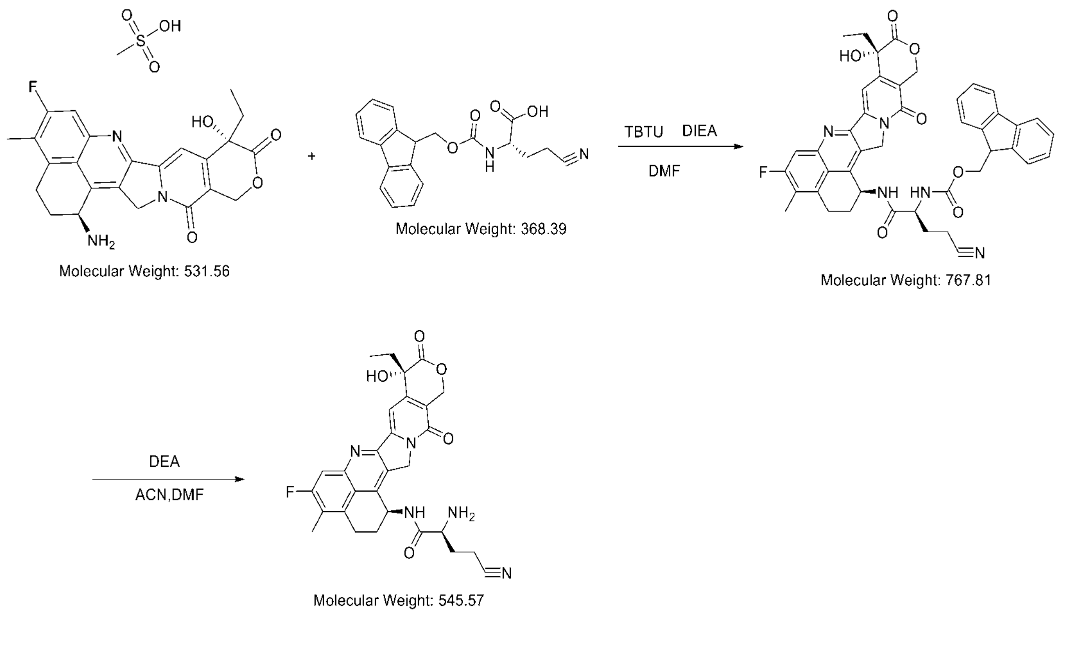

2. Synthesis of Intermediate 2, mcc-Ala-Ala-OH

SMCC (460 mg, 1.1 eq.) was added into a reaction flask, followed by $NH_2$-Ala-Ala-OH (200 mg, 1.0 eq.) and DMF (4 ml, 20 V), and the mixture was stirred. Then, DIPEA (161.4 mg, 1.0 eq.) was added. The mixture was heated to 60°C for reaction for 8 hours. A sample was taken for LC-MS detection. After the reaction was completed, the reaction mixture was cooled down to room temperature. DMF was removed by concentration under reduced pressure at 35°C. Then, purified water (4 ml, 20 V) and DIPEA (161.4 mg, 1.0 eq.) were added. EA (4 ml, 20 V) was added, and the mixture was stirred and washed. The organic phase was discarded. Then, EA (2 ml, 10 V) was added for extraction twice. The organic phase was discarded. The aqueous phase was retained, and the acidity was adjusted to pH 1-2 with 2 M hydrochloric acid. A large amount of white solid was precipitated out. The mixture was stirred for one hour, and filtered to obtain a white solid, namely Intermediate 2.

The reaction formula was as shown below:

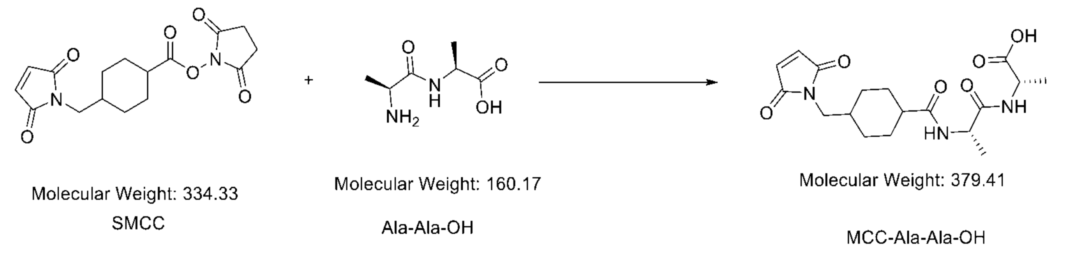


3. Synthesis of the product, mcc-Ala-Ala-((*S*)-2-amino-4-cyanobutanoic acid)-Exatecan

Intermediate 1, ((*S*)-2-amino-4-cyanobutanoic acid)-Exatecan (25 mg, 1.0 eq.) was added into a reaction flask, followed by DMF (1 mL), Intermediate 2, mcc-Ala-Ala-OH (19 mg, 1.1 eq.), TBTU (17.7 mg, 1.2 eq.), and DIPEA (8.88 mg, 1.5 eq.). After the reaction was carried out at room temperature for 2 hours, it was monitored by HPLC until it was completed. Preparation and purification were carried out by prep-HPLC. The product was collected.

The reaction formula was as shown below:

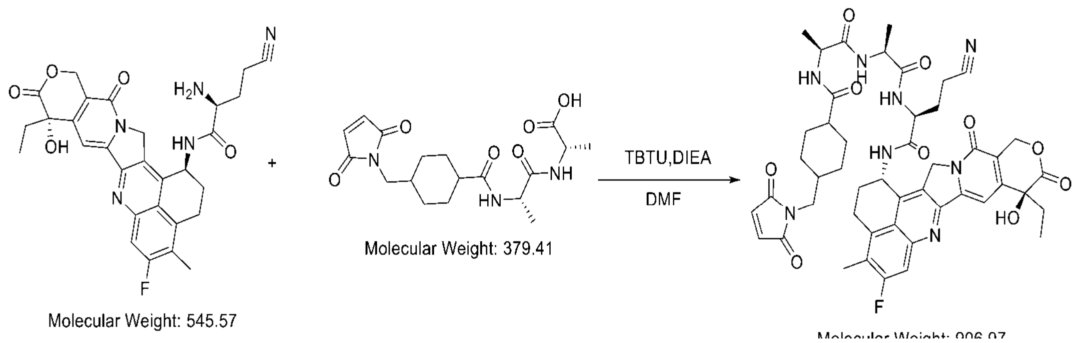


$^1$H NMR (400 MHz, DMSO-$d_6$) δ 8.30-8.37 (d, $J$=2.0 Hz, 1H), 7.87-7.96 (d, $J$=2.0 Hz, 2H), 7.75-7.86(d, $J$=2.0 Hz, 2H), 7.27-7.33(d, $J$ =8.0 Hz,1H), 6.98-7.02(m,2H), 5.45-5.54(m,1H), 5.40-5.45(m,2H), 5.23-5.32(m,1H), 5.04-5.13(m,1H), 4.25-4.37(m,1H), 3.97-4.09(m,1H), 3.56-3.66(m,4H), 3.10-3.26(m,4H), 2.35-2.46(m,4H), 2.00-2.13(m,3H), 1.77-1.92(m,3H), 1.66-1.75(m,2H), 1.56-1.64(m,3H), 1.45-1.53(m,1H), 1.12-1.26(m,4H), 1.01-1.13(m,4H), 0.80-0.94(m,4H).

VII. Synthesis route of mcc-Ala-Ala-Ala-Ala-Gln-Exatecan

1. Synthesis of Intermediate 1, mcc-Ala-Ala-Ala-Ala-Gln-OH

H-Gln(Trt)-OH 2-CTC resin (1.0 g, 0.5 mmol, 0.5 mmol/g) was added into a solid-phase reactor, followed by DCM (20 mL). The resin was shaken in a shaker for 20 min to be fully swollen. The resin was filtered and washed with DMF. 20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF. 20 mL of a 20% piperidine/DMF solution was added, and the reaction was carried out in a shaker for 15 min. After

filtering, 20 mL of a 20% piperidine/DMF solution was added again, and the reaction was carried out in a shaker for 15 min. The color of the resin in the Kaiser test was positive. The resin was washed with DMF to obtain H-Ala-Gln (Trt)-2-CTC-resin.

20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added into a reaction tube containing H-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF to obtain H-Ala-Ala-Gln (Trt)-2-CTC-resin.

20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added into a reaction tube containing H-Ala-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF to obtain H-Ala-Ala-Ala-Gln (Trt)-2-CTC-resin.

20 mL of a DMF solution containing Fmoc-Ala-OH (3.0 eq., 467 mg, 1.5 mmol), HBTU (3.0 eq., 569 mg, 1.5 mmol), and HOBT (3.0 eq., 202 mg, 1.5 mmol) was added into a reaction tube containing H-Ala-Ala-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. The resin was filtered and washed with DMF. 20 mL of a 20% piperidine/DMF solution was added, and the reaction was carried out in a shaker for 15 min. After filtering, 20 mL of a 20% piperidine/DMF solution was added again, and the reaction was carried out in a shaker for 15 min. The color of the resin in the Kaiser test was positive. The resin was washed with DMF to obtain H-Ala-Ala-Ala-Ala-Gln (Trt)-2-CTC-resin.

20 mL of a DMF solution containing SMCC (3.0 eq., 501 mg, 1.5 mmol) was added into a reaction tube containing H-Ala-Ala-Ala-Ala-Gln (Trt)-2-CTC-resin. After shaking well, DIPEA (3.0 eq., 0.26 mL, 1.5 mmol) was added, and the reaction was carried out in a shaker for 1 hr. The color of the resin in the Kaiser test was negative. After being filtered, the resin was washed with DMF, MeOH, and MTBE successively. The resin was dried in a vacuum drying oven at 30°C for 1 hr to obtain mcc-Ala-Ala-Ala-Ala-Gln(Trt)-2-CTC-resin (1.42 g, 0.5 mmol).

The resulting resin above was transferred to a 50 mL centrifuge tube. A cold 95% TFA/Tis solution (15 mL) was added, and the reaction was carried out in a shaker for 1 hr. After filtering, the filtrate was slowly added into a cold MTBE solution (150 mL). The mixture was left to stand for 1 hr, and a white precipitate was precipitated out. The precipitate was centrifuged and washed with a cold MTBE solution. The washed precipitate was collected and dried in a vacuum drying oven at 30°C for 4 hrs to obtain a white solid, namely Intermediate 1, mcc-Ala-Ala-Ala-Ala-Gln-OH.

The reaction formula was as shown below:

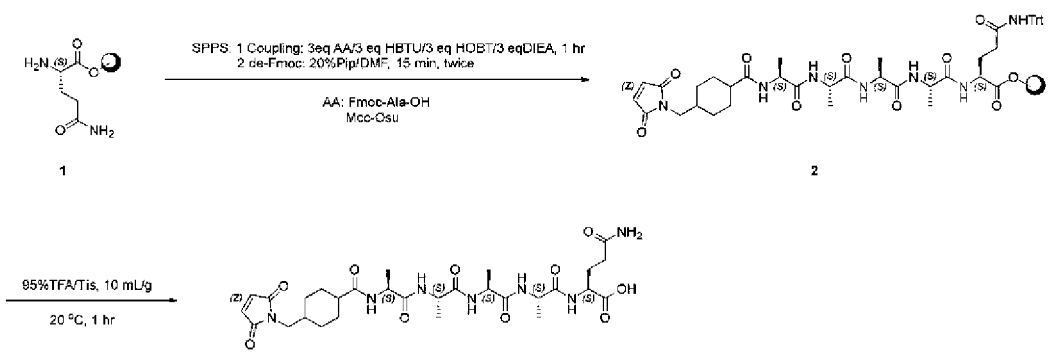


2. Synthesis of the product, mcc-Ala-Ala-Ala-Ala-Gln-Exatecan

Intermediate 1, mcc-Ala-Ala-Ala-Ala-Gln-OH (244 mg, 2.0 eq.) was added into a reaction flask, followed by DMF (2 mL), irinotecan mesylate (100 mg, 1.0 eq.), TBTU (132 mg, 2.2 eq.), and DIPEA (73 mg, 3.0 eq.). The mixture was stirred and reacted at room temperature for 1 hour. Preparation and purification were carried out by prep-HPLC. The product was collected.

The reaction formula was as shown below:

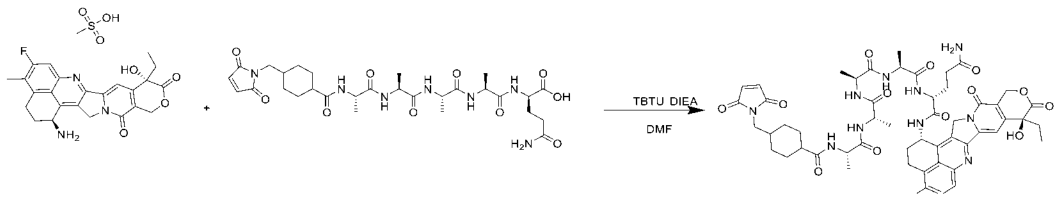


$^1$H NMR (400 MHz, DMSO-$d_6$) δ 8.25-8.33(d, *J* =2.0 Hz, 1H), 7.87-7.95 (d, *J* =2.0 Hz, 2H), 7.76-7.86(d, *J* =2.0 Hz, 2H), 7.28-7.34(d, *J* =8.0 Hz,1H), 7.20-7.26(m,1H), 6.98-7.02(m,1H), 6.73-6.76(m,1H), 5.47-5.55(m,1H), 5.40-5.41(m,1H), 5.26-5.36(m,2H), 5.05-5.15 (m,2H), 4.12-4.20(m,2H), 4.03-4.09(m,1H), 3.87-3.94(m,1H), 3.20-3.27(m,2H), 3.12-3.18(m,1H), 2.65-2.68(m,1H), 2.36-2.43(m,2H), 2.31-2.35(m,1H), 2.02-2.13(m,2H), 1.78-1.93(m,3H), 1.6-1.76(m,2H), 1.56-1.64(m,2H), 1.21-1.27(m,1H), 1.06-1.20(m,6H), 0.85-0.94(m,3H).

Example 3. Preparation of GPC3-ADC

1. Preparation of the antibody conjugate Hu 52H5D3B8-8-mc-AAN-Exatecan (ADC-1)

a. 10 mg of a Hu 52H5D3B8-8 antibody was taken and diluted with PBS buffer to a concentration of 5 mg/mL. The pH of the antibody was adjusted to 7.5 with a Tris-EDTA solution. The protein concentration was detected using Nanodrop, the net weight of the antibody solution was weighed, and the total protein amount was calculated. 8 folds by number of moles of a TCEP solution was added into the antibody, and the mixture was placed on a 3D shaker and reacted at room temperature for 120 min with continuous mixing.
b. An excess of mc-AAN-Exatecan solution (dissolved in DMSO) was added into the reduced antibody solution. After being mixed evenly, the mixture was placed on a 3D shaker and reacted at room temperature for 30 min with continuous mixing. After the reaction was completed, an excess of N-acetylcysteine solution was added into the reaction mixture. The mixture was placed on a 3D shaker and reacted at room temperature for 30 min with continuous mixing.
c. The conjugated product was purified using a 15 mL 30 KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-8-mc-AAN-Exatecan, which was stored at 4°C.
d. The protein concentration of the antibody drug conjugate was detected by the UV/BCA method, the DAR was detected by HIC (as shown in Figure 5), with a DAR of 8.0, and the purity was detected by SEC.

2. Preparation of the antibody conjugate Hu 52H5D3B8-8-mcc-AAN-Exatecan (ADC-2)

a. 10 mg of a Hu 52H5D3B8-8 antibody was taken and diluted with PBS buffer to a concentration of 5 mg/mL. The pH of the antibody was adjusted to 7.5 with a Tris-EDTA solution. The protein concentration was detected using Nanodrop, the net weight of the antibody solution was weighed, and the total protein amount was calculated. 8 folds by number of moles of a TCEP solution was added into the antibody, and the mixture was placed on a 3D shaker and reacted at room temperature for 120 min with continuous mixing.
b. An excess of mcc-AAN-Exatecan solution (dissolved in DMSO) was added into the reduced antibody solution. After being mixed evenly, the mixture was placed on a 3D shaker and reacted at room temperature for 30 min with continuous mixing. After the reaction was completed, an excess of N-acetylcysteine solution was added into the reaction mixture. The mixture was placed on a 3D shaker and reacted at room temperature for 30 min with continuous mixing.
c. The conjugated product was purified using a 15 mL 30 KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-8-mcc-AAN-Exatecan, which was stored at 4°C.
d. The protein concentration of the antibody drug conjugate was detected by the UV/BCA method, the DAR was detected by HIC (as shown in Figure 6), with a DAR of 8.0, and the purity was detected by SEC.

3. Preparation of the antibody conjugate Hu 52H5D3B8-8-mc-AAQ-Exatecan (ADC-3)

a. 10 mg of a Hu 52H5D3B8-8 antibody was taken and diluted with PBS buffer to a concentration of about 5

mg/mL. The pH of the antibody was adjusted to around 7.5 with a Tris-EDTA solution. The protein concentration was detected using Nanodrop, the net weight of the antibody solution was weighed, and the total protein amount was calculated. 8 folds by number of moles of a TCEP solution was added into the antibody, and the mixture was placed on a 3D shaker and reacted at room temperature for more than 120 min with continuous mixing.

b. An excess of mc-AAQ-Exatecan solution (dissolved in DMSO) was added into the reduced antibody solution. After being mixed evenly, the mixture was placed on a 3D shaker and reacted at room temperature for more than 30 min with continuous mixing. After the reaction was completed, an excess of N-acetylcysteine solution was added into the reaction mixture. The mixture was placed on a 3D shaker and reacted at room temperature for more than 30 min with continuous mixing.

c. The conjugated product was purified using a 15 mL 30 KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-8-mc-AAQ-Exatecan, which was stored at 4°C.

d. The protein concentration of the antibody drug conjugate was detected by the UV/BCA method, the DAR was detected by HIC (as shown in Figure 7), with a DAR of 8.0, and the purity was detected by SEC.

4. Preparation of the antibody conjugate Hu 52H5D3B8-8-mcc-AAQ-Exatecan (ADC-4)

a. 10 mg of a Hu 52H5D3B8-8 antibody was taken and diluted with PBS buffer to a concentration of 5 mg/mL. The pH of the antibody was adjusted to 7.5 with a Tris-EDTA solution. The protein concentration was detected using Nanodrop, the net weight of the antibody solution was weighed, and the total protein amount was calculated. 8 folds by number of moles of a TCEP solution was added into the antibody, and the mixture was placed on a 3D shaker and reacted at room temperature for 120 min with continuous mixing.

b. An excess of mcc-AAQ-Exatecan solution (dissolved in DMSO) was added into the reduced antibody solution. After being mixed evenly, the mixture was placed on a 3D shaker and reacted at room temperature for 30 min with continuous mixing. After the reaction was completed, an excess of N-acetylcysteine solution was added into the reaction mixture. The mixture was placed on a 3D shaker and reacted at room temperature for 30 min with continuous mixing.

c. The conjugated product was purified using a 15 mL 30 KD ultrafiltration centrifuge tube and exchanged into a storage solution (10 mM Histidine, with a pH of 5.5) with an exchange fold of greater than 1000. Then, the product was filtered through a 0.22 um sterilizing filter to obtain the antibody drug conjugate, Hu 52H5D3B8-8-mcc-AAQ-Exatecan, which was stored at 4°C.

d. The protein concentration of the antibody drug conjugate was detected by the UV/BCA method, the DAR was detected by HIC (as shown in Figure 8), with a DAR of 8.0, and the purity was detected by SEC.

5. Preparation of the antibody conjugate Hu 52H5D3B8-7-mcc-AAQ-Exatecan (ADC-5)

a. 10 mg of a Hu 52H5D3B8-7 antibody was taken and diluted with PBS buffer to a concentration of 5 mg/mL. The pH of the antibody was adjusted to 7.5 with a Tris-EDTA solution. The protein concentration was detected using Nanodrop, the net weight of the antibody solution was weighed, and the total protein amount was calculated. 10 folds by number of moles of a TCEP solution was added into the antibody, and the mixture was placed on a 3D shaker and reacted at room temperature for 120 min with continuous mixing.

b. An excess of mcc-AAQ-Exatecan solution (dissolved in DMSO) was added into the reduced antibody solution. After being mixed evenly, the mixture was placed on a 3D shaker and reacted at room temperature for 30 min with continuous mixing. After the reaction was completed, an excess of N-acetylcysteine solution was added into the reaction mixture. The mixture was placed on a 3D shaker and reacted at room temperature for 30 min with continuous mixing.

c. The conjugated product was purified using a 15 mL 30 KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-7-mcc-AAQ-Exatecan, which was stored at 4°C.

d. The protein concentration of the antibody drug conjugate was detected by the UV/BCA method, the DAR was detected by HIC (as shown in Figure 9), with a DAR of 7.9, and the purity was detected by SEC.

6. Preparation of the antibody conjugate Hu 52H5D3B8-8-mcc-AAQ-Exatecan (ADC-6)

a. 10 mg of a Hu 52H5D3B8-8 antibody was taken and diluted with PBS buffer to a concentration of 5 mg/mL. The pH of the antibody was adjusted to 7.5 with a Tris-EDTA solution. The protein concentration was detected using

Nanodrop, the net weight of the antibody solution was weighed, and the total protein amount was calculated. 2.1 folds by number of moles of a TCEP solution was added into the antibody, and the mixture was placed on a 3D shaker and reacted at room temperature for 120 min with continuous mixing.

b. An excess of mcc-AAQ-Exatecan solution (dissolved in DMSO) was added into the reduced antibody solution. After being mixed evenly, the mixture was placed on a 3D shaker and reacted at room temperature for 30 min with continuous mixing. After the reaction was completed, an excess of N-acetylcysteine solution was added into the reaction mixture. The mixture was placed on a 3D shaker and reacted at room temperature for 30 min with continuous mixing.

c. The conjugated product was purified using a 15 mL 30 KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-8-mcc-AAQ-Exatecan, which was stored at 4°C.

d. The protein concentration of the antibody drug conjugate was detected by the UV/BCA method, the DAR was detected by HIC (as shown in Figure 10), with a DAR of 4.0, and the purity was detected by SEC.

7. Preparation of the antibody conjugate Hu 52H5D3B8-7-mcc-AAQ-Exatecan (ADC-7)

a. 10 mg of a Hu 52H5D3B8-7 antibody was taken and diluted with PBS buffer to a concentration of 5 mg/mL. The pH of the antibody was adjusted to 7.5 with a Tris-EDTA solution. The protein concentration was detected using Nanodrop, the net weight of the antibody solution was weighed, and the total protein amount was calculated. 2.5 folds by number of moles of a TCEP solution was added into the antibody, and the mixture was placed on a 3D shaker and reacted at room temperature for 120 min with continuous mixing.

b. An excess of mcc-AAQ-Exatecan solution (dissolved in DMSO) was added into the reduced antibody solution. After being mixed evenly, the mixture was placed on a 3D shaker and reacted at room temperature for 30 min with continuous mixing. After the reaction was completed, an excess of N-acetylcysteine solution was added into the reaction mixture. The mixture was placed on a 3D shaker and reacted at room temperature for 30 min with continuous mixing.

c. The conjugated product was purified using a 15 mL 30 KD ultrafiltration centrifuge tube and exchanged into a storage solution (10 mM Histidine, with a pH of 5.5) with an exchange fold of greater than 1000. Then, the product was filtered through a 0.22 um sterilizing filter to obtain the antibody drug conjugate, Hu 52H5D3B8-7-mcc-AAQ-Exatecan, which was stored at 4°C.

d. The protein concentration of the antibody drug conjugate was detected by the UV/BCA method, the DAR was detected by HIC (as shown in Figure 11), with a DAR of 4.0, and the purity was detected by SEC.

8. Preparation of the antibody conjugate Hu 52H5D3B8-8-MCC-AA-(3-cyano-L-alanine)-Exatecan (ADC-8)

a. The GPC3 antibody Hu 52H5D3B8-8 was taken, and the pH of the antibody was adjusted to around ~7.5 with a Tris-EDTA solution. The protein concentration was detected using Nanodrop, the net weight of the antibody solution was weighed, and the total protein amount was calculated. A TCEP solution was added into the antibody, and the mixture was placed on a 3D shaker and reacted at room temperature for more than 120 min with continuous mixing to completely reduce the interchain disulfide bonds of the antibody.

b. An excess of MCC-AA-(3-cyano-L-alanine)-Exatecan solution (dissolved in DMSO) was added into the reduced antibody solution. After being mixed evenly, the mixture was placed on a 3D shaker and reacted at room temperature for more than 30 min with continuous mixing. After the reaction was completed, an excess of N-acetylcysteine solution was added into the reaction mixture. The mixture was placed on a 3D shaker and reacted at room temperature for more than 30 min with continuous mixing.

c. The conjugated product was purified using a 30KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-8-MCC-AA-(3-cyano-L-alanine)-Exatecan, which was stored at 4°C.

d. The protein concentration of the antibody drug conjugate was detected by the UV/BCA method, the DAR was detected by HIC (Figure 12), with a DAR of 8.0, and the purity was detected by SEC.

9. Preparation of the antibody conjugate Hu 52H5D3B8-8-MCC-AAAAQ-Exatecan (ADC-9)

a. The GPC3 antibody Hu 52H5D3B8-8 was taken, and the pH of the antibody was adjusted to around ~7.5 with a Tris-EDTA solution. The protein concentration was detected using Nanodrop, the net weight of the antibody solution was weighed, and the total protein amount was calculated. A TCEP solution was added into the antibody,

and the mixture was placed on a 3D shaker and reacted at room temperature for more than 120 min with continuous mixing to completely reduce the interchain disulfide bonds of the antibody.

b. An excess of MCC-AAN1-Exatecan solution (dissolved in DMSO) was added into the reduced antibody solution. After being mixed evenly, the mixture was placed on a 3D shaker and reacted at room temperature for more than 30 min with continuous mixing. After the reaction was completed, an excess of N-acetylcysteine solution was added into the reaction mixture. The mixture was placed on a 3D shaker and reacted at room temperature for more than 30 min with continuous mixing.

c. The conjugated product was purified using a 30KD ultrafiltration centrifuge tube and exchanged into a storage solution (10 mM Histidine, with a pH of around 5.5) with an exchange fold of greater than 1000. Then, the product was filtered through a 0.22 um sterilizing filter to obtain the antibody drug conjugate, Hu 52H5D3B8-8-MCC-AAAAQ-Exatecan, which was stored at 4°C.

d. The protein concentration of the antibody drug conjugate was detected by the UV/BCA method, the DAR was detected by HIC (Figure 13), with a DAR of 8.0, and the purity was detected by SEC.

Moreover, with reference to the above methods, those skilled in the art can also prepare other ADCs of the present invention such as GPC3 (e.g., Hu 52H5D3B8-8)-mcc-Ala-Ala-((*S*)-2-amino-4-cyanobutanoic acid)-Exatecan.

Example 4. Pharmacological and pharmacodynamic study of GPC3-ADC

1. Binding of GPC3-ADC to cells

It was confirmed by an FACS experiment that the binding of ADC to cells was basically unaffected by interchain Cys conjugation in the antibody.

Huh7 liver cancer cells growing in log phase were collected. After centrifugation, the cells were resuspended in FACS buffer (PBS + 3% FBS), the cell density was adjusted, and the cells were dispensed to a 96-well U-bottom cell culture plate, such that each well contained 200,000-500,000 cells. The antibody or ADC diluted with FACS buffer in a 4-fold gradient manner was added and uniformly mixed, such that the final starting concentration of each sample was finally 10 μg/mL. The 96-well plate was incubated at 4°C for 60 min. The cells in the wells were thoroughly washed with FACS buffer to remove unbound antibodies or ADCs. Goat anti-Human IgG (H+L) Cross-Adsorbed Secondary Antibody, Alexa Fluor™ 647 (Invitrogen, #A-21445), diluted with FACS buffer at 1 : 1,000 was added and incubated at 4°C in the dark for 30-60 min. The cells in the wells were thoroughly washed with FACS buffer to remove unbound secondary antibodies. The cell sample was analyzed for fluorescence signal by CytoFLEX flow cytometer (Beckman Coulter). The experimental results are as shown in Figure 14, in which the affinity of the GPC3 antibody for the Huh7 cells was not changed significantly before and after conjugation with the cytotoxin. The affinity of each test substance for the Huh7 cells is as shown in Table 3 and Figure 14.

Table 3: Binding of GPC3 antibody and each ADC to Huh7 cells ($EC_{50}$)

| Antibody/ADC name | $EC_{50}$ (ng/mL) |
|---|---|
| Hu 52H5D3B8-8 | 56.91 |
| Hu 52H5D3B8-8-MC-AAN-Exa (ADC-1) | 67.32 |
| Hu 52H5D3B8-8-MCC-AAN-Exa (ADC-2) | 60.97 |
| Hu 52H5D3B8-8-MC-AAQ-Exa (ADC-3) | 63.31 |
| Hu 52H5D3B8-8-MCC-AAQ-Exa (ADC-4) | 67.52 |

2. Internalization of antibody in GPC3-ADC

The internalization of each ADC molecule was confirmed and compared.

Huh7 liver cancer cells growing in log phase were collected. After centrifugation, the cells were washed once and resuspended in a pre-cooled DMEM medium containing 3% FBS, the cell density was adjusted, and the cells were dispensed to a 96-well U-bottom cell culture plate, such that each well contained 500,000-800,000 cells. The antibody or ADC diluted with DMEM medium containing 3% FBS was added and uniformly mixed, such that the final concentration of the sample was 10 μg/mL. The 96-well plate was incubated on ice for 60 min. The cells in the wells were thoroughly washed with pre-cooled FACS buffer (PBS + 3% FBS) to remove unbound antibodies or ADCs. The cells in the wells were evenly dispensed to four 96-well U-bottom plates. After centrifugation, the cells were resuspended with DMEM medium. One 96-well plate was placed on ice, and the other three plates were incubated in a cell incubator at 37°C. After 0.5 h, 1 h, and 2 h,

one of the culture plates at 37°C was transferred to ice, respectively. After 5-10 min since the third culture plate was transferred to ice, the diluted Goat anti-Human IgG (H+L) Cross-Adsorbed Secondary Antibody, Alexa Fluor™ 647 (Invitrogen, #A-21445) was added and incubated on ice in the dark for 30 min. The cells in the wells were washed with pre-cooled FACS buffer to remove unbound secondary antibodies. The cell sample was analyzed for fluorescence signal by CytoFLEX flow cytometer (Beckman Coulter) and expressed by MFI (GeoMean fluorescence intensity). Except for incubation at 37°C, the whole process of the experiment was kept on ice or at 4°C.

The calculation method for the percentage reduction of molecules on the cell surface was as follows:

$$\% \text{ reduction of molecules on cell surface} = (MFI_{\text{on ice}} - MFI_{37°C}) / MFI_{\text{on ice}} * 100\%.$$

The experimental results are as shown in Table 4, in which the internalization ability of the antibody Hu 52H5D3B8-8 on the Huh7 cells were not changed before and after conjugation with the cytotoxin, and the internalization ability of each ADC molecule was similar. The results of the endocytosis of each test substance on the Huh7 cells (represented by the percentage reduction of molecules on the cell surface) are as shown in Table 4 and Figure 15.

Table 4: Endocytosis of GPC3 antibody and each ADC on Huh7 cells

| Antibody/ADC name | Percentage reduction of GPC3 on cell surface (%) | | |
|---|---|---|---|
| | 0.5 h | 1 h | 2 h |
| Hu 52H5D3B8-8 | 18.6 | 37.1 | 52.8 |
| Hu 52H5D3B8-8-MC-AAN-Exa (ADC-1) | 17.9 | 32.9 | 52.2 |
| Hu 52H5D3B8-8-MCC-AAN-Exa (ADC-2) | 20.5 | 38.2 | 52.2 |
| Hu 52H5D3B8-8-MC-AAQ-Exa (ADC-3) | 20.0 | 32.8 | 50.1 |
| Hu 52H5D3B8-8-MCC-AAQ-Exa (ADC-4) | 18.9 | 36.2 | 50.2 |

Example 5. Pharmacodynamic study *in vitro*

GPC3-targeting ADC molecules with conjugation to various linker-payloads and different DAR values were compared for their cell killing activities. Non-binding means that the antibody is a non-binding human IgG1 isotype control that does not bind to a target on the surface of the tumor cells used in the experiment and was used as a negative control antibody.

Liver cancer cells growing in log phase were collected. After centrifugation, the cells were resuspended in a new medium, and the cells were counted. The cells were inoculated into a 96-well, clear-bottom, black cell culture plate (Costar) and cultured overnight in a cell incubator. The next day, the ADC molecules were diluted in a 4-fold gradient manner with a medium, and the diluted solution was carefully transferred to the black culture plate such that the final starting concentration of each sample was finally 600 or 500 ng/mL. After culturing in a cell incubator for 6 days, PrestoBlue reagent (Invitrogen) was added to take up 1/10 of the well volume, followed by incubation in a cell incubator for 1 h. The fluorescence signal was read by SpectraMax M5 plate reader, wherein the excitation and emission wavelengths of the instrument were set to 560 nm and 590 nm, respectively. The obtained fluorescence signal data were analyzed by SoftMax Pro 6.5 software.

1. Experimental reagents and sources:

Test drug

| Name | Preservation condition |
|---|---|
| Hu 52H5D3B8-8-MC-AAN-Exa (ADC-1) | 2-8°C |
| Hu 52H5D3B8-8-MCC-AAN-Exa (ADC-2) | 2-8°C |

(continued)

| Name | Preservation condition |
|---|---|
| Hu 52H5D3B8-8-MC-AAQ-Exa (ADC-3) | 2-8°C |
| Hu 52H5D3B8-8-MCC-AAQ-Exa (ADC-4) | 2-8°C |
| Hu 52H5D3B8-7-MCC-AAQ-Exa (ADC-5) | 2-8°C |
| Hu 52H5D3B8-8-MCC-AAQ-Exa (ADC-6) | 2-8°C |
| Hu 52H5D3B8-7-MCC-AAQ-Exa (ADC-7) | 2-8°C |
| Hu 52H5D3B8-8-MCC-AA-(3-cyano-L-alanine)-Exa (ADC-8) | 2-8°C |
| Hu 52H5D3B8-8-MCC-AAAAQ-Exa (ADC-9) | 2-8°C |
| Non-binding-MCC-AAQ-Exa (DAR8) | 2-8°C |
| Non-binding-MCC-AAQ-Exa (DAR4) | 2-8°C |

Cell line

| Cell line | Cell type | GPC3 expression level | Source | Culture solution |
|---|---|---|---|---|
| Huh7 | Human liver cancer cells | Overexpression ($9 * 10^3$ to $1.25 * 10^5$) | Chinese Academy of Sciences | DMEM + 10% FBS |

2. Experimental results:

The average $EC_{50}$ of cell killing activity of each GPC3-ADC is as shown in Table 5. Figures 16-18 are representative diagrams of the killing effects of various GPC3-ADCs on the Huh7 cells.

Table 5: $EC_{50}$ values of cell killing activities of various GPC3-ADCs in Huh7 cells

| Test substance | $EC_{50}$ value (ng/mL) |
|---|---|
| Hu 52H5D3B8-8-MC-AAN-Exa (ADC-1) | 3.59 |
| Hu 52H5D3B8-8-MCC-AAN-Exa (ADC-2) | 9.39 |
| Hu 52H5D3B8-8-MC-AAQ-Exa (ADC-3) | 11.90 |
| Hu 52H5D3B8-8-MCC-AAQ-Exa (ADC-4) | 14.92 |
| Hu 52H5D3B8-7-MCC-AAQ-Exa (ADC-5) | 16.38 |
| Hu 52H5D3B8-8-MCC-AAQ-Exa (ADC-6) | 30.65 |
| Hu 52H5D3B8-7-MCC-AAQ-Exa (ADC-7) | 27.92 |
| Hu 52H5D3B8-8-MCC-AA-(3-cyano-L-alanine)-Exa (ADC-8) | 7.34 |
| Hu 52H5D3B8-8-MCC-AAAAQ-Exa (ADC-9) | 4.78 |

Moreover, other ADCs of the present invention such as GPC3 (e.g., Hu 52H5D3B8-8)-mcc-Ala-Ala-((*S*)-2-amino-4-cyanobutanoic acid)-Exatecan also exhibited significant specific cell killing activities in the Huh7 cells.

As can be seen from the above results, such as those in Table 5 and Figures 16-18, the various GPC3-ADCs of the present invention all exhibited significant specific cell killing activities in the Huh7 cells. In addition, the corresponding GPC3-ADC molecules with a DAR of about 8 had a stronger cell killing effect than the GPC3-ADC with a DAR of about 4.

Example 6. Pharmacodynamic study *in vivo*

The *in vivo* anti-tumor activity of GPC3-ADC was tested in the LIV#219 PDX model of mice with human liver cancer.

The LIV219 PDX model had a higher expression level of GPC3 RNA, and a positive expression of GPC3 as confirmed by FACS analysis. The process of the establishment of a LIV#219 PDX model of nude mice with human liver

cancer (Genendesign Co., Ltd.) was as follows: A tumor tissue with a volume size of about 15-30 $mm^3$ was transplanted subcutaneously under the right rear back of the BALB/c nude mice. When the tumor volume reached 150-250 $mm^3$, the mice were grouped by the randomized block method, and the day when grouping was carried out was recorded as Day 0. Six mice were included in each group. Tumor volume uniformity and body weight balance between groups were ensured. There were a total of 15 groups, including vehicle group, Non-binding-MC-AAN-Exa (DAR8) (10 mg/kg) control administration group, Non-binding-MC-AAQ-Exa (DAR8) (10 mg/kg) control administration group, Non-binding-MCC-AAN-Exa (DAR8) (10 mg/kg) control administration group, Non-binding-MCC-AAQ-Exa (DAR8) (10 mg/kg) control administration group, Hu 52H5D3B8-8-MC-AAN-Exa (ADC-1, 10 mg/kg) administration group, Hu 52H5D3B8-8-MCC-AAN-Exa (ADC-2, 10 and 3 mg/kg) administration groups, Hu 52H5D3B8-8-MC-AAQ-Exa (ADC-3, 10 and 3 mg/kg) administration groups, and Hu 52H5D3B8-8-MCC-AAQ-Exa (ADC-4, 10 and 3 mg/kg) administration groups. On Day 0 and Day 7, administration was carried out via the tail vein once, respectively.

Data analysis: The mice were measured twice a week for body weight and tumor volume during the experiment. The calculation formula for the tumor volume (TV) is: $TV = l \times w^2 / 2$, in which l and w represent the measured length and width of the tumor, respectively. According to the measurement results, the relative tumor volume (RTV) was calculated by $RTV = V_f / V_0$, in which $V_0$ was the tumor volume measured at the time of grouping for administration (i.e., Day 0), and $V_f$ was the tumor volume measured on the last day. Relative tumor proliferation rate (T/C) (%) = (RTV in drug administration group / RTV in vehicle group) × 100%. When T/C (%) ≤ 40% and $P < 0.05$, the test sample was believed to have a significant inhibitory effect on tumor growth.

The experimental results are as shown in Figures 19 and 20. On Day 29, the relative tumor proliferation rate T/C (%) of the Hu 52H5D3B8-8-MC-AAN-Exa (ADC-1, 10 mg/kg) administration group was 7.88% ($P < 0.05$); the relative tumor proliferation rates T/C (%) of the Hu 52H5D3B8-8-MCC-AAN-Exa (ADC-2, 10 and 3 mg/kg) administration groups were 12.44% ($P < 0.05$) and 77.71% ($P > 0.05$), respectively; the relative tumor proliferation rates T/C (%) of the Hu 52H5D3B8-8-MC-AAQ-Exa (ADC-3, 10 and 3 mg/kg) administration groups were 6.75% ($P < 0.05$) and 47.29% ($P > 0.05$), respectively; and the relative tumor proliferation rates T/C (%) of the Hu 52H5D3B8-8-MCC-AAQ-Exa (ADC-4, 10 and 3 mg/kg) administration groups were 7.37% ($P < 0.05$) and 53.71% ($P > 0.05$), respectively. The change of the average body weight of the mice in each group at Day 29 compared to Day 0 was within 0.87%-4.88%.

The experimental results showed that at a dose of 10 mg/kg, all ADCs had a significant inhibitory effect on tumor growth; and at a dose of 3 mg/kg, Hu 52H5D3B8-8-MC-AAQ-Exa (ADC-3) and Hu 52H5D3B8-8-MCC-AAQ-Exa (ADC-4) exhibited a smaller relative tumor growth proliferation rate. The tumor-bearing mice were well tolerated to all test samples.

Example 7. Pharmacodynamic study *in vivo*

The *in vivo* anti-tumor activities of Hu 52H5D3B8-8-MCC-AAN-Exa (ADC-2) and Hu 52H5D3B8-8-MCC-AAQ-Exa (ADC-4) were also tested in the LI6653 PDX model of mice with human liver cancer for comparison.

The LI6653 PDX model (Sino-American Crown Bioscience (Taicang) Co., Ltd.) was confirmed for its positive expression of GPC3 (++) by the IHC test. The process of the establishment of a PDX model of nude mice with human liver cancer was as follows: Tumor tissues were harvested from tumor-bearing mice in a model with a human-derived liver cancer xenograft, cut into tumor fragments with a diameter of 2-3 mm, and inoculated subcutaneously at the right anterior scapular region of BALB/c Nude mice. When the average tumor volume of the tumor-bearing mice reached about 170-200 $mm^3$, they were randomized and administrated according to tumor size, and the day when grouping was carried out was recorded as Day 0. Six mice were included in each group. Tumor volume uniformity and body weight balance between groups were ensured. There were a total of 6 groups, including vehicle group, Non-binding-MCC-AAN-Exa (DAR8) (10 mg/kg) control administration group, Hu 52H5D3B8-8-MCC-AAN-Exa (ADC-2, 10 and 3 mg/kg) administration groups, and Hu 52H5D3B8-8-MCC-AAQ-Exa (ADC-4, 10 and 3 mg/kg) administration groups. On Day 0 and Day 7, administration was carried out via the tail vein once, respectively.

Data analysis: The mice were measured twice a week for body weight and tumor volume during the experiment. The calculation formula for the tumor volume (TV) is: $TV = l \times w^2 / 2$, in which l and w represent the measured length and width of the tumor, respectively. According to the measurement results, the relative tumor volume (RTV) was calculated by $RTV = V_f / V_0$, in which $V_0$ was the tumor volume measured at the time of grouping for administration (i.e., Day 0), and $V_f$ was the tumor volume measured on the last day. Relative tumor proliferation rate (T/C) (%) = (RTV in drug administration group / RTV in vehicle group) × 100%. When T/C (%) ≤ 40% and $P < 0.05$, the test sample was believed to have a significant inhibitory effect on tumor growth.

The experimental results are as shown in Figures 21 and 22. On Day 16, the relative tumor proliferation rates T/C (%) of the Hu 52H5D3B8-8-MCC-AAN-Exa (ADC-2, 10 and 3 mg/kg) administration groups were 17.46% ($P < 0.05$) and 31.81% ($P < 0.05$), respectively; and the relative tumor proliferation rates T/C (%) of the Hu 52H5D3B8-8-MCC-AAQ-Exa (ADC-4, 10 and 3 mg/kg) administration groups were 13.05% ($P < 0.05$) and 21.10% ($P < 0.05$), respectively. The change of the average body weight of the mice in each group at Day 16 compared to Day 0 was within 4.38%-11.97%.

The experimental results showed that at a dose of 10 mg/kg, both ADCs had a significant inhibitory effect on

tumor growth; and at a dose of 3 mg/kg, Hu 52H5D3B8-8-MCC-AAQ-Exa (ADC-4) exhibited a smaller relative tumor growth proliferation rate compared to Hu 52H5D3B8-8-MCC-AAN-Exa (ADC-2). The tumor-bearing mice were well tolerated to all test samples.

## Claims

1. An antibody drug conjugate, or a pharmaceutically acceptable salt or solvate thereof, or a solvate of the salt, **characterized in that** the antibody drug conjugate has a structure represented by formula I,

$$Ab\text{-}(L\text{-}D)_p \qquad \text{formula I}$$

in which:

Ab is an anti-GPC3 antibody or an antigen binding fragment thereof, and the anti-GPC3 antibody comprises a heavy chain variable region and a light chain variable region, wherein CDR1, CDR2, and CDR3 in the heavy chain variable region comprise the sequences as set forth in SEQ ID NO: 1, SEQ ID NO: 2, and SEQ ID NO: 3, or mutants thereof, respectively, and CDR1, CDR2, and CDR3 in the light chain variable region comprise the sequences as set forth in SEQ ID NO: 9, SEQ ID NO: 10, and SEQ ID NO: 11, or mutants thereof, respectively;
L is a linker selected from mc-AAN, mc-AAQ, mcc-AAN, mcc-AAQ, mcc-Ala-Ala-(3-cyano-alanine), mcc-Ala-Ala-(2-amino-4-cyanobutanoic acid), and mcc-Ala-Ala-Ala-Ala-Gln;
D is a cytotoxin, which is Exatecan; and
p is any numerical value between 1 and 10; and
wherein the mcc or mc end of the L linker is covalently connected to Ab, and the other end of the L linker is covalently connected to D.

2. The antibody drug conjugate, or the pharmaceutically acceptable salt or solvate thereof, or the solvate of the salt according to claim 1, **characterized in that** the structural formula of L-D before conjugation to Ab is as shown below:

| | |
|---|---|
| 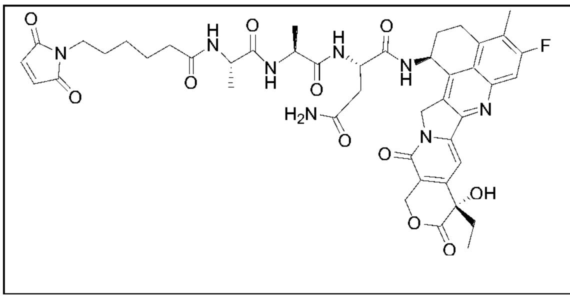 | 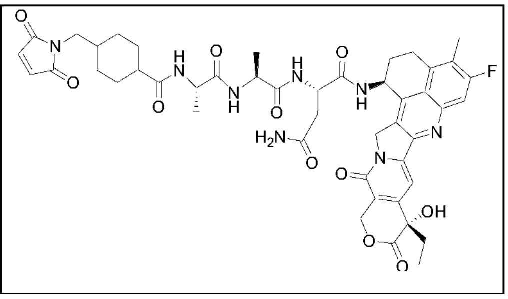 |

| | |
|---|---|
| 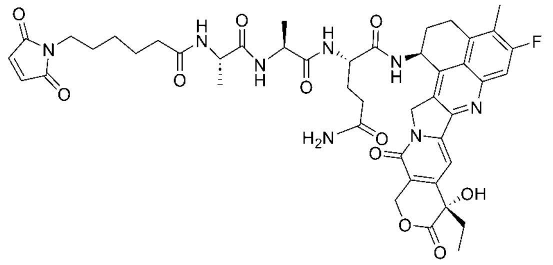 | 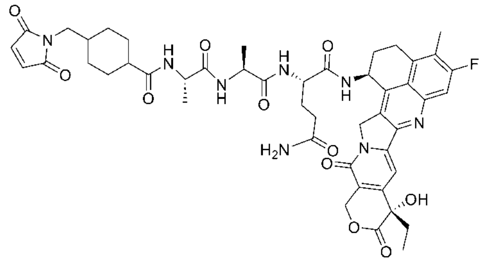 |
| 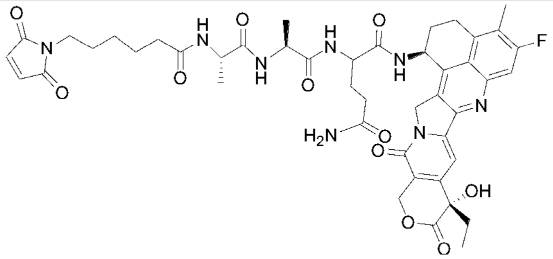 | 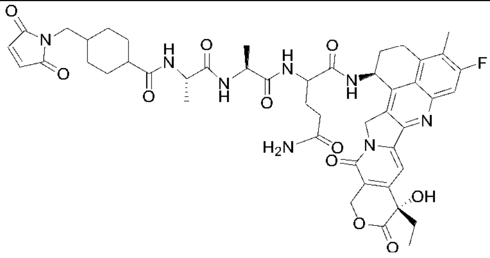 |
| 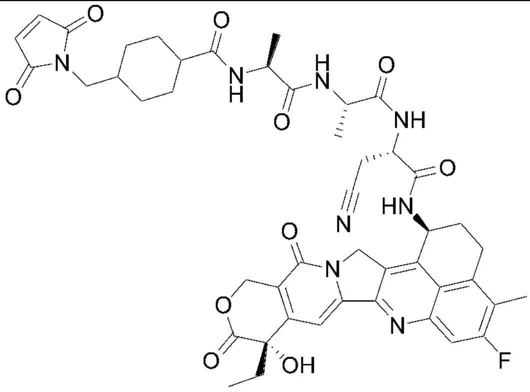 | 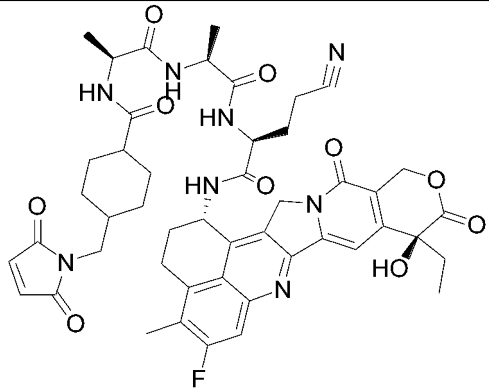 |
| 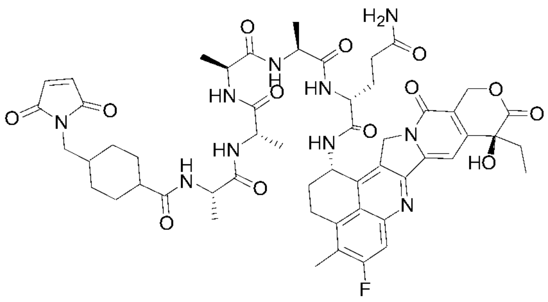 | |

in which succinimide at one end of each L-D is conjugated to mercapto in Ab.

3. The antibody drug conjugate, or the pharmaceutically acceptable salt or solvate thereof, or the solvate of the salt according to either claim 1 or 2, **characterized in that** the antibody drug conjugate has a structure represented by formula (IIa), (IIb), (IIc), (IId), (IIe), (IIf), (IIg), (IIh), or (IIi),

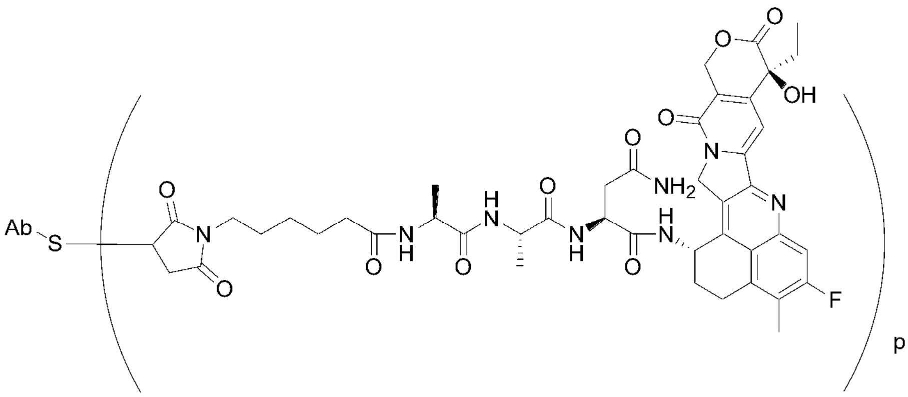

(IIa),

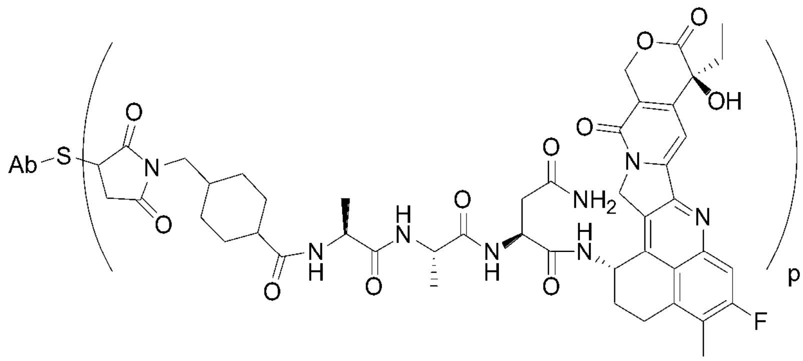

(IIb),

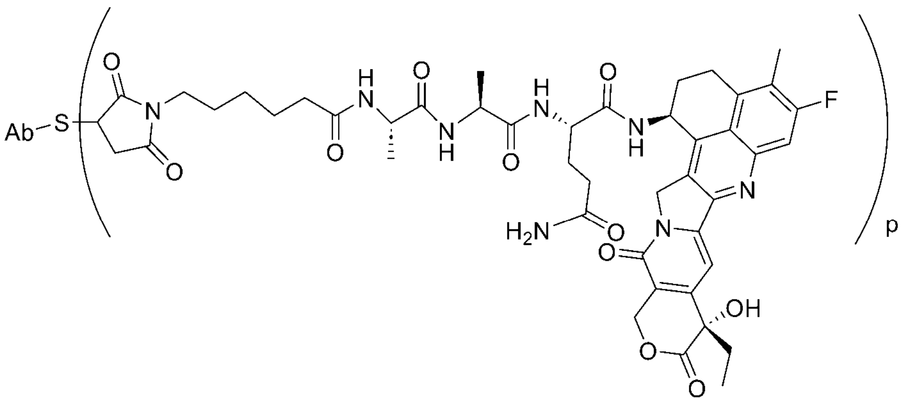

(IIc),

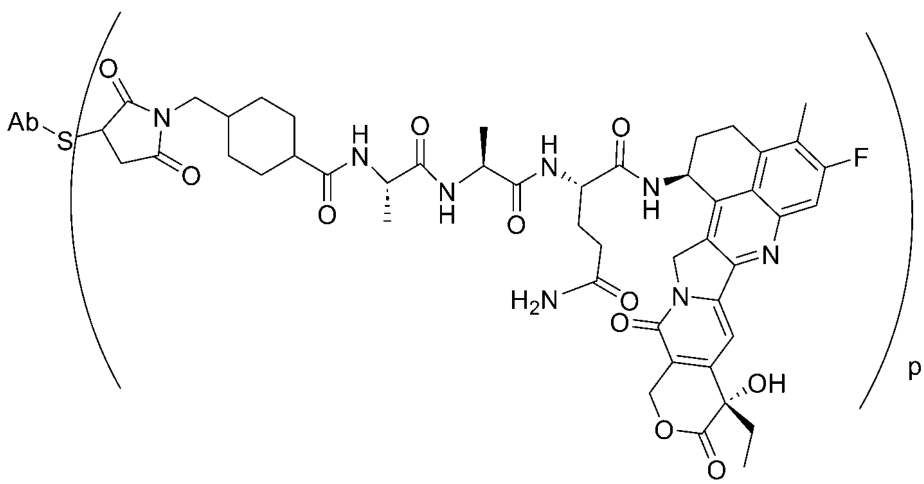

(IId),

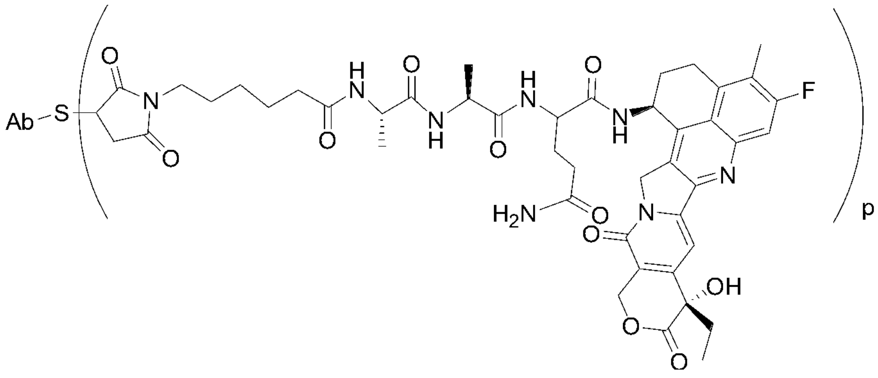

(IIe),

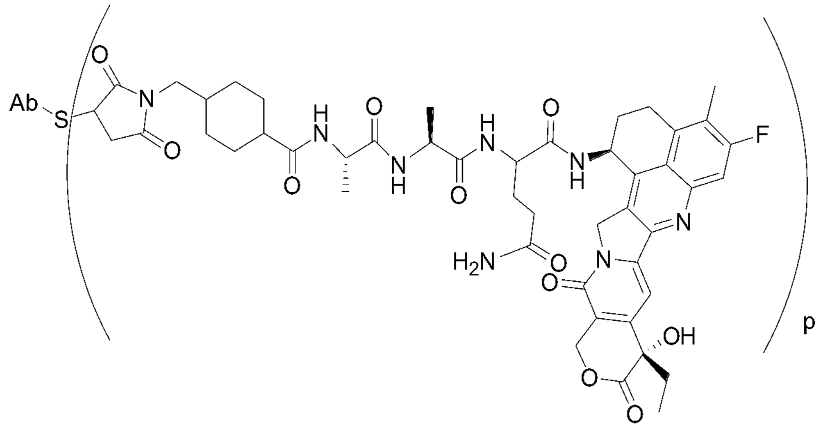

(IIf),

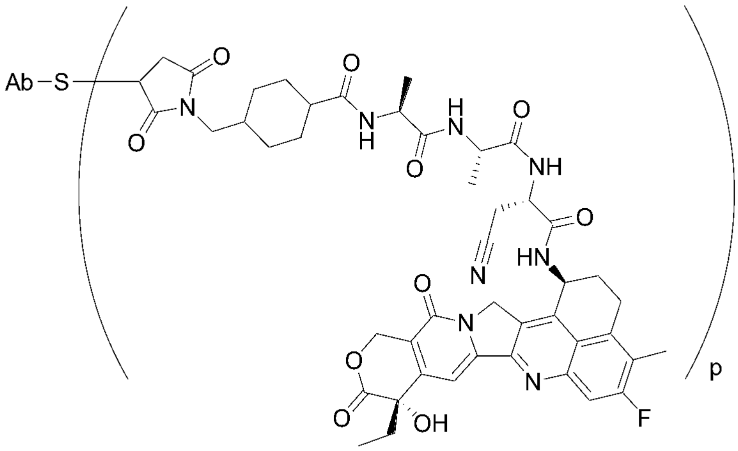

(IIg),

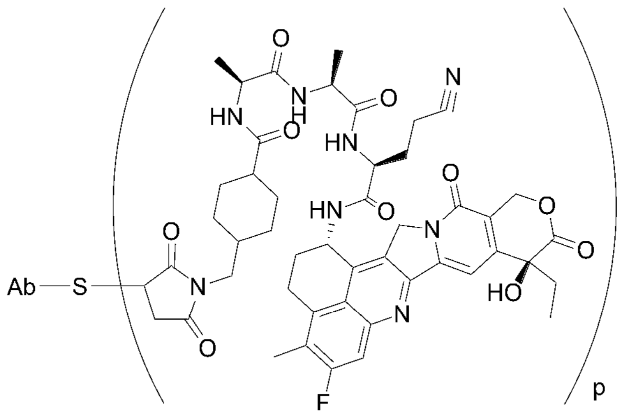

(IIh),

or

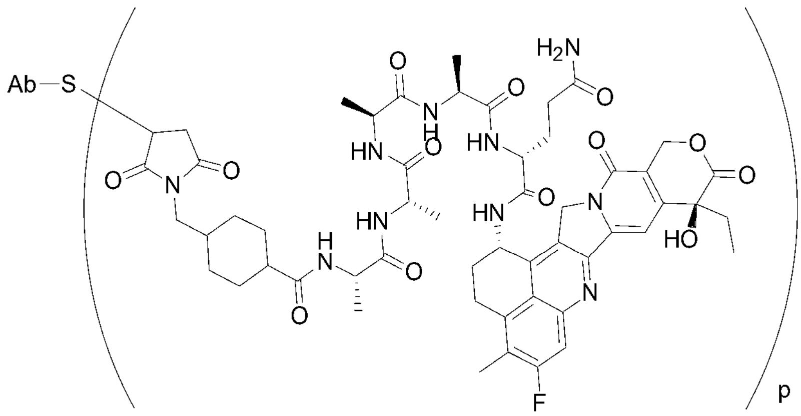

(IIi).

**4.** The antibody drug conjugate, or the pharmaceutically acceptable salt or solvate thereof, or the solvate of the salt according to any one of claims 1-3, **characterized in that** the anti-GPC3 antibody has one or both of the technical features selected from (1) and (2) below:

(1) FR1, FR2, FR3, and FR4 regions in the heavy chain variable region of the anti-GPC3 antibody comprise the sequences as set forth in SEQ ID NO: 4, SEQ ID NO: 5, SEQ ID NO: 6, and SEQ ID NO: 7, or mutants thereof, respectively; and
(2) FR1, FR2, FR3, and FR4 regions in the light chain variable region of the anti-GPC3 antibody comprise the sequences as set forth in SEQ ID NO: 12, SEQ ID NO: 13, SEQ ID NO: 14, and SEQ ID NO: 15, or mutants thereof, respectively, or FR1, FR2, FR3, and FR4 regions in the light chain variable region of the anti-GPC3 antibody comprise the sequences as set forth in SEQ ID NO: 12, SEQ ID NO: 13, SEQ ID NO: 17, and SEQ ID NO: 15, or mutants thereof, respectively.

**5.** The antibody drug conjugate, or the pharmaceutically acceptable salt or solvate thereof, or the solvate of the salt according to any one of claims 1-4, **characterized in that** the anti-GPC3 antibody has one or both of the technical features selected from (1) and (2) below:

(1) the heavy chain variable region of the anti-GPC3 antibody comprises the sequence as set forth in SEQ ID NO: 8 or a mutant thereof; and
(2) the light chain variable region of the anti-GPC3 antibody comprises the sequence as set forth in SEQ ID NO: 16 or SEQ ID NO: 18, or a mutant thereof.

**6.** The antibody drug conjugate, or the pharmaceutically acceptable salt or solvate thereof, or the solvate of the salt according to any one of claims 1-5, **characterized in that** the anti-GPC3 antibody has one or both of the technical features selected from (1) and (2) below:

(1) a heavy chain constant region of the anti-GPC3 antibody is selected from human IgG, IgM, IgA, IgD, and IgE constant regions, or mutants of the constant regions; and
preferably, the IgG is selected from IgG1, IgG2, IgG3, and IgG4; and
(2) a light chain constant region of the anti-GPC3 antibody is selected from human lambda constant region or kappa constant region, or a mutant of the constant region.

**7.** The antibody drug conjugate, or the pharmaceutically acceptable salt or solvate thereof, or the solvate of the salt according to any one of claims 1-6, **characterized in that** the anti-GPC3 antibody has one or both of the technical features selected from (1) and (2) below:

(1) the heavy chain constant region of the anti-GPC3 antibody comprises the sequence as set forth in SEQ ID NO: 19, or a sequence with more than 70%, preferably more than 75%, 80%, 85%, 90%, 95%, or 99% homology with the sequence as set forth in SEQ ID NO: 19; and
preferably, the sequence of the heavy chain constant region of the anti-GPC3 antibody is as set forth in SEQ ID NO: 19; and
(2) the light chain constant region of the anti-GPC3 antibody comprises the sequence as set forth in SEQ ID NO: 20, or a sequence with more than 70%, preferably more than 75%, 80%, 85%, 90%, 95%, or 99% homology with the sequence as set forth in SEQ ID NO: 20; and

preferably, the sequence of the light chain constant region of the anti-GPC3 antibody is as set forth in SEQ ID NO: 20.

**8.** The antibody drug conjugate, or the pharmaceutically acceptable salt or solvate thereof, or the solvate of the salt according to any one of claims 1-7, **characterized in that** p is any numerical value between 2 and 8; and preferably, p is any numerical value between 4 and 8 (e.g., 4.0, 7.9, or 8.0).

**9.** A pharmaceutical composition comprising the antibody drug conjugate, or the pharmaceutically acceptable salt or solvate thereof, or the solvate of the salt according to any one of claims 1-8;

optionally, the pharmaceutical composition further comprises at least one pharmaceutical auxiliary material;
or optionally, the pharmaceutical composition further comprises at least one of a chemotherapeutic drug, an immunotherapeutic drug, and an immunosuppressant for treating a tumor.

**10.** Use of the antibody drug conjugate, or the pharmaceutically acceptable salt or solvate thereof, or the solvate of the salt according to any one of claims 1-8 or the pharmaceutical composition according to claim 9 in the preparation of a drug for preventing and/or treating a GPC3 positivity-associated disease,

preferably, the GPC3 positivity-associated disease is selected from liver cancer, lung cancer, gastric cancer, head and neck cancer, esophageal cancer, Merkel cell cancer, liposarcoma, breast cancer, ovarian cancer, melanoma, squamous cell carcinoma, renal cell carcinoma, pancreatic cancer, prostate cancer, colorectal cancer, glioma, kidney cancer, bladder cancer, cervical cancer, gallbladder carcinoma, and glioblastoma; and
more preferably, the GPC3 positivity-associated disease is liver cancer.

**11.** A method for non-therapeutically inhibiting tumor angiogenesis, delaying tumor progression, inhibiting tumor growth or inhibiting tumor cell proliferation *in vitro*, comprising: contacting a tumor cell with the antibody drug conjugate, or the pharmaceutically acceptable salt or solvate thereof, or the solvate of the salt according to any one of claims 1-8 or the pharmaceutical composition according to claim 9, wherein the tumor is a GPC3-expressing tumor;

preferably, the GPC3-expressing tumor is selected from liver cancer, lung cancer, gastric cancer, head and neck cancer, esophageal cancer, Merkel cell cancer, liposarcoma, breast cancer, ovarian cancer, melanoma, squamous cell carcinoma, renal cell carcinoma, pancreatic cancer, prostate cancer, colorectal cancer, glioma, kidney cancer, bladder cancer, cervical cancer, gallbladder carcinoma, and glioblastoma; and
more preferably, the GPC3-expressing tumor is liver cancer.

**12.** A method for treating and/or preventing a GPC3 positivity-associated disease, comprising: administering to a subject in need thereof a therapeutically and/or prophylactically effective amount of the antibody drug conjugate, or the pharmaceutically acceptable salt or solvate thereof, or the solvate of the salt according to any one of claims 1-8 or the pharmaceutical composition according to claim 9,

preferably, the GPC3 positivity-associated disease is selected from liver cancer, lung cancer, gastric cancer, head and neck cancer, esophageal cancer, Merkel cell cancer, liposarcoma, breast cancer, ovarian cancer, melanoma, squamous cell carcinoma, renal cell carcinoma, pancreatic cancer, prostate cancer, colorectal cancer, glioma, kidney cancer, bladder cancer, cervical cancer, gallbladder carcinoma, and glioblastoma; and
more preferably, the GPC3 positivity-associated disease is liver cancer.

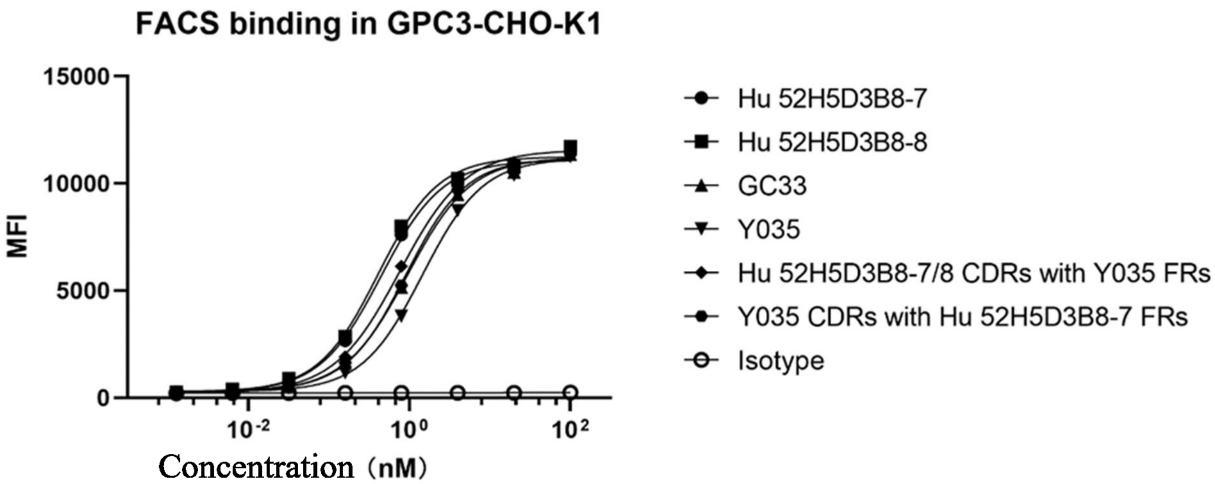


*FIG. 1*

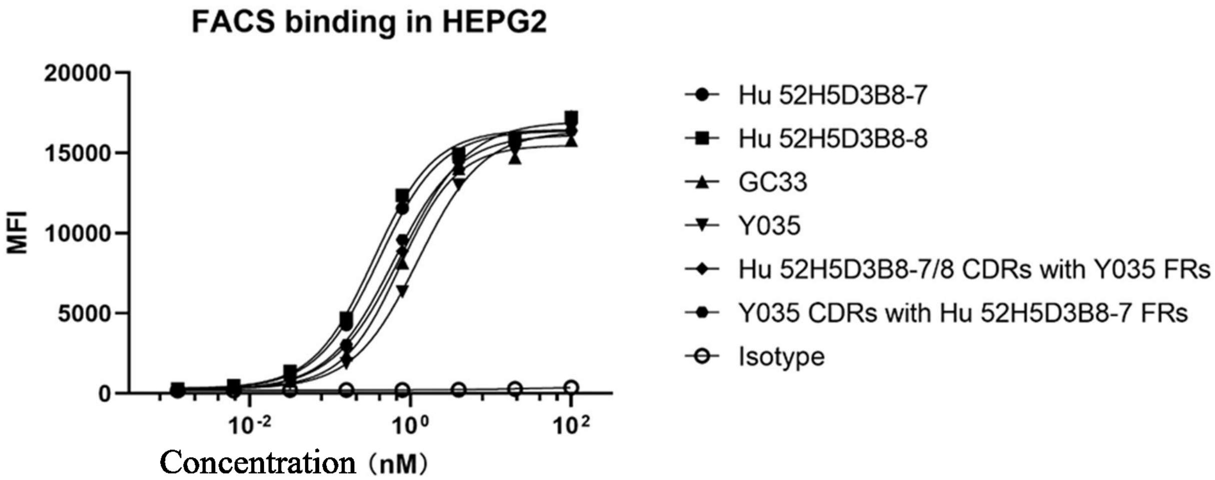


*FIG. 2*

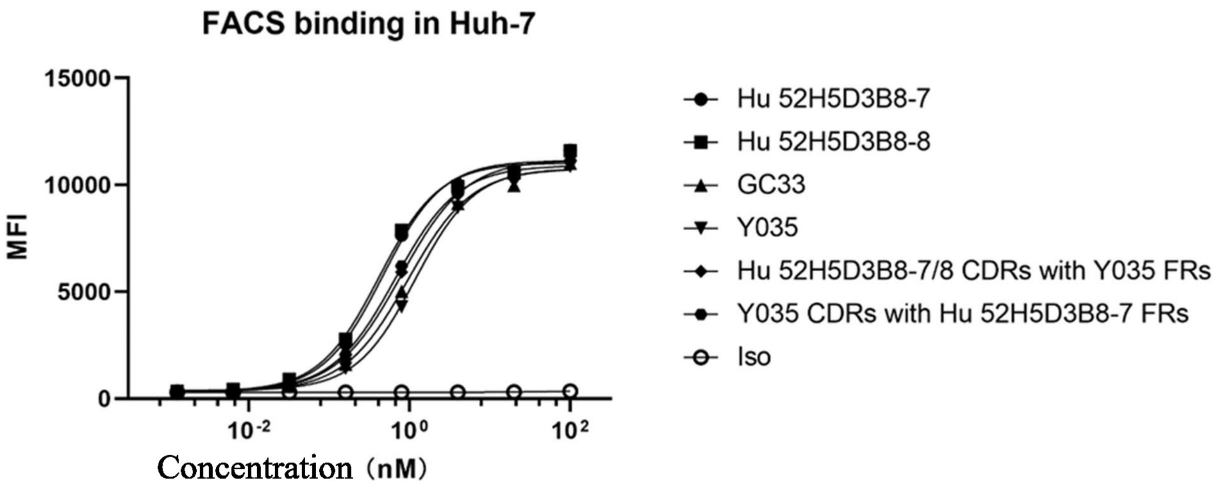


*FIG. 3*

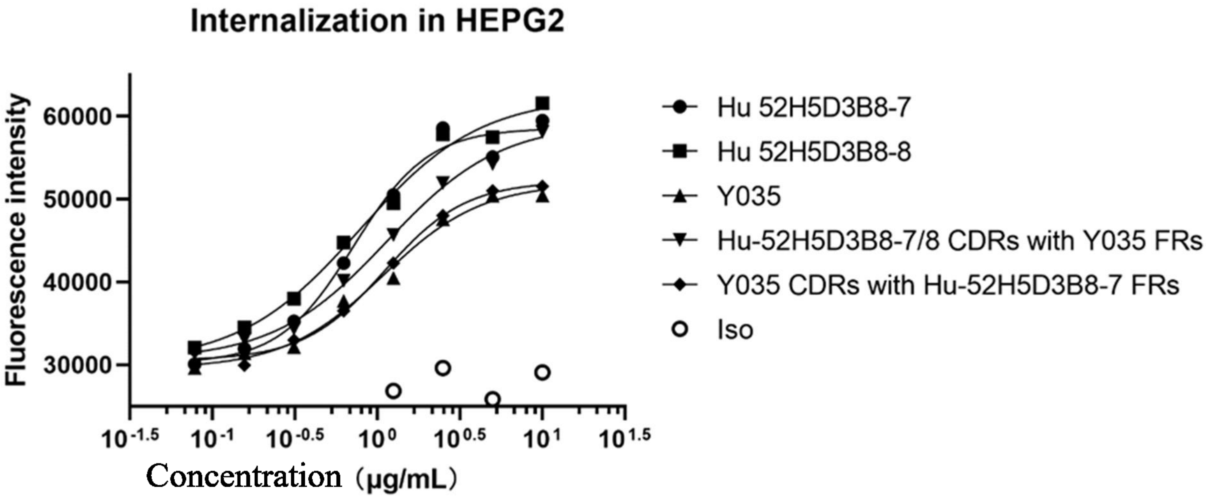


*FIG. 4*

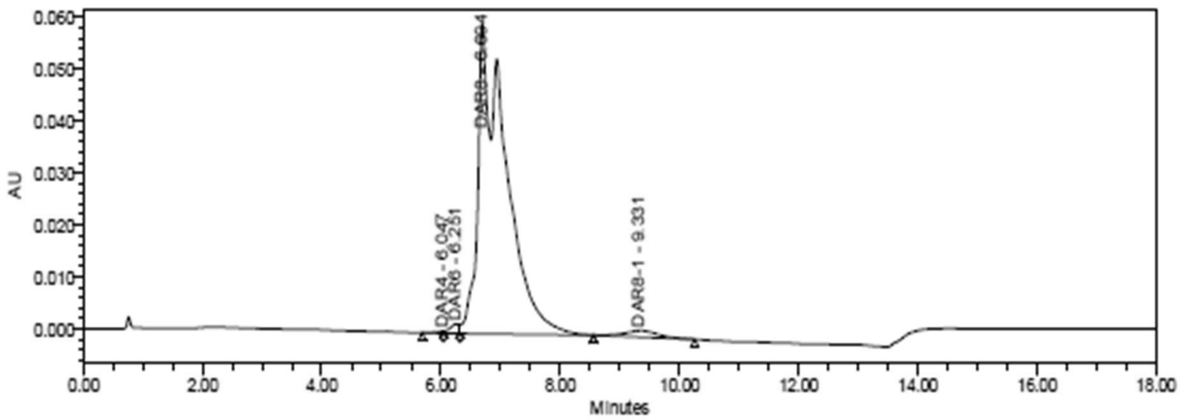


*FIG. 5*

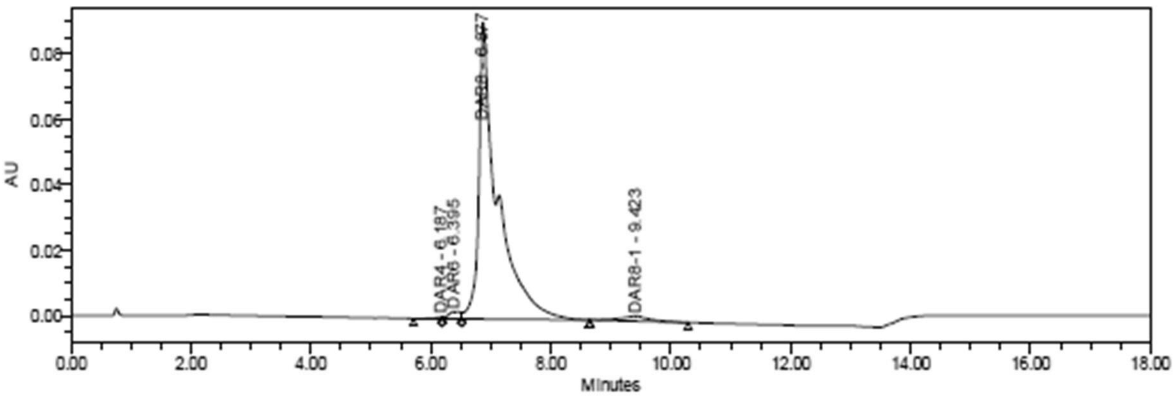


*FIG. 6*

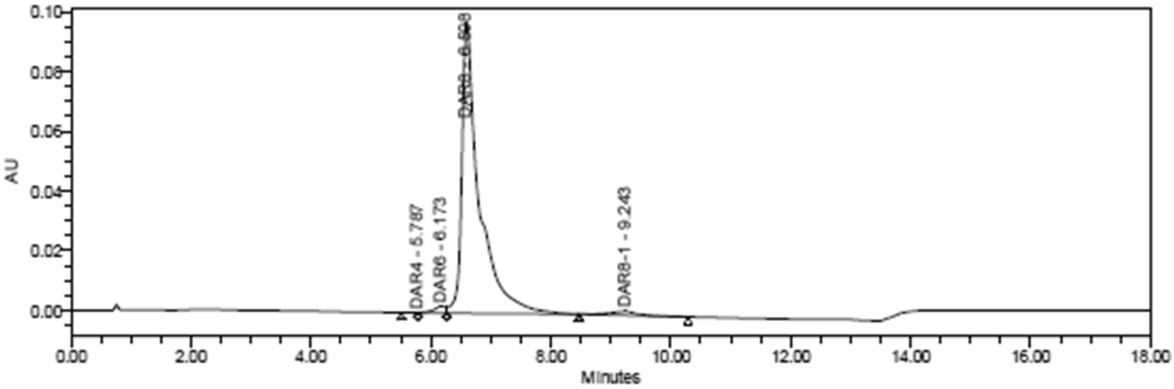


*FIG. 7*

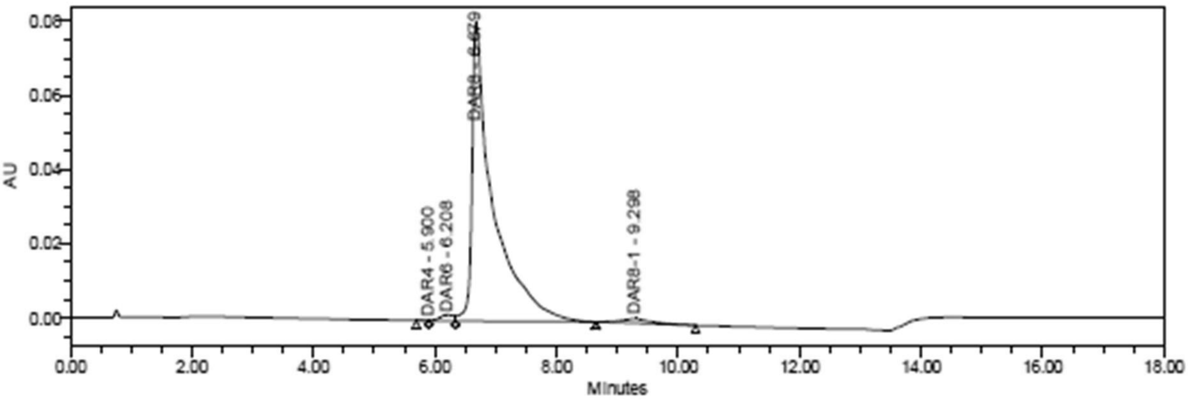


*FIG. 8*

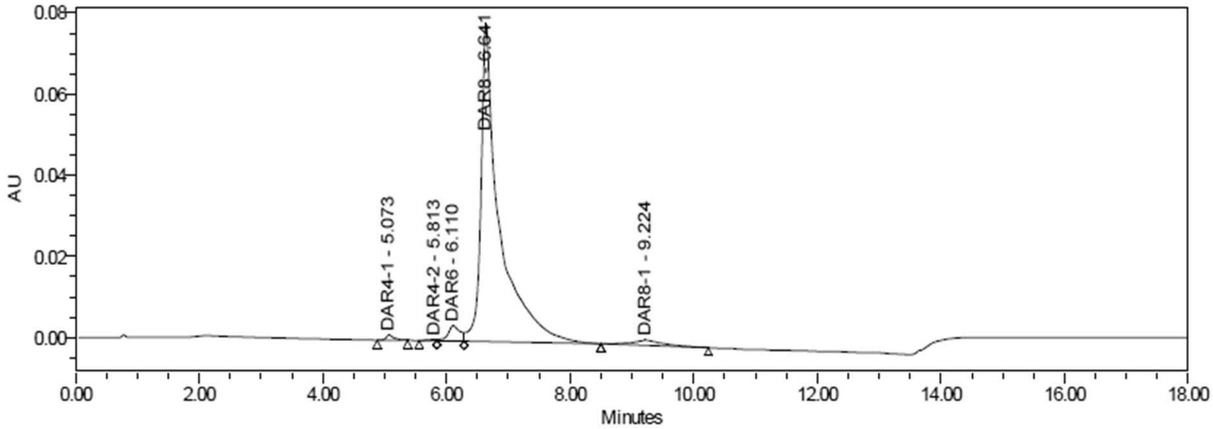


*FIG. 9*

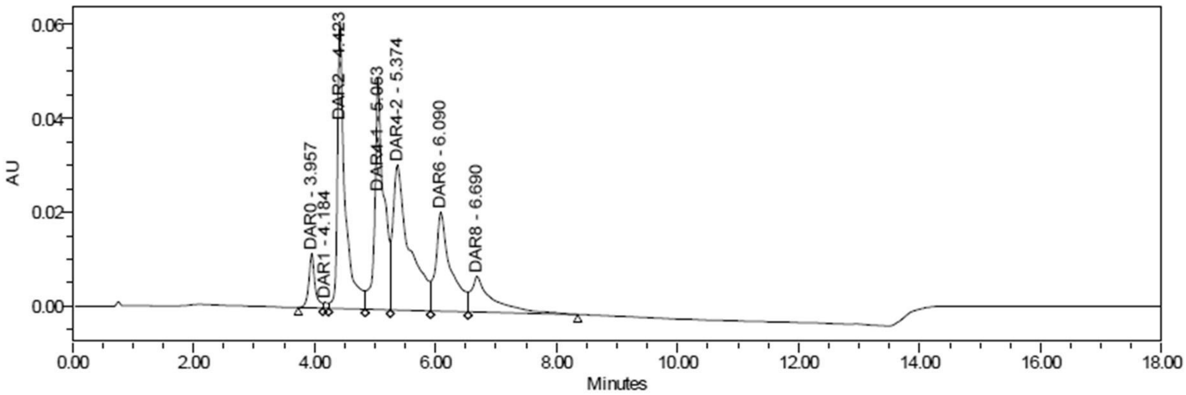


*FIG. 10*

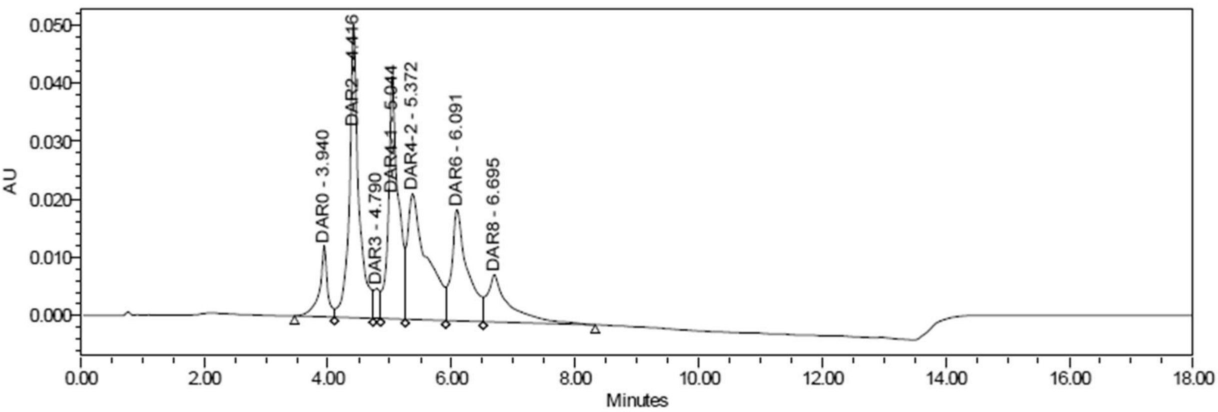


*FIG. 11*

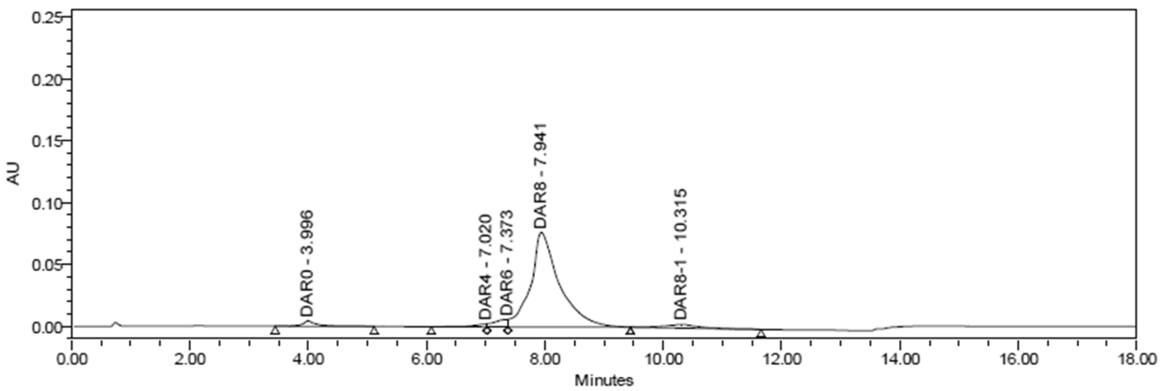


*FIG. 12*

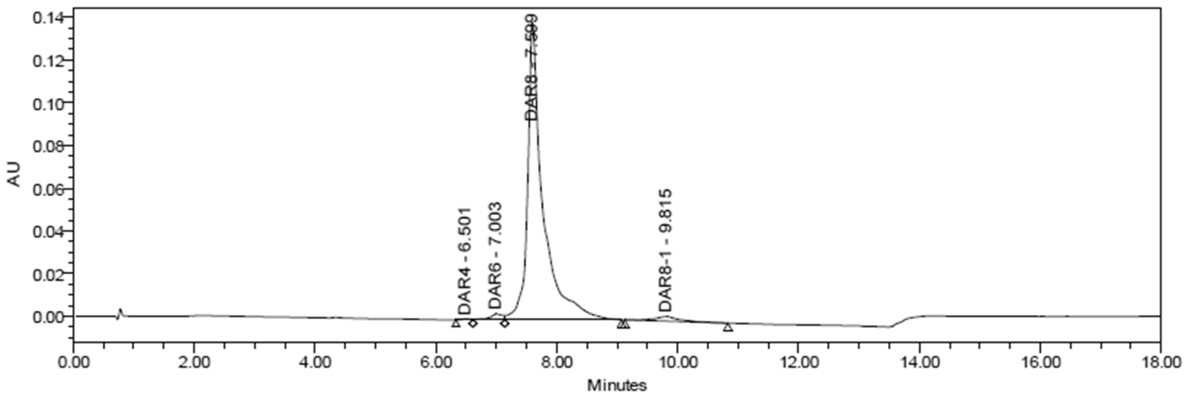
AU
0.14
0.12
0.10
0.08
0.06
0.04
0.02
0.00
DAR4 - 6.501
DAR6 - 7.003
DAR8 - 7.599
DAR8-1 - 9.815
0.00
2.00
4.00
6.00
8.00
10.00
12.00
14.00
16.00
18.00
Minutes

*FIG. 13*

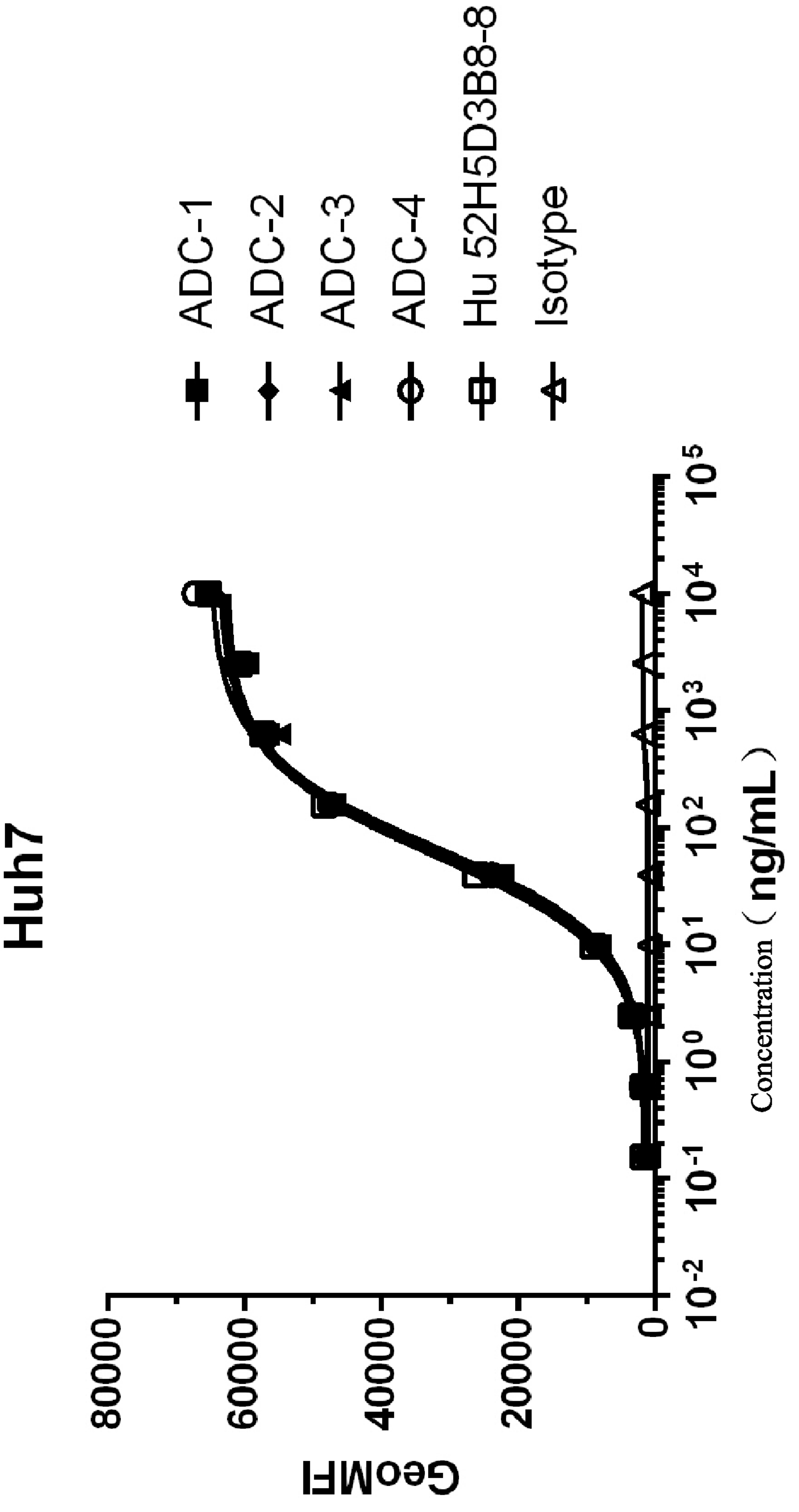
Huh7
GeoMFI
80000
60000
40000
20000
0
10-2
10-1
100
101
102
103
104
105
Concentration（ng/mL）
ADC-1
ADC-2
ADC-3
ADC-4
Hu 52H5D3B8-8
Isotype

*FIG. 14*

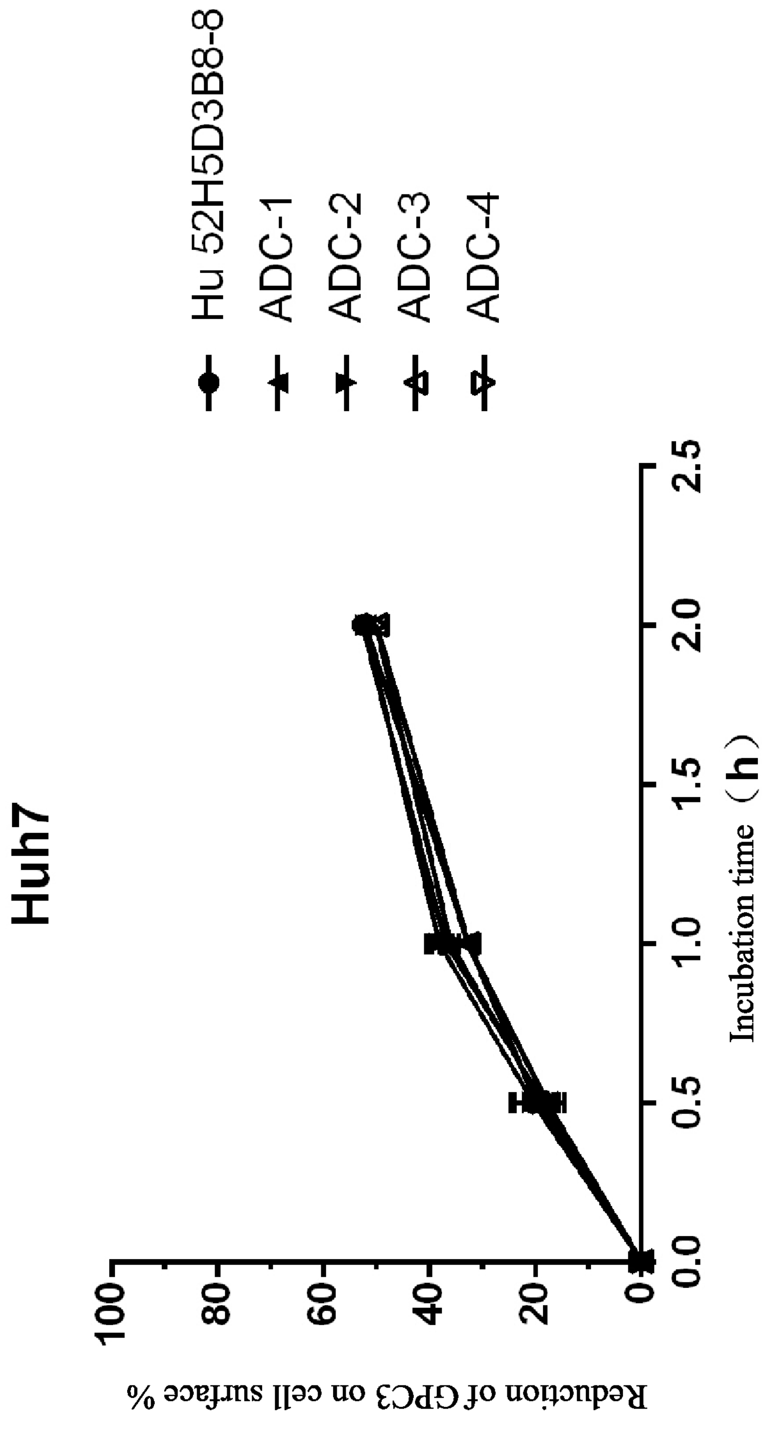
Huh7
Reduction of GPC3 on cell surface %
100
80
60
40
20
0
0.0
0.5
1.0
1.5
2.0
2.5
Incubation time（h）
Hu 52H5D3B8-8
ADC-1
ADC-2
ADC-3
ADC-4

*FIG. 15*

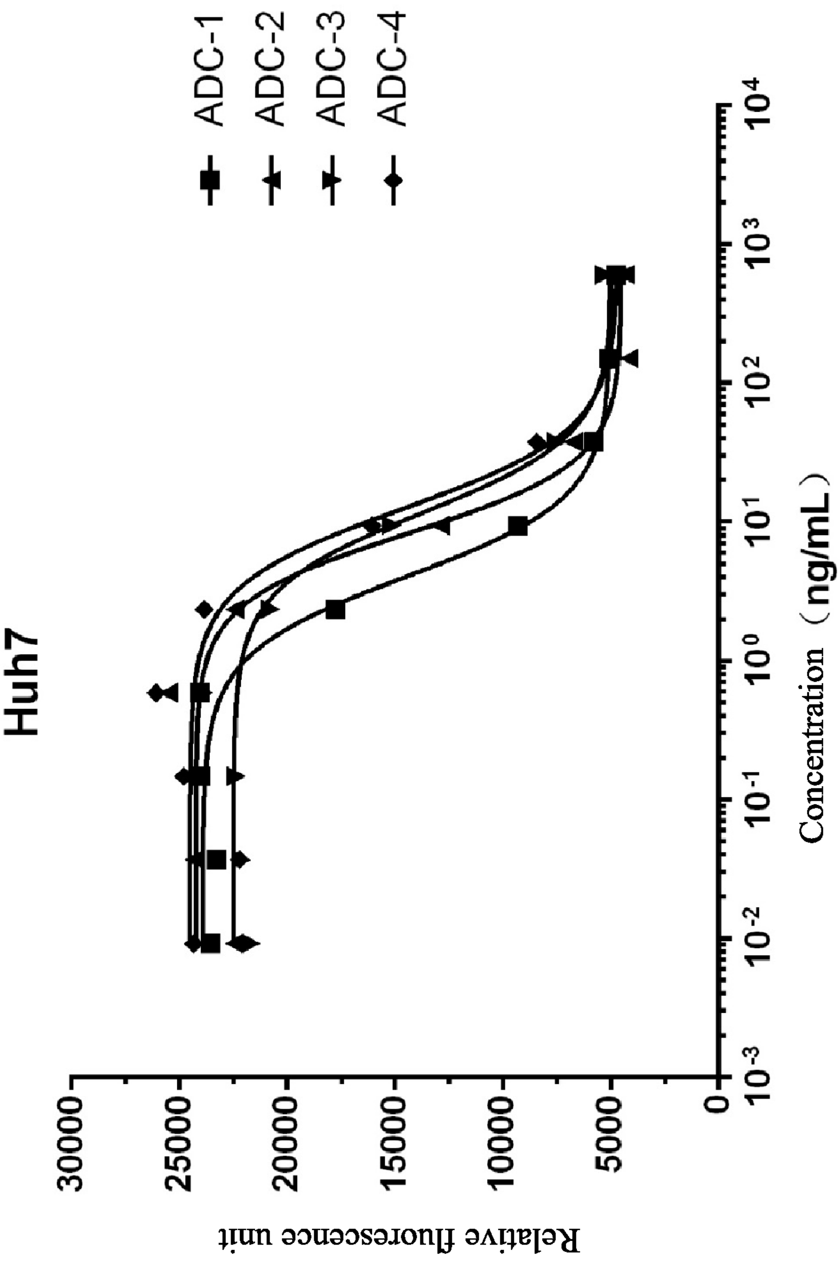
Huh7
Relative fluorescence unit
30000
25000
20000
15000
10000
5000
0
Concentration（ng/mL）
ADC-1
ADC-2
ADC-3
ADC-4

*FIG. 16*

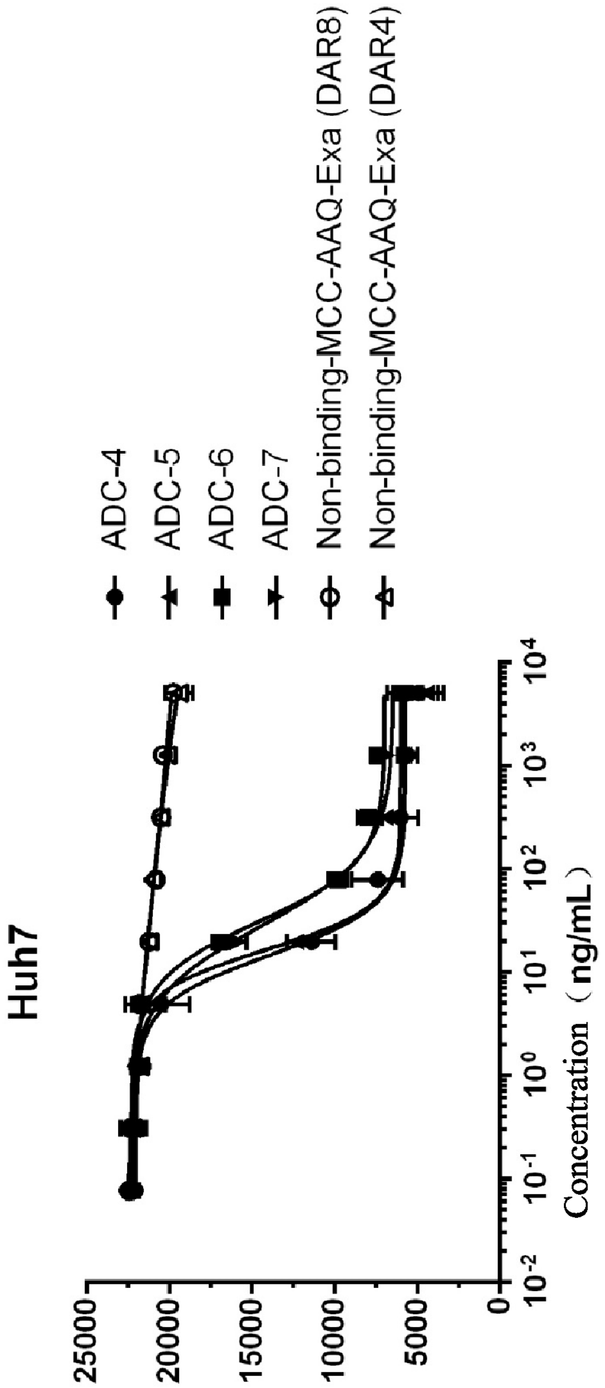
Huh7
Relative fluorescence unit
25000
20000
15000
10000
5000
0
$10^{-2}$
$10^{-1}$
$10^{0}$
$10^{1}$
$10^{2}$
$10^{3}$
$10^{4}$
Concentration（ng/mL）
ADC-4
ADC-5
ADC-6
ADC-7
Non-binding-MCC-AAQ-Exa (DAR8)
Non-binding-MCC-AAQ-Exa (DAR4)

*FIG. 17*

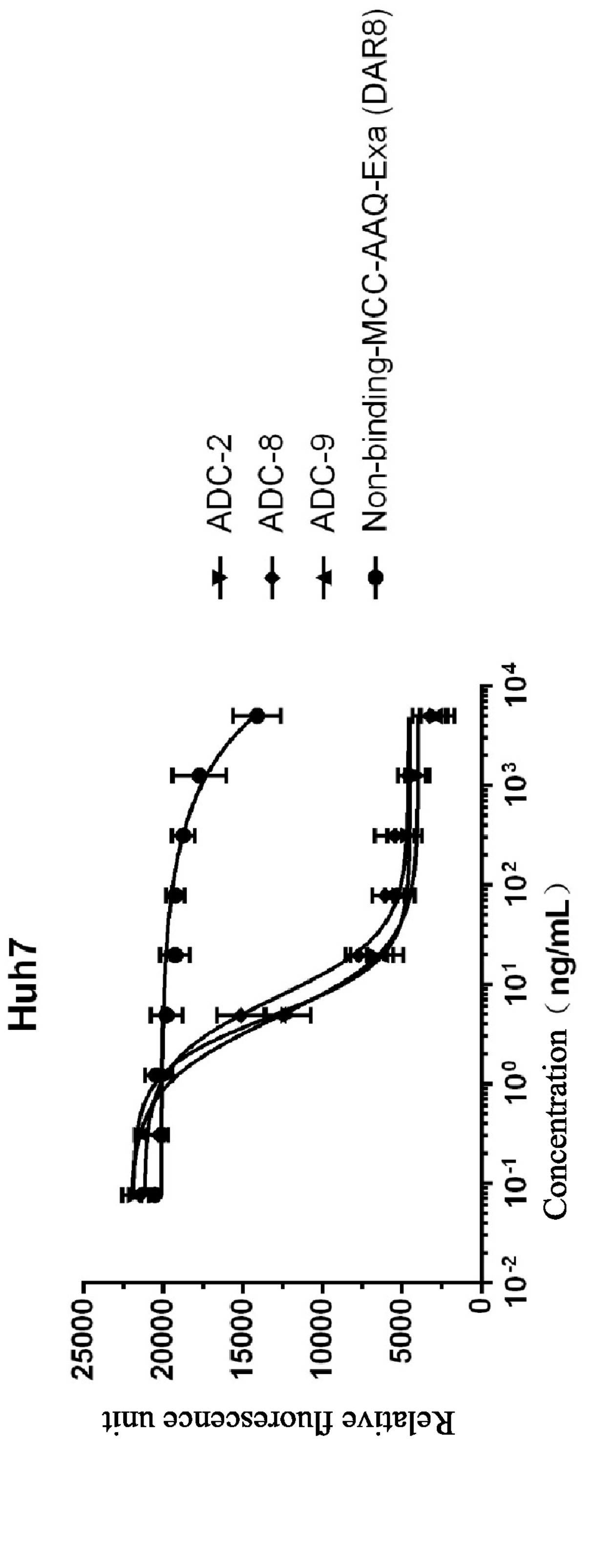

Huh7
Relative fluorescence unit
25000
20000
15000
10000
5000
0
$10^{-2}$
$10^{-1}$
$10^{0}$
$10^{1}$
$10^{2}$
$10^{3}$
$10^{4}$
Concentration（ng/mL）
ADC-2
ADC-8
ADC-9
Non-binding-MCC-AAQ-Exa (DAR8)


*FIG. 18*

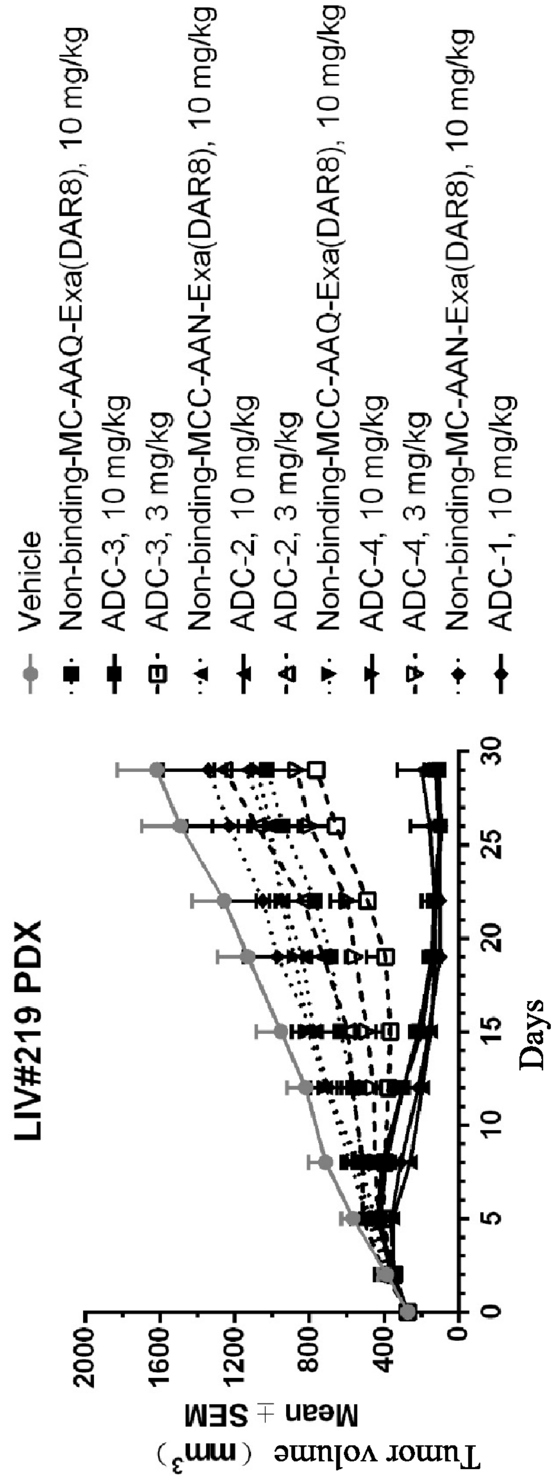
LIV#219 PDX
Tumor volume (mm3)
Mean ± SEM
2000
1600
1200
800
400
0
0
5
10
15
20
25
30
Days
Vehicle
Non-binding-MC-AAQ-Exa(DAR8), 10 mg/kg
ADC-3, 10 mg/kg
ADC-3, 3 mg/kg
Non-binding-MCC-AAN-Exa(DAR8), 10 mg/kg
ADC-2, 10 mg/kg
ADC-2, 3 mg/kg
Non-binding-MCC-AAQ-Exa(DAR8), 10 mg/kg
ADC-4, 10 mg/kg
ADC-4, 3 mg/kg
Non-binding-MC-AAN-Exa(DAR8), 10 mg/kg
ADC-1, 10 mg/kg

*FIG. 19*

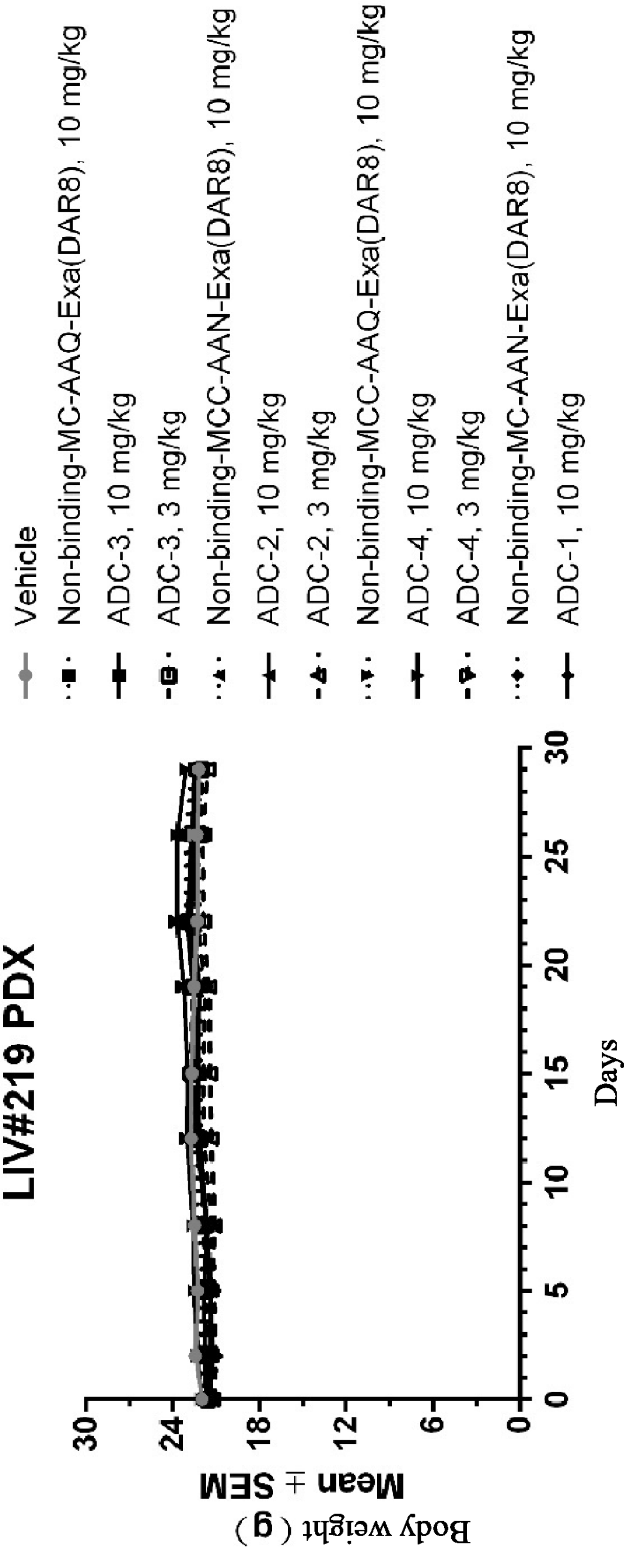
LIV#219 PDX
Body weight ( g )
Mean ± SEM
30
24
18
12
6
0
0
5
10
15
20
25
30
Days
Vehicle
Non-binding-MC-AAQ-Exa(DAR8), 10 mg/kg
ADC-3, 10 mg/kg
ADC-3, 3 mg/kg
Non-binding-MCC-AAN-Exa(DAR8), 10 mg/kg
ADC-2, 10 mg/kg
ADC-2, 3 mg/kg
Non-binding-MCC-AAQ-Exa(DAR8), 10 mg/kg
ADC-4, 10 mg/kg
ADC-4, 3 mg/kg
Non-binding-MC-AAN-Exa(DAR8), 10 mg/kg
ADC-1, 10 mg/kg

*FIG. 20*

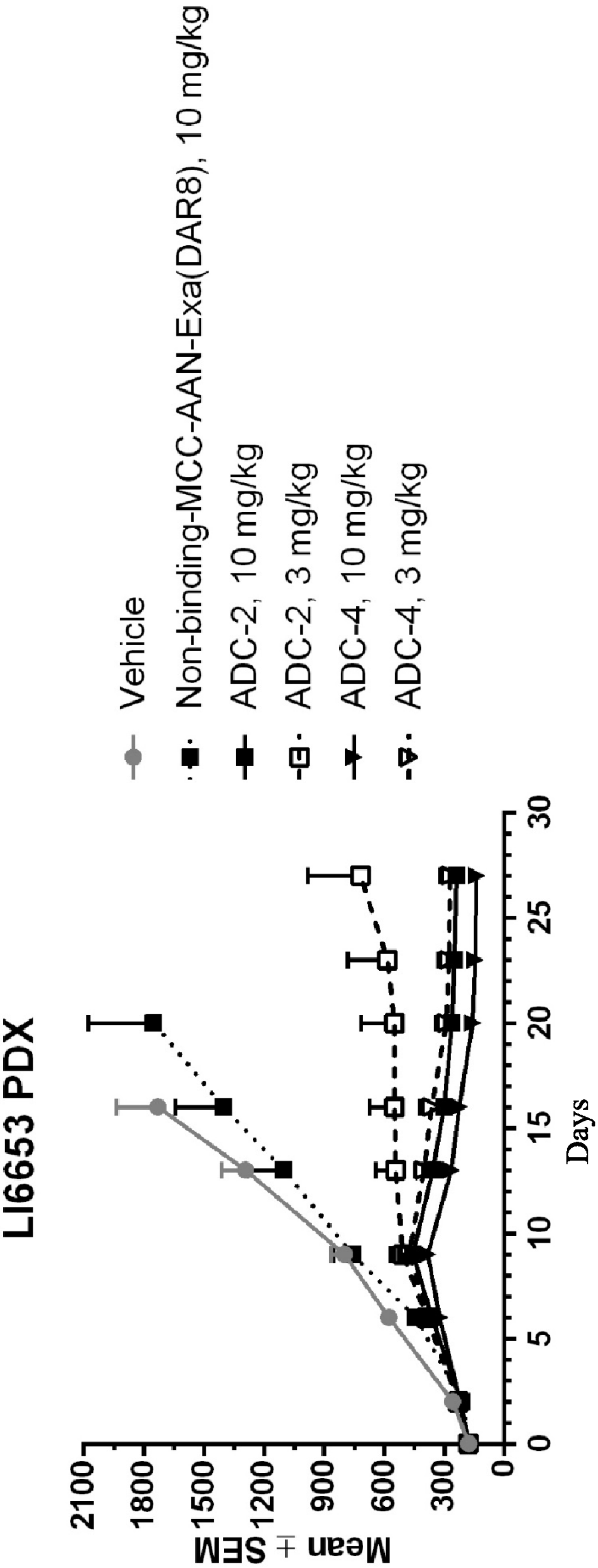
LI6653 PDX
Tumor volume (mm3)
Mean ± SEM
2100
1800
1500
1200
900
600
300
0
0
5
10
15
20
25
30
Days
Vehicle
Non-binding-MCC-AAN-Exa(DAR8), 10 mg/kg
ADC-2, 10 mg/kg
ADC-2, 3 mg/kg
ADC-4, 10 mg/kg
ADC-4, 3 mg/kg

*FIG. 21*

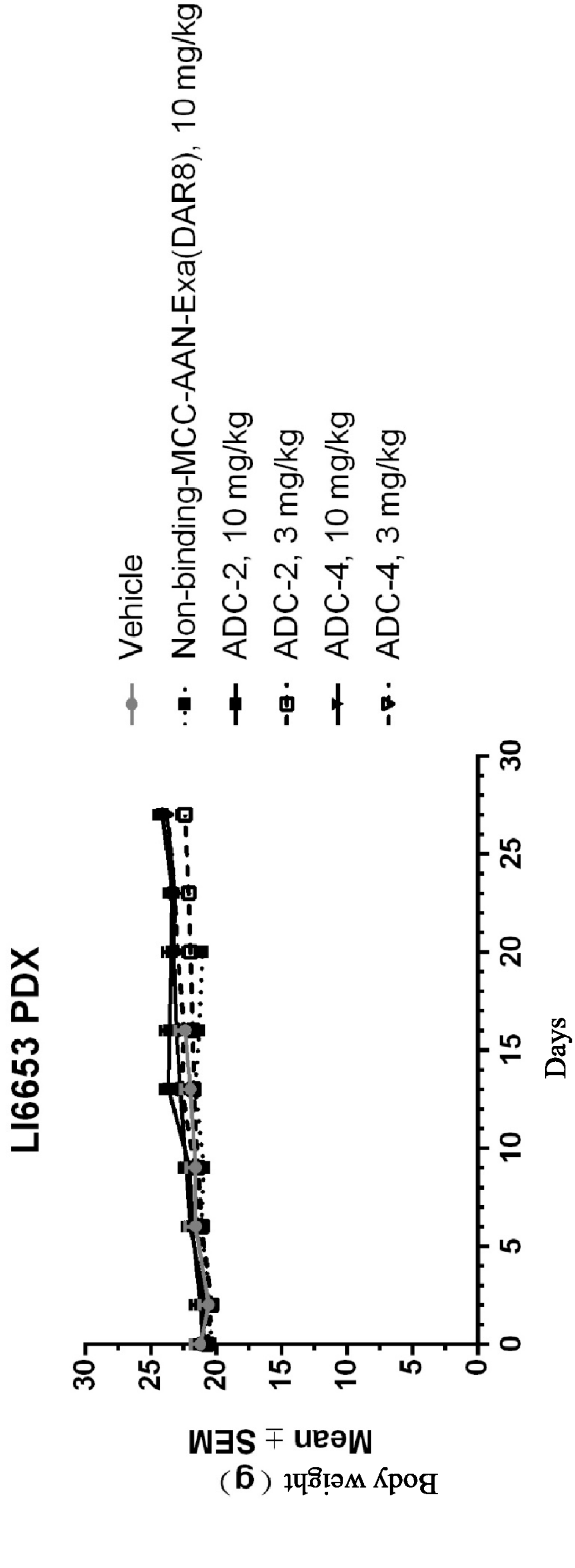
LI6653 PDX
Body weight ( g )
Mean ± SEM
Days
0
5
10
15
20
25
30
Vehicle
Non-binding-MCC-AAN-Exa(DAR8), 10 mg/kg
ADC-2, 10 mg/kg
ADC-2, 3 mg/kg
ADC-4, 10 mg/kg
ADC-4, 3 mg/kg

*FIG. 22*

**INTERNATIONAL SEARCH REPORT**

International application No. **PCT/CN2024/116899**

**A. CLASSIFICATION OF SUBJECT MATTER**

A61K47/68(2017.01)i; A61K47/54(2017.01)i; C07K16/30(2006.01)i; A61P35/00(2006.01)i; C12N15/13(2006.01)i

According to International Patent Classification (IPC) or to both national classification and IPC

**B. FIELDS SEARCHED**

Minimum documentation searched (classification system followed by classification symbols)

IPC: A61K, C12N, C07K, A61P

Documentation searched other than minimum documentation to the extent that such documents are included in the fields searched

Electronic data base consulted during the international search (name of data base and, where practicable, search terms used)

CNTXT; ENTXTC; WPABSC; DWPI; STN; 中国专利生物序列检索系统, China Patent Biological Sequence Search System; NCBI; EBI; CNKI; 百度学术, Baidu Scholar: 上海美雅珂生物, 偶联, ADC, 依喜替康, 伊喜替康, 171335-80-1, 52H5D3B8, 结构检索, structure search, 序列检索, sequence search, PGC3, 肝癌, 丙氨酸, 天冬氨酸, 谷氨酰胺, hepatocellular carcinoma , exatecan, antibody, conjugate, Ala, Asn, Gln

**C. DOCUMENTS CONSIDERED TO BE RELEVANT**

| Category* | Citation of document, with indication, where appropriate, of the relevant passages | Relevant to claim No. |
|---|---|---|
| Y | CN 116669772 A (ARDEAGEN CORPORATION) 29 August 2023 (2023-08-29)<br>abstract, claims 44-45, and description, paragraph 335 | 1-12 |
| Y | WO 2023061505 A1 (CONCEPT TO MEDICINE BIOTECH CO., LTD.) 20 April 2023 (2023-04-20)<br>abstract, claim 1, and sequence listing A | 1-12 |
| Y | CN 115429894 A (SHANGHAI HANSOH BIOMEDICAL CO., LTD. et al.) 06 December 2022 (2022-12-06)<br>description, paragraph 388 | 1-12 |
| Y | WO 2022150831 A1 (INNOVATIVE CELLULAR THERAPEUTICS HOLDINGS, LTD. et al.) 14 July 2022 (2022-07-14)<br>sequence 177 | 1-12 |
| A | CN 114790246 A (CHENGDU KANGHONG BIOTECHNOLOGY CO., LTD.) 26 July 2022 (2022-07-26)<br>claims 1-10 | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **09 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/116899**

**C. DOCUMENTS CONSIDERED TO BE RELEVANT**

| Category* | Citation of document, with indication, where appropriate, of the relevant passages | Relevant to claim No. |
|---|---|---|
| A | WO 2022171115 A1 (WIGEN BIOMEDICINE TECHNOLOGY (SHANGHAI) CO., LTD.) 18 August 2022 (2022-08-18)<br>compound 335, and embodiment 13 | 1-12 |

Form PCT/ISA/210 (second sheet) (July 2022)

**INTERNATIONAL SEARCH REPORT**

International application No.
**PCT/CN2024/116899**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/116899**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11-12**
because they relate to subject matter not required to be searched by this Authority, namely:

Claims 11-12 relate to a method for in-vitro non-therapeutic inhibition of tumor angiogenesis, delay of tumor progression, inhibition of tumor growth or inhibition of tumor cell proliferation, and a method for treating and/or preventing GPC3 positive-related diseases, which fall within subject matter for which no search is required by the International Searching Authority as defined in PCT Rule 39.1(iv). A search is performed on the basis that the designation of the subject matter thereof is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/116899**

| Patent document cited in search report | Publication date (day/month/year) | Patent family member(s) | Publication date (day/month/year) |
|---|---|---|---|
| CN 116669772 A | 29 August 2023 | CA 3197543 A1 | 27 May 2022 |
| | | JP 2023552290 A | 15 December 2023 |
| | | US 2024024499 A1 | 25 January 2024 |
| | | EP 4247433 A1 | 27 September 2023 |
| | | WO 2022109334 A1 | 27 May 2022 |
| WO 2023061505 A1 | 20 April 2023 | JP 2024537323 A | 10 October 2024 |
| | | EP 4416187 A1 | 21 August 2024 |
| CN 115429894 A | 06 December 2022 | None | |
| WO 2022150831 A1 | 14 July 2022 | US 2024024476 A1 | 25 January 2024 |
| | | EP 4274586 A1 | 15 November 2023 |
| CN 114790246 A | 26 July 2022 | WO 2022156799 A1 | 28 July 2022 |
| WO 2022171115 A1 | 18 August 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311141587
- CN 202411179009
- US 7919086 B2
- CN 106397593 B


### Non-patent literature cited in the description


- Kabat Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- Fundamental Immunology. Raven Press, 1989
- **HOLLIGER et al.** *Nat Biotechnol*, 2005, vol. 23, 1126-1136
- **BIRD et al.** *Science*, 1988, vol. 242, 423-426
- **HUSTON et al.** *Proc. Natl. Acad. Sci. USA*, 1988, vol. 85, 5879-5883
- **PLUCKTHUN**. The Pharmacology of Monoclonal Antibodies. Springer-Verlag, 1994, vol. 113, 269-315
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol*, 2001
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **MORIMOTO et al.** *J. Biochem. Biophys. Methods*, 1992, vol. 24, 107-117
- **BRENNAN et al.** *Science*, 1985, vol. 229, 81
- **HUDSON**. *Curr. Opin. Immunol*, 1999, vol. 11, 548-557
- **LITTLE et al.** *Immunol. Today*, 2000, vol. 21, 364-370
- **CARTER et al.** *Bio/Technology*, 1992, vol. 10, 163-167
- **ALTSCHUL et al.** *Nucl. Acid. Res.*, 1977, vol. 25, 3389-3402
- **ALTSCHUL et al.** *J. Mol. Biol.*, 1990, vol. 215, 403-410
- REMINGTON'S PHARMACEUTICAL SCIENCES. Mack Publishing Company, 1995
- Immunology-A Synthesis. Sinauer Associates, 1991
- *CHEMICAL ABSTRACTS*, 35661-39-3
- *CHEMICAL ABSTRACTS*, 6066-82-6
- *CHEMICAL ABSTRACTS*, 56-41-7
- *CHEMICAL ABSTRACTS*, 87512-31-0
- *CHEMICAL ABSTRACTS*, 70-47-3
- *CHEMICAL ABSTRACTS*, 109-89-7
- *CHEMICAL ABSTRACTS*, 55750-63-5
- *CHEMICAL ABSTRACTS*, 169869-90-3